(19) Europäisches Patentamt
European Patent Office
Office européen des brevets

(11) Publication number: **0 058 392**
**B1**

# (12) EUROPEAN PATENT SPECIFICATION

(45) Date of publication of patent specification: **21.11.85**

(21) Application number: **82101006.3**

(22) Date of filing: **11.02.82**

(51) Int. Cl.$^4$: **C 07 D 513/04,**
C 07 D 513/14, A 61 K 31/47
// (C07D513/04, 277:00,
221:00),(C07D513/04, 285:00,
221:00),(C07D513/14, 317:00,
277:00, 221:00),(C07D513/14,
317:00, 279:00, 221:00)

## (54) Substituted carboxylic acid derivatives.

(30) Priority: **18.02.81 JP 23582/81**
**12.12.81 JP 200670/81**

(43) Date of publication of application:
**25.08.82 Bulletin 82/34**

(45) Publication of the grant of the patent:
**21.11.85 Bulletin 85/47**

(84) Designated Contracting States:
**CH DE FR GB IT LI**

(56) References cited:
**GB-A-1 292 525**

**Patent Abstracts of Japan Vol. 1, No. 131, 28 October 1977 page 2902C77**

(73) Proprietor: **NIPPON SHINYAKU COMPANY, LIMITED**
**14, Kisshoin Nishinosho Monguchicho**
**Minami-ku Kyoto-shi Kyoto (JP)**

(72) Inventor: **Matsumura, Shingo**
**22-40 Kaburagi Tanabe-cho**
**Kyoto-fu (JP)**
Inventor: **Kise, Masahiro**
**Fukunaga-cho Aneyakoji Tokinokoji**
**Higashiiru Nakakyo-ku Kyoto (JP)**
Inventor: **Ozaki, Masakuni**
**7-25 Mitani Terada**
**Joyo-shi Kyoto-fu (JP)**
Inventor: **Tada, Shinichi**
**1691-103 Mabuchi-cho**
**Omiyawata-shi Shiga-ken (JP)**
Inventor: **Kazuno, Kenji**
**151-16 Kohirai Ritto-cho**
**Shiga-ken (JP)**
Inventor: **Watanabe, Hisao**
**1-6-353-9 Ninomaru-cho Mukaijima**
**Fushimi-ku Kyoto (JP)**
Inventor: **Kunimoto, Katsutoshi**
**1710-46 Oaza Tane**
**Notogawa-cho Shiga-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 058 392 B1

Inventor: **Enomoto, Hiroshi**
**26-3-7-707 Mibahashiri**
**Baba Nagaokakyo-shi Kyoto-fu (JP)**
Inventor: **Kitano, Masahiko**
**9-4 Donoue-cho Matsugasaki**
**Sakyo-ku Kyoto (JP)**
Inventor: **Morita, Iwao**
**28 Koaza Tohokugumi Oaza Taga**
**Ide-cho Kyoto-fu (JP)**
Inventor: **Tsuda, Masami Nippon Shinyaku**
**Otowa Dormitory 39 Sakanotsuji-cho**
**Oyake Yamashina-ku Kyoto (JP)**

(74) Representative: **Wey, Hans-Heinrich, Dipl.-Ing. et al**
**Patentanwälte Müller-Börner & Wey**
**Widenmayerstrasse 49**
**D-8000 München 22 (DE)**

**Description**

The present invention relates to novel quinolinecarboxylic acid derivatives having antibacterial activity and, more particularly, it relates to compounds represented by the following formula (Z) or pharmaceutically acceptable acid addition salts thereof:

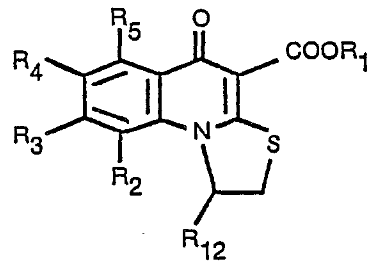

(Z)

in which:

$R_2$ is hydrogen, alkali metal, alkali earth metal, ($C_1$—$C_2$)-alkyl or pivaloyloxymethyl

$R_2$ is hydrogen or halogen

$R_3$ is hydrogen, halogen or piperazinyl with or without substituents as ($C_1$—$C_2$)-alkyl, oxy-methoxypropyl or oxyethyl, or phthalidyl

$R_4$ is hydrogen or halogen

$R_3$ and $R_4$ may form a ring with carbon chain containing oxygen

$R_5$ is hydrogen, halogen or piperazinyl

$R_{12}$ is ($C_1$—$C_2$)-alkyl.

A compound according to the above given formula (Z), in which $R_3$ and $R_4$ are methylenedioxy forming a ring, represents a preferred embodiment of the present invention.

Examples of pharmaceutically acceptable salts of the compounds represented by the general formula (Z) are metal salts such as salts with lithium, sodium, potassium, and calcium; salts with organic bases such as ethanoldiamine and diethanolamine; salts with inorganic acids such as hydrochloric acid, sulfuric acid, and phosphoric acid; and salts with organic acids such as acetic acid, methanesulfonic acid, succinic acid, and lactic acid.

As remedies for diseases infected by Gram negative bacteria, nalidixic acid, piromidic acid or pipenidic acid have been widely used as synthetic antibacterials in these days. Such compounds are not satisfactory for therapy of diseases caused by Gram positive bacteria and by Pseudomonas aeruginosa which is increasing in recent years and is hardly curable disease. In order to solve the problem, various compounds have been synthesized and their antibacterial activity to many bacteria has been studied. As improved type of known antibacterials, the following compounds are now in a stage of development. They are 6-halogeno-1-substituted-7-(4-substituted piperazino)-4-oxo-1,4-dihydroquinoline-3-carboxylic acid derivatives (cf. Japanese Laid Open Patent Applications Sho-53-65887, 53-141286, 54-66686, 55-47658, and others) and 6-fluoro-1,8-naphthylidine derivatives (Japanese Laid Open Patent Application Sho-55-83785).

Among synthetic antibacterials which were already known or which have been in a stage of development, there is no compound having substituent at 2-position of substituted quinoline carboxylic acids. From the viewpoint of literatures, Journal of Medicinal Chemistry (volume 20, page 791, 1977; and volume 21, page 485, 1978) discloses the compounds having methyl and hydroxy substituents but none of them exhibit antibacterial activity.

In order to find 2-substituted compounds having antibacterial activity, the compounds (I) which have not been known in literatures yet and which possess new skeleton have been synthesized and it has thereby been found that they exhibit strong antibacterial activity.

Antibacterial activity of the compounds (Z) are so strong that it goes without saying as to their activity to P. aeruginosa and they are effective to both Gram positive and Gram negative bacteria in such small concentrations that are not disclosed in literatures. Antibacterials are generally apt to effective only to Gram positive or to Gram negative bacteria. Furthermore, to our surprise, the compounds are very strongly antibacterial to Eumycetes or true fungi. Compounds of the present invention can be safely used in therapy by oral administration to human beings.

Examples of manufacture methods of the present invention compounds are then illustrated by using reaction equations. Thus, 4-oxo-1,4-dihydroquinoline-3-carboxylic acid ethyl ester (VII) which is very important intermediate can be synthesized by the following routes.

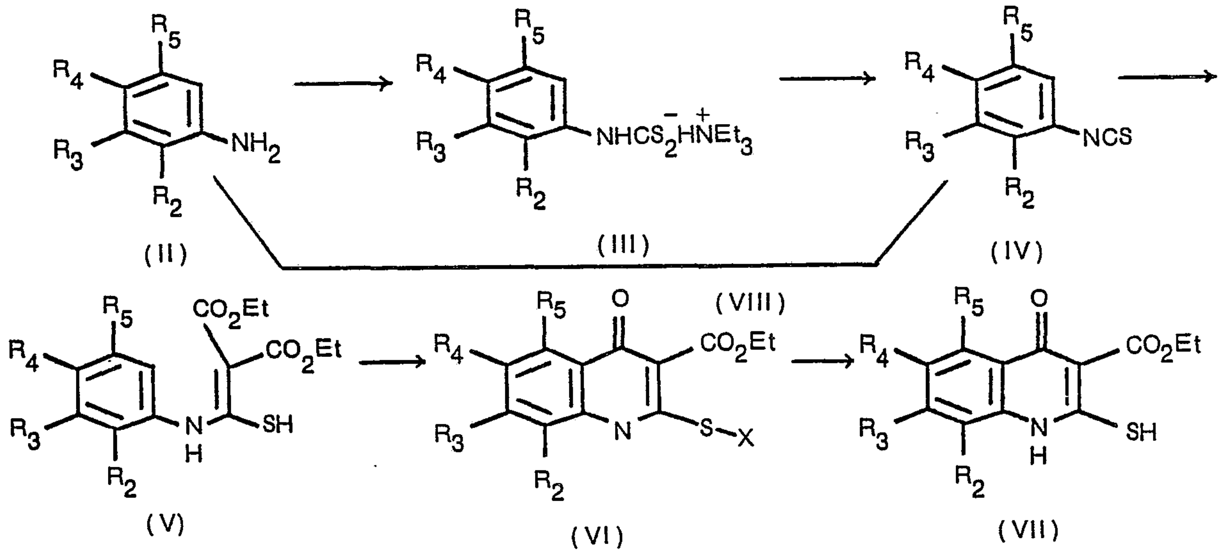

(II) (III) (IV)

(V) (VI) (VIII) (VII)

in which meanings of $R_2$, $R_3$, $R_4$ and $R_5$ are the same as those given in the explanation for the compound (I).

Thus, substituted aniline (II) is made to react with carbon disulfide with cooling in the presence or absence of adequate solvents such as benzene in the presence of an excess of triethylamine or other various amines and various alkali metal to give salt with substituted phenyl dithiocarbamic acid (III). This is then made to react with ethyl chlorocarbonate in such solvents as chloroform or methylene chloride in the presence of triethylamine or is made to react with copper sulfate, lead nitrate, iron sulfate, zinc sulfate, and the like to afford substituted phenyl isothiocyanate (IV). (IV) can also be directly manufactured from (II) by the known method (cf. Organic Synthesis, Collective Volume 1, page 447).

(IV) is then made to react with sodium salt of diethyl malonate to give diethyl substituted phenyl aminomercaptomethylenemalonate (V). This is protected with common protective group for thiol (cf. The Chemistry of the Thiol Group, Part two, Sal Patei, John Wiley and Sons, page 669, 1974) or with substituted alkyl by the known method to give (VI). Examples of useful and common protective groups X for sulfur atom are substituted benzyl, alkoxymethyl, 2,4-dinitrophenyl, disulfide (as a dimer of the compound (V)), alkylthiomethyl, substituted carbamoyl, diphenylmethyl, triphenyl methyl, picolyl, acetamidomethyl, β,β,β-trifluoro-α-acylaminoethyl, β,β-diethoxycarbonylethyl, acetyl, benzoyl, benzyloxycarbonyl, tetrahydropyranyl, benzylthiomethyl, phenylthiomethyl, isobutyroxymethyl, and the like. Taking substituted benzyl as an example, the reaction will be illustrated in some detail. Thus, *p*-metoxybenzyl chloride is made to react with the compound (V) in a solvent such a acetonitrile, dimethylformamide, tetrahydrofuran, and the like in the presence of an alkali such as sodium carbonate, potassium carbonate, and the like, and the resulting compound is subjected to cyclization by heating in high-boiling solvents such as dichloro benzene, tetraline, diphenyl ether, diethylene glycol dimethyl ether, and the like, to give the compound (VI) quantitatively.

The resulting (VI) is then subjected to a known adequate deprotection treatment to give the very important intermediate (VII) in good yield. For example, in case of *p*-methoxybenzyl derivative of (VI) is used, the compound is treated with methanesulfonic acid, trifluoromethanesulfonic acid, trifluoroacetic acid, or a mixture thereof in the presence of anisole under cooling whereupon the protective group can be removed in good yield.

The resulting (VII) is, for example, made to react with dihalide in the presence of potassium carbonate in dimethyl formamide solvent to afford compounds group (VIII) in which nitrogen atom and sulfur atom of 2-mercaptoquinoline are contained in the same ring. Also, when the compound (VII) is made to react with halogeno substituted aldehyde (in the formula, B stands for optionally substituted alkylene with zero to four carbon atoms and

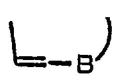

is included in A) to give (IX) in which there is unsaturated bond in the same ring.

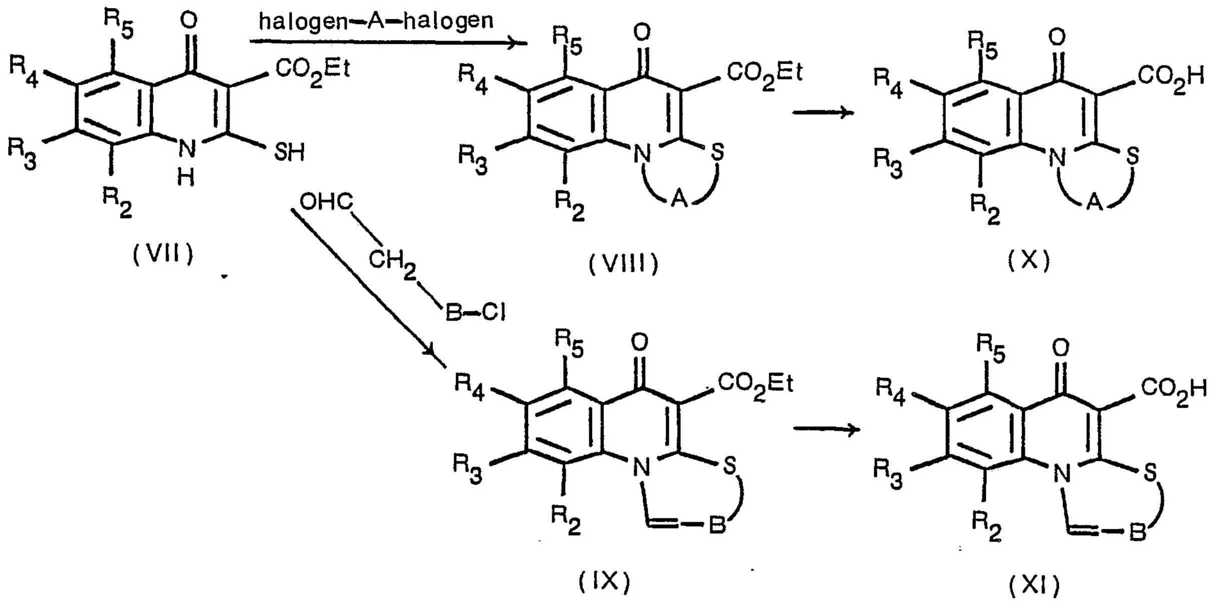


(VII) (VIII) (X)

(IX) (XI)

The resulting (VIII) and (IX) are hydrolyzed in alcohol with sodium hydroxide, potassium hydoxide, and the like to give (X) and (XI), respectively, in good yields. When $R_3$ is halogen in (X) and (XI), such compounds are subjected to a condensation with secondary amines represented by a general formula (XII)

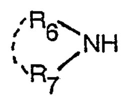

(XII)

(in which, $R_6$ and $R_7$ are the same or different and they represent lower alkyl group or may form five- to seven membered heterocyclic ring together with adjacent nitrogen atom; the said heterocyclic ring may contain other hetero atoms such as nitrogen, oxygen, and sulfur atoms as its constituent, may have substituent(s), or may form a salt) to give the compounds represented by general formulas (XIII) and (XIV).

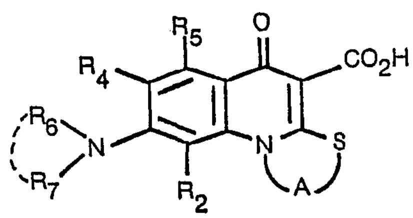

(XIII)

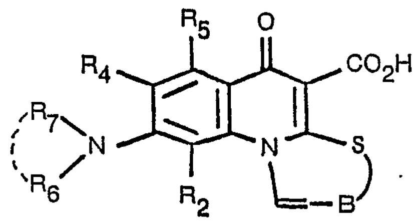

(XIV)

When $R_5$ is halogen in (X) and (XI), they may also be condensed with secondary amines of a formula (XII).

The resulting compound represented by the formula (XV) thus prepared

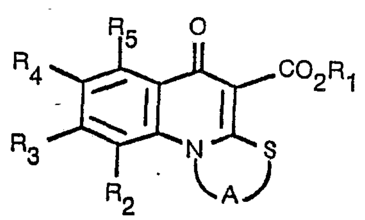

(XV)

is oxidized by the known method to give the corresponding sulfoxide and sulfone. Also, it may be changed to the corresponding sulfylimine and sulfoxyimine by the known method. Thus, the compound represented by the formula (Z) and acid addition salts thereof can be manufactured in good yield in low cost.

Another synthetic route is as follows. Thus, the compounds (VIII) and (XVII) can be prepared starting from the compound (V) by the following way.

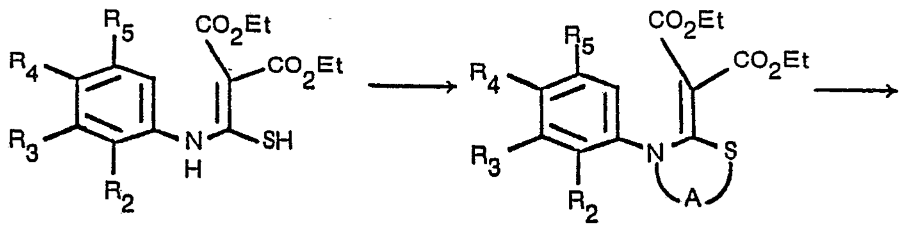

(V) (XVI)

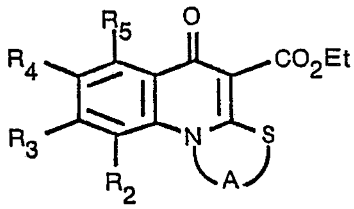

+

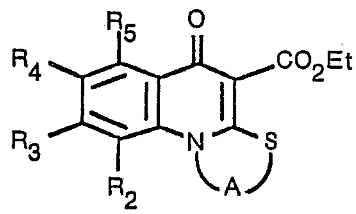

(VIII) (XVII)

in which A, $R_2$, $R_3$, $R_4$ and $R_5$ are the same as already defined.

Thus, the compound (V) is made to react with, instead of with a protective group for sulfur atom, dihalide in dimethyl formamide solvent in the presence of potassium carbonate or is made to react with halogeno substituted aldehyde to give the compound (XVI). The resulting (XVI) is then subjected to a cyclization reaction by the known method. For example, a method by heating or cyclization by the use of acidic substances such as phosphorus oxychloride, phosphorus pentachloride, phosphorus trichloride, thionyl chloride, concentrated sulfuric acid, polyphosphoric acid, polyphosphoric acid esters, and the like may be listed. In carrying out the cylcization reaction using acidic substances, equimolar to an excess molar (preferably ten to twenty times molar) to the compound (XVI) of such substances are used and the mixture is generally heated at 100 to 150°C for 0.5 to 2 hours. When $R_2$ is hydrogen in this case, the compound (XVII) besides (VIII) is produced as a cyclization product. Separation of (VIII) and (XVII) can be done by known method such as, for example, by recrystallization or by column chromatography.

When A is ethylene in the present invention compound (Z), it can be prepared by the following route:

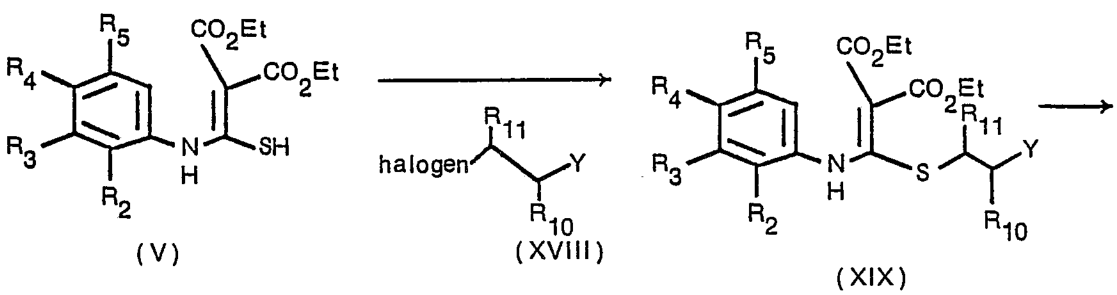

(V) (XVIII) (XIX)

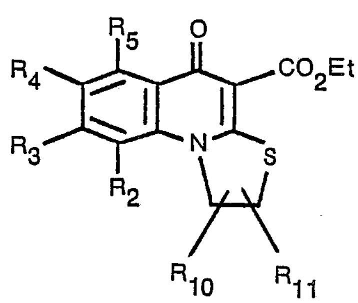

(XX)

in which $R_2$, $R_3$, $R_4$ and $R_5$ are the same as already defined. $R_{10}$ and $R_{11}$ are hydrogen or lower alkyl. Y is hydroxy, alkoxy, acetoxy, trimethylsilyloxy, alkylthio, substituted amino, and the like.

Thus, the compound represented by the general formula (V) is made to react with the compound represented by the general formula (XVIII) to give the compound represented by the general formula (XIX). The resulting compound is heated in high-boiling solvents such a dichlorobenzene, tetraline, diphenyl ether, diethylene glycol dimethyl ether, and the like to give the compound (XX) in high yield.

*Example of Antibacterial Tests:*

The following antibacterial tests were carried out according to a method of Chemotherapeutic Society of Japan. The results are given in the following table. Compounds used as comparision are AM-715 and gentamycin.

AM-715 = 1-ethyl-6-fluoro-4-oxo-7-(1-piperazinyl)-1,4-dihydroquinoline-3-carboxylic acid.

Minimum concentration for inhibiting growth is given in μg/ml.

TABLE

| | AM–715 | Gentamycin | Compound of Ex. 7 | Compound of Ex. 9 |
|---|---|---|---|---|
| Staphylococcus aureus 209P IC) | 0.19 | 0.1 | ≦0.19 | 0.19 |
| Staphylococcus aureus Smith | 0.39 | 0.19 | 0.19 | 0.19 |
| Staphylococcus aureus No. 41 | 0.39 | 0.39 | 0.19 | 0.19 |
| Streptococcus faecalis | 3.13 | 25 | 1.56 | 3.13 |
| List. monocytogenes RIMD1205030 | 3.13 | 0.78 | 1.56 | 1.56 |
| M. luteus ATCC 9341 | 12.5 | 1.56 | 12.5 | 6.25 |
| M. lysodeikticus | 6.25 | 0.19 | 1.56 | 6.25 |
| B. subtilis ATCC 6633 | 0.1 | ≦0.1 | 0.1 | 0.1 |
| E. coli NIHJ JC–2 | 0.1 | 3.13 | 0.1 | 0.39 |
| E. coli NIHJ | 0.05 | 1.56 | 0.05 | 0.1 |
| E. coli KC–14 | 0.05 | 3.13 | 0.05 | 0.1 |
| E. coli No. 29–3 | 0.025 | 3.13 | 0.05 | 0.05 |
| K. pneumoniae | 0.025 | 0.39 | 0.1 | 0.19 |
| K. sp. No. 18 | 0.1 | 3.13 | 0.19 | 0.39 |
| Se. marcescens IFO 3736 | 0.19 | 1.56 | 0.19 | 1.56 |
| Se. marcescens T–55 | 0.78 | 6.25 | 0.19 | 1.56 |
| Sal. typhymurium | 0.05 | 3.13 | 0.1 | 0.36 |
| P. vulgaris HX–19 | 0.05 | 1.56 | 0.025 | 0.025 |
| P. mirabilis 181 | 0.1 | 12.5 | 0.19 | 0.05 |
| Sh. flexneri | 0.025 | 3.13 | 0.05 | 0.11 |
| Ps. aeruginosa No. 12 | 0.78 | 0.78 | 1.56 | 3.13 |
| Ps. aeruginosa E–2 | 0.78 | 6.25 | 1.56 | 3.13 |
| Alc. faecalis | 6.25 | 6.25 | 1.56 | 3.13 |
| Aci. calcoaceticus | 6.25 | 1.56 | 0.78 | 1.56 |

Examples of manufacture methods of the present invention compounds are as follows by which the present invention is further illustrated.

Example 1

(1) 3-(4-Chlorophenyl)-1,3-thiazolidin-2-ylidene-malonic acid diethyl ester

Sodium hydride (purity: 50 percent) (5.7 grams) (0.118 mol) is suspended in 200 ml of dry dioxane and 18.9 g (0.118 mol) of diethyl malonate was dropped thereinto with stirring. The mixture was stirred at room temperature for thirty minutes and then 20.0 grams (0.118 mol) of 4-chlorophenyl isothiocyanate was dropped thereinto. The mixture was stirred for one hour, the content was concentrated in vacuo, the residue was washed with ether, and dried in vacuo to give 38.0 grams of sodium salt of diethyl 4-chlorophenylaminomercaptomethylenemalonate, yellow powdery crystals.

The above sodium salt (5.0 grams; 0.014 mol) was dissolved in 15 ml of dimethyl formamide, 2.3 grams (0.017 mol) of anhydrous potassium carbonate was added thereto, 3.1 grams (0.017 mol) of ethylene dibromide was dropped thereinto at room temperature with stirring, the mixture was stirred at room temperature overnight, the content was diluted with ice water, extracted with chloroform, and the residue was crystallized from ethyl acetate to give 4.4 grams (88.4 percent) of the title compound.

Elementary analysis calculated as $C_{16}H_{18}ClNO_4S$: C 54.01, H 51.0, N 3.44; Found: C 54.12, H 5.01, N 3.91.

Infrared absorption spectra (KBr, $cm^{-1}$): 1710, 1665, 1510, 1295.

Nuclear magnetic resonance spectra $\delta(CCl_4)$: 1.01 (6H, triplet, $—CH_2\underline{CH_3} \times 2$), 3.10 (2H, triplet, $C_5—H$), 3.75 4H, multiplet, $—\underline{CH_2}CH_3 \times 2$), 4.00 (2H, triplet, $C_4—H$), 6.90—7.40 (4H, multiplet, aromatic ring-H).

(2) 7-Chloro-5-oxo-1,2-dihydro-5H-thiazolo(3,2-a)quinoline-4-carboxylic acid

3-(4-Chlorophenyl)-1,3-thiazolidin-2-ylidenemalonic acid diethyl ester (2.76 grams; 7.8 mmol) was heated with stirring for 1.5 hours at 90°C with 28 grams of polyphosphoric acid. After cooled, the content was poured into ice water, extracted with chloroform, washed with water, dried, and concentrated to give crystalline residue which was recrystallized from ethanol to give 1.65 grams (68.2 percent) of ethyl 7-chloro-5-oxo-1,2-dihydro-5H-thiazolo(3,2-a)quinoline-4-carboxylate. Melting point 321°C.

Elementary analysis calculated as $C_{14}H_{12}ClNO_3S$: C 54.28, H 3.90, N 4.52; Found: C 54.45, H 3.82, N 4.45.

Infrared absorption spectra (KBr, $cm^{-1}$): 1700, 1670, 1590, 1480.

Nuclear magnetic resonance spectra δ ($CF_3CO_2S$): 1.60 (3H, triplet, $—CH_2\underline{CH_3}$), 3.86 (2H, triplet, $C_2—H$), 4.72 (2H, quartet, $—\underline{CH_2}CH_3$), 5.22 (2H, triplet, $C_1—H$), 7.70—8.50 (3H, multiplet, aromatic ring-H).

The above compound (1.50 grams; 4.8 mmol) was suspended in a mixture of 3.0 grams of sodium hydroxide, 60 ml of ethanol and 40 ml of water, the mixture was heated to reflux for thirty minutes, acidified with acetic acid when the mixture was hot, the crystals separated were collected by filtration, washed with water, dried with air, and recrystallized from dimethyl formamide to give the title compound, 860 mg (63.2 percent), melting point 310 to 320°C (decomposition).

Elementary analysis calculated as $C_{12}H_{18}ClNO_3S$: C 51.16, H 2.86, N 4.97; Found: C 51.32, H 2.69, N 4.97.

Infrared absorption spectra (KBr, $cm^{-1}$): 1695, 1590, 1480.

Nuclear magnetic resonance spectra ($CF_3CO_2D$): 3.86 (2H, triplet, $C_2—H$), 5.25 (2H, triplet, $C_1—H$), 7.80 to 8.50 (3H, multiplet, aromatic ring-H).

Example 2

7-Fluoro-5-oxo-1,2-dihydro-5H-thiazolo(3,2-a)quinoline-4-carboxylic acid

Diethyl 3-(5-fluorophenyl)-1,3-thiazolidin-2-ylidenemalonate (5.0 grams; 0.015 mol) was heated with 50.0 grams of polyphosphoric acid at 90°C for 1.5 hours with stirring. After cooled, the content was poured into ice water, extracted with chloroform, washed with water, dried, and concentrated to give crystalline residue which was recrystallized from ethanol to give 1.9 grams (43.2 percent) of ethyl 7-fluoro-5-oxo-1,2-dihydro-5H-thiazolo(3,2-a)quinoline-4-carboxylate.

Melting point: 225 to 226°C. Elementary analysis calculated as $C_{14}H_{12}FNO_3S$: C 57.33, H 4.12, N 4.78; Found: C 57.40, H 4.12, N 4.58.

Infrared absorption spectra (KBr, $cm^{-1}$): 1700, 1610, 1490, 1180.

Nuclear magnetic resonance spectra δ ($CF_3CO_2D$): 1.60 (3H, triplet $—CH_2\underline{CH_3}$), 5.26 (2H, triplet, $C_1—H$), 3.87 (2H, triplet, $C_2—H$), 4.57 (quartet, $—\underline{CH_2}CH_3$), 7.70 to 8.40 (3H, multiplet, aromatic ring-H).

The above compound (1.5 grams) (5.1 mmol) was suspended in a mixture of 3.0 grams of sodium hydroxide, 60 ml of ethanol and 40 ml of water, and the mixture was heated to reflux for thirty minutes. The mixture was acidified with acetic acid when it is still hot, the separated crystals were collected by filtration, washed with water, dried with air, and recrystallized from dimethyl formamide to give the title compound. The yield was 850 mg (63.0 percent). Melting point 315 to 325°C (decomposition). Elementary analysis calculated as $C_{12}H_8FNO_3S$: C 54.33, H 3.04, N 5.28. Found: C 54.57, H 2.83, N 5.20.

Infrared absorption spectra (KBr, $cm^{-1}$): 1690, 1600, 1495, 1160.

Nuclear magnetic resonance spectra δ ($CF_3CO_2D$): 3.88 (2H, triplet, $C_2—H$), 5.29 (2H, triplet, $C_1—H$), 7.70 to 8.40 (3H, multiplet, aromatic ring-H).

Example 3

7,8-Dichloro-5-oxo-1,2-dihydro-5H-thiazolo[3,2-a]quinoline-4-carboxylic acid

Diethyl 3-(3,4-dichlorophenyl)-1,3-thiazolidin-2-ylidenemalonate (5.7 grams, 0.015 mol) was heated with stirring for 1.5 hours at 90°C with 32 grams of polyphosphoric acid, cooled, poured into ice water, the crystals separated out therefrom were collected by filtration, washed with water, and air dried to give 4.2

grams (83.7 percent) of powdery crystals. This was a mixture of ethyl 7,8-dichloro-5-oxo-1,2-dihydro-5H-thiazolo[3,2-a]quinoline-4-carboxylate and ethyl 6,7-dichloro-5-oxo-1,2-dihydro-5H-thiazolo[3,2-a]-quinoline-4-carboxylate. The mixture was recrystallized from dimethyl formamide twice to give 1.10 grams of ethyl 6,7-dichloro-5-oxo-1,2-dihydro-5H-thiazol[3,2-a]quinoline-4-carboxylate. Melting point 266 to 267°C. Elementary analysis calculated as $C_{14}H_{11}Cl_2NO_3S$: C 48.85, H 3.22, N 4.07; Found: C 49.03, H 3.19, N 4.11.

Infrared absorption spectra (KBr, $cm^{-1}$): 1710, 1680, 1570, 1460.

Nuclear Magnetic resonance spectra δ ($CF_3CO_2D$): 1.60 (3H, triplet, —$CH_2CH_3$, 3,81 (2H, triplet, $C_2$—H), 4.73 (2H, quartet, —$CH_2CH_3$), 5.22 (2H, triplet, $C_1$H) 7.73 (1H, doublet, $C_8$—H), 8.11 (1H, doublet, $C_9$—H).

A mixture (3.0 grams) recovered from the recrystallization mother liquor of the above compound was subjected to a column chromatography with 200 grams of silica gel (Wako Gel C-300, trademark) and chloroform to give 940 mg of ethyl 7,8-dichloro-5-oxo-1,2-dihydro-5H-thiazolo[3,2-a]quinoline-4-carboxylate. Melting point 255 to 257°C. Elementary analysis calculated as $C_{14}H_{11}Cl_2NO_3S$: C 48,85, H 3.22, N 4.07; Found: C 48.97, H 3.21, N 3.96.

Infrared absorption spectra: (KBr, $cm^{-1}$): 1710, 1670, 1590, 1470, 1340, 1140.

Nuclear magnetic resonance spectra δ ($CF_3CO_2D$): 1.60 (3H, triplet, —$CH_2CH_3$), 3,84 (2H, triplet, $C_2$—H), 4.70 (2H, quartet, —$CH_2CH_3$), 5.20 (2H, triplet, $C_1$—H), 8.00 (1H, singlet, $C_9$—H), 8.60 (1H, singlet, $C_6$—H).

The above compound (2.2 grams; 6.4 mmol) was suspended in a mixture of 4.0 grams of sodium hydroxide, 100 ml of ethanol and 100 ml of water and the mixture was heated to reflux for forty minutes. After cooled, it was acidified with acetic acid, the crystals separated out were collected by filtration, washed with water and dried to give 1.81 grams (89.6 percent) of the title compound. Melting point 305 to 315°C (decomposition). Elementary analysis calculated as $C_{12}H_7Cl_2NO_3S$: C 45.59, H 2.23, N 4.43; Found: C 45.44, H 2.50, N 4.21.

Infrared absorption spectra: (KBr, $cm^{-1}$): 1700, 1590, 1460, 1270.

Nuclear magnetic resonance spectra δ ($CF_3CO_2D$): 3.81 (2H, triplet, $C_2$—H), 5.20 (2H, triplet, $C_1$—H), 7.99 (1H, singlet, $C_9$—H), 8.55 (1H, singlet, $C_6$—H).

Example 4

6,7-Dichloro-5-oxo-1,2-dihydro-5H-thiazolo[3,2-a]quinoline-4-carboxylic acid

Ethyl 6,7-dichloro-5-oxo-1,2-dihydro-5H-thiazolo[3,2-a]quinoline-4-carboxylate (1.0 gram, 2.9 mmol) obtained in Example 3 was suspended in a mixture of 2.0 grams of sodium hydroxide, 140 ml of ethanol and 60 ml of water, the mixture was heated to reflux for thirty minutes, acidified with acetic acid when it was still hot, crystals separated out were collected by filtration, washed with water, and dried and recrystallized from dimethyl formamide to give 580 mg (62.8 percent) of the title compound. Melting point 305 to 310°C (decomposition). Elementary analysis calculated as $C_{12}H_7Cl_2NO_3S$: C 45.59, H 2.23, N 4.43; Found: C 45.85, H 2.19, N 4.37

Infrared absorption spectra: (KBr, $cm^{-1}$): 1695, 1570, 1450, 1110.

Nuclear magnetic resonance spectra δ ($CF_3CO_2D$): 3.81 (2H, triplet, $C_2$—H), 5.20 (2H, triplet, $C_1$—H), 7.65 (1H, doublet, $C_8$—H), 8.08 (1H, doublet, $C_9$—H).

Example 5

7-Chloro-6-(1-piperazinyl)-5-oxo-1,2-dihydro-5H-thiazolo[3,2-a]quinoline-4-carboxylic acid

A mixture of 500 mg (1.58 mmol) of 6,7-dichloro-5-oxo-1,2-dihydro-5H-thiazolo(3,2-a)quinoline-4-carboxylic acid, 1.0 gram (11.6 mmol) of piperazine, and 20 ml of pyridine was heated to reflux for ten hours. The content was then concentrated under reduced pressure, water was added to the residue, insoluble matters were taken out by filtration, washed with water, dried, and recrystallized from dimethyl formamide to give the title compound. Yield was 420 mg (72.7 percent). Melting point: 244 to 245°C (decomposition). Elementary analysis calculated as $C_{16}H_{16}ClN_3O_3S.2\frac{1}{2}H_2O$: C 46.77, H 5.15, N 10.22; Found: C 46.66, H 4.90, N 9.81.

Infrared absorption spectra: (KBr, $cm^{-1}$): 1690, 1610, 1575, 1450, 1360.

Nuclear magnetic resonance spectra δ ($CF_3CO_2D$):

3.40—4.60 (8H, multiplet, 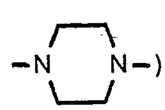),

4.70—5.50 (4H, multiplet, $C_1$—H, $C_2$—H), 7.80—8.20 (2H, multiplet, aromatic ring-H).

Example 6

8-Bromo-7-fluoro-5-oxo-1,2-dihydro-5H-thiazolo(3,2-a)-quinoline-4-carboxylic acid

Diethyl 3-(3-bromo-4-fluorophenyl)-1,3-thiazolydin-2-ylidene-malonate (6.0 grams, 0.0143 mmol) was heated with stirring at 90°C in 60 grams of polyphosphoric acid. After three hours, the content was poured over into ice water, the crystals separated out were collected by filtration, washed with water, and air dried. Then 5.14 grams (97%) of mixture of ethyl 8-bromo-7-fluoro-5-oxo-1,2-dihydro-5H-thiazolo(3,2-a)-quinoline-4-carboxylate and 6-bromo-7-fluoro-5-oxo-1,2-dihydro-5H-thiazolo(3,2-a)quinoline-4-carboxylic acid ethyl ether was obtained.

The above mixture (4.65 grams) was subjected to a column chromatography on 500 grams of silica gel using chloroform as a developer and from the distillate in initial stage, 1.41 grams of ethyl 8-bromo-7-fluoro-5-oxo-1,2-dihydro-5H-thiazolo(3,2-a)-quinoline-4-carboxylate was obtained. Melting point was 258 to 262°C. Elementary analysis calculated as $C_{14}H_{11}BrFNO_3S$: C 45.18, H 2.98, N 3.76; Found: C 45.32, H 2.98, N 3.27.

Infrared absorption spectra: (KBr, $cm^{-1}$): 1705, 1675, 1600, 1480.

Nuclear magnetic resonance spectra δ ($CF_3CO_2D$): 1.50 (3H, triplet, —$CH_2CH_3$), 3.82 (2H, triplet, $C_2$—H), 4.68 (2H, quartet, —$CH_2CH_3$), 5.20 (2H, triplet, $C_1$—H), 8.00—8.40 (2H, multiplet, aromatic ring-H).

Then ethyl 6-bromo-7-fluoro-5-oxo-1,2-dihydro-5H-thiazolo(3,2-a)quinoline-4-carboxylate was distilled out. This was recrystallized from dimethyl formamide. Melting point was 267°C (decomposition). Elementary analysis calculated as $C_{14}H_{11}BrFNO_2S$: C 45.18, H 2.98, N 3.76; Found: C 45.19, H 3.02, N 3.71.

Infrared absorption spectra (KBr, $cm^{-1}$): 1710, 1675, 1620, 1470.

Nuclear magnetic resonance spectra δ ($CF_3CO_2D$): 1.60 (3H, triplet, —$CH_2CH_3$), 3.70 (2H, triplet, $C_2$—H), 4.71 (2H, quartet, —$CH_2CH_3$), 5.22 (2H, triplet, $C_1$—H), 7.70—8.00 (2H, multiplet, aromatic ring-H).

Then 1.0 gram of the fractionated ethyl 8-bromo-7-fluoro-5-oxo-1,2-dihydro-5H-thiazolo(3,2-a)-quinoline-4-carboxylate was suspended in a solution of 2.0 grams of sodium hydroxide (0.05 mmol), 50 ml of water and 20 ml of ethanol, the mixture was heated to reflux for four hours, the transparent solution was acidified with acetic acid after being cooled, crystals separated out were collected by filtration, washed with water, and dried. Thus, 860 mg (93.5 percent) of the title compound was obtained. Colorless powdery crystals were then recrystallized from dimethyl formamide. Melting point was 288 to 290°C (decomposition). Elementary analysis calculated as $C_{12}H_7BrFNO_3S$: C 41.88, 2.05, N 4.07; Found: C 42.19, H 2.02, N 4.06.

Infrared absorption spectra: (KBr, $cm^{-1}$): 1695, 1590, 1470.

Nuclear magnetic resonance spectra δ ($CF_3CO_2D$): 3.80 (2H, triplet, $C_2$—H), 5.16 (2H, triplet, $C_2$—H), 7.70—8.30 (2H, multiplet, aromatic ring-H).

Example 7

7-Fluoro-8-(4-methyl-1-piperazinyl)-5-oxo-1,2-dihydro-5H-thiazolo (3,2-a)-quinoline-4-carboxylic acid

A mixture of 500 mg (1.45 mmol) of 8-bromo-7-fluoro-5-oxo-1,2-dihydro-5H-thiazolo(3,2-a) quinoline-4-carboxylic acid, 730 mg (7.25 mmol) of N-methylpiperazine and 10 ml of pyridine was heated to reflux for twelve hours on an oil bath. The content was then concentrated under reduced pressure, water was added to the residue, insoluble matters were collected by filtration, washed with water, dried, and the resulting powder was recrystallized from dimethyl formamide to give 116 mg (22.0 percent) of the title compound. Melting point was 267 to 271°C (decomposition).

Elementary analysis calculated as $C_{17}H_{18}FN_3O_3S$: C 56.19, H 4.99, N 11.56; Found: C 55.81, H 4.67, N 11.28.

Infrared absorption spectra: (KBr, $cm^{-1}$): 1695, 1625, 1590.

Nuclear magnetic resonance spectra δ ($CF_3CO_2D$): 3.19 (3H, singlet, —$NCH_3$),

3.35—4.50 (10H, multiplet, $C_2$—H, 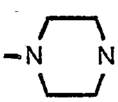).

5.18 (2H, triplet, $C_1$—H), 7.18 (1H, singlet, $C_9$—H), 8.12 (1H, doublet, $C_6$—H).

Example 8

8-Bromo-7-fluoro-1-methyl-5-oxo-1,2-dihydro-5H-thiazolo(3,2-a)quinoline-4-carboxylic acid

Diethyl 3-(3-bromo-4-fluorophenyl)-4-methyl-1,3-thiazolidin-2-ylidenemalonate (4.2 grams) was dissolved in 40 grams of polyphosphoric acid and the solution was heated with stirring at 90°C for three hours. After the reaction, the content was poured over into ice water and extracted with chloroform to give 3.5 grams of syrupy substance. This was subjected to a column chromatagraphy on 350 grams of silica gel using chloroform as a developer and, from the initial distillate, 620 mg of ethyl 8-bromo-7-fluoro-1-methyl-5-oxo-1,2-dihydro-5H-thiazolo(3,2-a)quinoline 4-carboxylate was obtained, Melting point was 211°C. Elementary analysis calculated as $C_{15}H_{13}BrFNO_3S$: C 46.65, N 3.39, N 3.63; Found: C 46.59, H 3.28, N 3.57.

Infrared absorption spectra: (KBr, $cm^{-1}$): 1720, 1670, 1640, 1605.

Nuclear magnetic resonance δ ($CDCl_3$): 1.40 (3H, triplet, —$CH_2CH_3$), 1.48 (3H, doublet, $C_1$—$CH_3$), 2.93 (1H, doublet, $C_2$—H), 3.61 (1H, doublet doublet, $C_2$—H), 4.32 (2H, quartet, —$CH_2CH_3$), 5.29 (1H, multiplet, $C_1$—H), 7.44 (1H, triplet, $C_9$—H), 7.94 (1H, doublet $C_6$—H).

Then 450 mg of ethyl 6-bromo-7-fluoro-methyl-5-oxo-1,2-dihydro-2H-thiazolo(3,2-a)-quinoline-4-carboxylate was obtained from the succeeding distillate. Melting point was 219°C. Elementary analysis calculated as $C_{15}H_{13}BrFNO_3S$: C 46.65, H 3.39, N 3.63; Found: C 46.74, H 3.31, N 3.51.

Infrared absorption spectra: (KBr, $cm^{-1}$): 1680, 1620, 1470.

Nuclear magnetic resonance spectra δ ($CF_3CO_2D$): 1.60 (3H, triplet, —$CH_2CH_3$), 1.65 (3H, doublet, $C_1$—$CH_3$), 3.36 (1H, doublet, $C_2$—HH), 3.98 (1H, doublet, doublet, $C_2$—H), 4.70 (2H, quartet, —$CH_2CH_3$), 5.94 (1H, multiplet, $C_1$—H), 7.70—8.10 (2H, multiplet, aromatic ring-H).

Then 600 mg (1.55 mmol) of ethyl 8-bromo-7-fluoro-1-methyl-5-oxo-1,2-dihydro-5H-thiazolo(3,2-a)quinoline-4-carboxylate separated by column chromatography was suspended in 50 ml of ethanol containing 1.2 grams (0.02 mol) of potassium hydroxide and the mixture was heated to reflux for 1.5 hours. The content was concentrated under reduced pressure and, after water was added to the residue, insoluble matters were filtered and removed. This was acidified with acetic acid and crystals separated therefrom were collected by filtration, washed with water, and dried to give 520 mg (93.7 percent) of the title compound. Recrystallized from dimethyl formamide. Melting point 290 to 291°C (decomposition). Elementary analysis calculated as $C_{13}H_9BrFNO_3S$: C 43.59, H 2.53, N 3.91; Found: C 43.78, H 2.55, N 3.93.

Infrared absorption spectra: (KBr, $cm^{-1}$): 1659, 1590, 1470.

Nuclear magnetic resonance spectra δ ($CF_3CO_2D$): 1.70 (3H, doublet, $C_1$—$CH_3$), 3.20—4.30 (2H, multiplet, $C_2$—H), 5.60—6.20 (1H, multiplet, $C_1$—H), 7.70—8.70 (2H, multiplet, aromatic ring-H).

Example 9

7-Fluoro-1-methyl-8-(4-methyl-1-piperazinyl)-5-oxo-1,2-dihydro-5H-thiazolo(3,2-a)-quinoline-4-carboxylic acid

A mixture of 400 mg (1.12 mmol) of 8-bromo-7-fluoro-1-methyl-5-oxo-1,2-dihydro-5H-thiazolo(3,2-a)-quinoline-4-carboxylic acid, 1.1 grams (1.12 mmol) of N-methylpiperazine and 20 ml of pyridine was heated to reflux for forty eight hours in an oil bath. When the reaction was completed, the content was concentrated in vacuo, then water was added to the residue, insoluble matter was collected by filtration, washed with water and dried to give 240 mg of the title compound. Recrystallized from dimethyl formamide. Melting point was 292 to 294°C (decomposition). Elementary analysis calculated at $C_{18}H_{20}FN_3O_3S$: C 57.28, H 5.34, N 11.13; C 57.03, H 5.25, N 10.87.

Infrared absorption spectra: (KBr, $cm^{-1}$): 1700, 1630, 1480.

Nuclear magnetic resonance spectra δ ($CF_3CO_2D$): 1.70 (3H, doublet, $C_2$—$CH_3$), 3.15 (3H, singlet, —$NCH_3$),

3.30—4.50 (10H, multiplet, 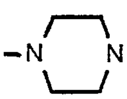, $C_2$—H),

5.60—6.20 (1H, multiplet, $C_1$—H), 7.20 (1H, wide singlet, $C_9$—H), 8.11 (1H, doublet, $C_6$—H).

Example 10

Ethyl 7,8-methylenedioxy-5-oxo-1,2-dihydro-5H-thiazolo(3,2-a)quinoline-4-carboxylate

Diethyl 3-(3,4-methylenedioxyphenyl)-1,3-thiazolidin-2-ylidene malonate (5.37 grams, 0.015 mol) was heated with stirring in 50.0 grams of polyphosphoric acid at 80 to 90°C and, after 1.5 hours, the content was poured over ice water, insoluble matter was extracted with chloroform, washed with water, dried, concentrated, and the residue was recrystallized from dimethyl formamide to give 1.45 grams (30.9 percent) of the title compound, melting point 308°C (decomposition). Elementary analysis calculated as $C_{15}H_{13}NO_2S$: C 56.42, H 4.10, N 4.39; Found: C 56.00, H 4.04, N 4.21.

Infrared absorption spectra: (KBr, $cm^{-1}$): 1660, 1625, 1585, 1230, 1030.

Nuclear magnetic resonance spectra δ ($CF_3CO_2D$): 1.60 (3H, triplet, —$CH_2\underline{CH_3}$), 3.78 (2H, triplet, $C_2$—H), 4.70 (2H, quartet, —$\underline{CH_2}CH_3$), 5.10 (2H, triplet, $C_1$—H), 6.25 (2H, singlet, O—$\underline{CH_2}$—O), 7.14 (1H, singlet, $C_9$—H), 7.70 (1H, singlet, $C_6$—H).

Example 11

7,8-Methylenedioxy-5-oxo-1,2-dihydro-5H-thiazolo(3,2-a)-quinoline-4-carboxylic acid

Ethyl 7,8-methylenedioxy-5-oxo-1,2-dihydro-5H-thiazolo(3,2-a)quinoline-4-carboxylate (1.38 grams, 4.3 mmol) was suspended in a mixture of 2.0 grams of sodium hydroxide, 40 ml of ethanol and 30 ml of water and the whole mixture was heated to reflux for thirty minutes. Then it was acidified with acetic acid when the solution was still hot, the crystals separated out were collected by filtration, washed with water and air dried and the resulting crude crystals were recrystallized from dimethyl formamide to give 870 mg (69.0 percent) of the title compound, melting point 315 to 320°C (decomposition). Elementary analysis calculated as $C_{13}H_9NO_5S$: C, 53.61, H 3.11, N 4.81; Found: C 53.68, H 2.92, N 4.66.

Infrared absorption spectra (KBr, $cm^{-1}$): 1695, 1630, 1270, 1040.

Nuclear magnetic resonance spectra δ ($CF_3CO_2D$): 3.80 (2H, triplet, $C_2$—H), 5.15 (2H, triplet, $C_1$—H), 6.28 (2H, singlet, —$O\underline{CH_2}O$—), 7.20 (1H, singlet, $C_9$—H), 7.71 (1H, singlet, $C_6$—H).

Example 12

Pivaloyloxymethyl 7,8-methylenedioxy-5-oxo-1,2-dihydro-5H-thiazolo(3,2-a)-quinoline-4-carboxylate

7,8-Methylenedioxy-5-oxo-1,2-dihydro-5H-thiazolo(3,2-a)-quinoline-4-carboxylic acid (200 mg, 0.687 mmol) was dissolved in 5 ml of dimethyl formamide, then 152 mg of anhydrous potassium carbonate (1.1 mmol) and 328 mg (2.18 mmol) of pivaloyloxymethyl chloride were added thereto, the mixture was heated with stirring at 100°C for six hours, the content was concentrated under reduced pressure, water was added to the residue, then insoluble matter was collected by filtration, washed with water, air dried and recrystallized from a mixture of chloroform and ether to give 200 mg of the title compound (71.9

percent yield). Melting point 229.0 to 229.5°C. Elementary analysis calculated as $C_{19}H_{19}NO_7S.\frac{1}{4}H_2O$: C 55.67, H 4.79, N 3.42; Found: C 55.65, H 4.59, N 3.49.

Infrared absorption spectra (KBr, $cm^{-1}$): 1740, 1685, 1600, 1120.

Nuclear magnetic resonance spectra δ ($CF_3CO_2D$): 1.33 (9H, singlet, —$C(CH_3)_3$), 3.76 (2H, triplet, $C_2$—H), 5.70 (2H, triplet, $C_2$—H), 6.16 (2H, singlet, —$COOCH_2OCO$—), 6.20 (2H, singlet, —$OCH_2O$—), 7.12 (1H, singlet, $C_9$—H), 7.68 (1H, singlet, $C_6$—H).

Example 13

8-Chloro-9-fluoro-5-oxo-1,2-dihydro-5H-thiazolo(3,2-a)-quinoline-4-carboxylic acid

Diethyl 3-(3-chloro-2-fluorophenyl)-1,3-thiazolidin-2-ylidene malonate (7.0 grams, 0.0187 mol) was heated with stirred for two hours in 70 grams of polyphosphoric acid at 90°C. After cooled, the content was poured over into ice water, extracted with chloroform, washed with water, dried and concentrated to give 5.9 grams of oily product. This was recrystallized from ethyl acetate to give 5.05 grams (82.4 percent) of ethyl 8-chloro-9-fluoro-5-oxo-1,2-dihydro-5H-thiazolo(3,2-a)-quinoline-4-carboxylate, melting point 216°C. Elementary analysis calculated as $C_{14}H_{11}ClFNO_3S$: C 51.30, H 3.38, N 4.27; Found: C 51.31, H 3.28, N 4.06.

Infrared absorption spectra (KBr, $cm^{-1}$): 1665, 1625, 1590, 1180.

Nuclear magnetic resonance spectra δ ($CF_3CO_2D$): 1.60 (3H, triplet, —$CH_2CH_3$), 3.81 (2H, triplet, $C_2$—H), 4.29 (2H, quartet, —$CH_2CH_3$), 5.3 to 5.7 (2H, multiplet, $C_1$—H), 7.75 (1H, doublet, doublet, $C_7$—H), 8.27 (1H, doublet, doublet, $C_6$—H).

Then the above resulting compound (5.9 grams, 0.018 mol) was suspended in a mixture of 12.0 grams of sodium hydroxide, 100 ml of ethanol and 100 ml of water and the whole mixture was heated to reflux for two hours. After cooled, it was acidified with acetic acid and the crystals separated out were collected by filtration, washed with water, and air dried and finally recrystallized from dimethyl formamide to give 3.9 grams (72.4 percent) of the title compound, melting point 293 to 296°C (decomposition). Elementary analysis calculated as $C_{12}H_7ClFNO_3S$: C 48.09, H 2.35, N 4.67; Found: C 48.18, H 2.34, N 4.56.

Infrared absorption spectra (KBr, $cm^{-1}$): 1640, 1550, 1500, 1250.

Nuclear magnetic resonance spectra δ ($CF_3CO_2D$): 3.83 (2H, triplet, $C_2$—H), 5.3—5.7 (2H, multiplet, $C_1$—H), 7.80 (1H, doublet, doublet, $C_7$—H), 8.32 (1H, doublet, doublet, $C_6$—H).

Example 14

9-Fluoro-8-(4-methyl-1-piperazinyl)-5-oxo-1,2-dihydro-5H-thiazolo(3,2-a)-quinoline-4-carboxylic acid

8-Chloro-9-fluoro-5-oxo-1,2-dihydro-5H-thiazolo(3,2-a)-quinoline-4-carboxylic acid (0.8 gram, 2.7 mmol) was dissolved in 5 ml of pyridine, then 2.7 grams (27 mmol) of N-methylpiperazine was added thereto, and the mixture was heated to reflux for forty eight hours. The content was concentrated under reduced pressure, water was added to the residue, and the insoluble matter was collected by filtration, wahed with water, dried and recrystallized from dimethyl formamide to give 324 mg (42.0 percent) of the title compound, melting point 265 to 266°C (decomposition). Elementary analysis calculated as $C_{17}H_{18}FN_3O_3S$: C 56.19, H 4.99, N 11.56; Found: C 56.23, H 4.96, N 11.56

Infrared absorption spectra (KBr, $cm^{-1}$): 1700, 1615, 1580, 1495, 1255.

Nuclear magnetic resonance spectra δ ($CF_3CO_2D$): 3.16 (3H, singlet, H—$CH_3$),

3.20 to 4.40 (10H, multiplet, 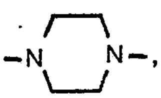, $C_2$—H),

5.45 (2H, triplet, $C_1$—H), 7.41 (1H, doublet, doublet, $C_7$—H), 8.30 (1H, doublet, doublet, $C_6$—H).

Example 15.

Ethyl 6-chloro-7-fluoro-5-oxo-1,2-dihydro-5H-thiazolo(3,2-a)-quinoline-4-carboxylate and Ethyl 8-chloro-7-fluoro-5-oxo-1,2-dihydro-5H-thiazolo(3,2-a)-quinoline-4-carboxylate

Diethyl 3-(3-chloro-4-fluorophenyl)1,3-thiazolidin-2-ylidene malonate (7.15 grams, 0.019 mol) was added to 71 grams of polyphosphoric acid and the mixture was heated with stirring in an oil bath at 120°C for two hours. After the reaction was completed, the mixture was poured over into ice water with ice cooling. After the mixture was stirred for a while, crystals separated out were collected by filtration and 5.08 grams of beige powder. The powder was separated by purification using chloroform as a developer in dry column to give the title compounds.

Physical properties and instrumental data for the 6-chloro-7-fluoro compound are as follows. Yield 2.97 grams (58.5 percent), melting point 278°C.

Infrared absorption spectra (KBr, $cm^{-1}$): 1710, 1680, 1620

Elementary analysis calculated as $C_{14}H_{11}FClNO_2S$: C 51.30, H 3.38, N 4.27; Found: C 51.38, H 3.25, N 4.11.

Nuclear magnetic resonance spectra δ ($CF_3CO_2D$): 1.63 (3H, triplet, —$CH_3$), 3.85 (2H, multiplet, $C_2$—H), 4.78 (2H, quartet, —$OCH_2$—), 5.28 (2H, multiplet, $C_1$—H), 7.81 (1H, singlet, $C_9$—H), 7.91 (1H, doublet, $C_8$—H).

Physical properties and instrumental data for the 8-chloro-7-fluoro compound are as follows. Pale yellow powdery crystals. Yield 2.04 grams (40.2 percent), melting point 266°C.

Infrared absorption spectra (KBr, $cm^{-1}$): 1750, 1680, 1590

Elementary analysis calculated as $C_{14}H_{11}FClNO_3S.\frac{1}{4}\ H_2O$): C 50.61, H 3.49, N 4.22; Found: C 50.69, H 3.24, N 4.07

Nuclear magnetic resonance spectra δ ($CF_3CO_2D$): 1.65 (3H, triplet, —$CH_3$), 3.28 (2H, triplet, $C_2$—H), 4.75 (2H, quartet, —$CH_2$—), 5.23 (2H, triplet, $C_1$—H), 7.50—8.20 (2H, multiplet, aromatic ring-H).

Example 16

6-Chloro-7-fluoro-5-oxo-1,2-dihydro-5H-thiazolo(3,2-a)-quinoline-4-carboxylic acid

One gram (3.05 mmol) of ethyl 6-chloro-7-fluoro-5-oxo-1,2-dihydro-5H-thiazolo(3,2-a)-quinoline-4-carboxylate was added to 50 ml of a mixture of ethanol and water (1:1), then 3 ml of 2N sodium hydroxide solution was added thereto, and the mixture was heated to reflux for two hours. When the reaction was completed, the mixture was cooled to a room temperature and crystals separated out were collected by filtration to give 700 mg of sodium salt. The resulting sodium salt was suspended in water at hot state and dimethyl formamide was added thereto to prepare uniform solution. The solution was adjusted to pH ≑ 7.0 using 1N aqueous solution of acetic acid, crystals separated out were collected by filtration, washed with acetone and ether, ari dried and the title compound was obtained. White powder. Yield 629 mg (68.8 percent). Melting point 326°C (decomposition). Elementary analysis calculated as $C_{12}H_7FClNO_3S$: C48.09, H 2.35, N 4.67, Found: C 48.28, H 2.15, N 4.44

Infrared absorption spectra (KBr, $cm^{-1}$): 1690, 1595

Nuclear magnetic resonance spectra δ ($CF_3CO_2D$): 3.85 (2H, triplet, $C_2$—H), 5.27 (2H, triplet, $C_1$—H), 7.6—8.0 (2H, multiplet, aromatic ring-H)

Example 17

7-Fluoro-6-(1-piperazinyl)-5-oxo-1,2-dihydro-5H-thiazolo(3,2-a)-quinoline-4-carboxylic acid

6-Chloro-7-fluoro-1,2-dihydro-5-oxo-5H-thiazolo(3,2-a)-quinoline-4-carboxylic acid (410 mg, 1.3 mmol) and 1.3 grams (15 mmol) were added to 20 ml of pyridine and the mixture was heated to reflux for nine hours. When the reaction was completed, the mixture was allowed to cool to a room temperature, separated crystals were collected by filtration, washed with small amounts of acetone and ether, and air dried to give the title compound. Yellow powders. Yield 445 mg (93.7 percent). Melting poin t 249°C (decomposition). Elementary analysis calculated as $C_{16}H_{16}FN_3O_3S.H_2O$C C 51.06, H 4.82, N 11.16; Found: C 51.00, H 4.94, N 11.26.

Infrared absorption spectra (KBr, $cm^{-1}$): 3000—2400, 1600

Nuclear magnetic resonance spectra δ ($CF_3CO_2D$):

3.50—5.50 (12H, multiplet, $C_1$—H, $C_2$—H, 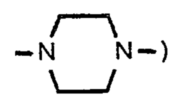 ),

7.80—8.30 (2H, multiplet, aromatic ring-H)

Example 18

8-Chloro-7-fluoro-5-oxo-1,2-dihydro-5H-thiazolo(3,2-a)-quinoline-4-carboxylic acid

Six hundred mg (1.83 mmol) of ethyl 8-chloro-7-fluoro-5-oxo-1,2-dihydro-5H-thiazolo(3,2-a)-quinoline-4-carboxylate obtained in Example 15 was added to a mixed solvent of ethanol and water (1:1), then 2 ml of 2N aqueous solution of sodium hydroxide was added thereto, and the mixture was heated to reflux for two hours. When the reaction was completed, ethanol was evaporated therefrom, the residual aqueous solution was adjusted to pH nearly 7.0 with 1N aqueous solution of acetic acid and, after stirring for a while, crystals separated out therefrom were collected by filtration, successively washed with ethanol, acetone and ether and air dried to give 501 mg (91.4 percent) of the title compound, melting point 318°C (decomposition). elementary analysis calculated as $C_{12}H_7FClNO_3S$: C 48.09, H 2.35, N. 4.67; Found: C 48.28, H 2.14, N 4.37.

Infrared absorption spectra (KBr, $cm^{-1}$): 1700, 1590

Nuclear magnetic resonance spectra δ ($CF_3CO_2D$): 3.82 (2H, triplet, $C_2$—H), 5.23 (2H, triplet, $C_1$—H), 8.00 (1H, doublet, $C_9$—H), 8.23 (1H, doublet, $C_6$—H)

Example 19

7-Fluoro-8-(4-methyl-1-piperazinyl)-5-oxo-1,2-dihydro-5H-thiazolo(3,2-a)-quinoline-4-carboxylic acid

A mixture of 1 gram (3.3 mmol) of 8-chloro-7-fluoro-5-oxo-1,2-dihydro-5H-thiazolo(3,2-a)-quinoline-4-carboxylic acid, 1.65 g (0.0165 mol) of N-methylpiperazine and 20 ml of pyridine was heated to reflux. After twelve hours, 1.65 g (0.0165 mol) of N-methylpiperazine was added thereto again and the heating to reflux was continued for twelve hours. When the reaction was completed, the mixture was allowed to cool to a room temperature, crystals separated out therefrom were collected by filtration, washed with pyridine and ether and air dried to give the title compound. Yellow powdery crystals. Yield 720 mg (60 percent). Melting point 265°C (decomposition). Elementary analysis calculated as $C_{17}H_{18}FN_3O_3S$: C 56.19, H 4.99, N 11.56; Found: C 56.19, H 4.97, N 11.61.

Both infrared absorption spectra and nuclear magnetic resonance spectra coincided with those of the Example 7.

## Example 20

7-Fluoro-8-(1-piperazinyl)-5-oxo-1,2-dihydro-5H-thiazolo(3,2-a)-quinoline-4-carboxylic acid

To one gram of 8-chloro-7-fluoro-5-oxo-1,2-dihydro-5H-thiazolo(3,2-a)-quinoline-4-carboxylic acid (33 mmol) were added 2.87 grams (33 mmol) of anhydrous piperazine and 30 ml of pyridine and the resulting suspension was heated to reflux for twelve hours in an oil bath. Pyridine was evaporated therefrom under reduced pressure, water was added to the residue, and the mixture was again concentrated under reduced pressure. Water was added to the resulting residue, then 4 ml of 2N aqueous solution of sodium hydroxide was added thereto, and the mixture was warmed to prepared uniform solution. Then this was adjusted to pH nearly seven with 1N hydrochloric acid. Crystals separated out therefrom were collected by filtration, washed with water, washed with acetone and ether, and air dried to give the title product as powdery crystals. Yield 997.8 mg (90.7 percent). Melting point 287 to 288°C (decomposition). Elementary analysis calculated as $C_{16}H_{16}FN_3O_3S.3H_2O$: C 47.64, H 5.50, N 10.41; Found: C 47.60, H 5.42, N 10.63.

Infrared absorption spectra (KBr, $cm^{-1}$): 3000—2400, 1600, 1565

Nuclear magnetic resonance δ ($CF_3CO_2D$):

3.82 (10H, multiplet, $C_2$—H, 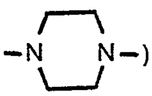),

5.15 (2H, multiplet $C_1$—H), 7.15 (1H, doublet, $C_9$—H), 8.11 (1H, doublet, $C_6$—H).

## Example 21

7-Fluoro-8-(4-phthalidyl-1-piperazinyl)-5-oxo-1,2-dihydro-5H-thiazolo(3,2-a)-quinoline-4-carboxylic acid

A mixture of 300 mg (0.9 mmol) of 7-fluoro-8-(1-piperazinyl)-5-oxo-1,2-dihydro-5H-thiazolo(3,2-a)-quinoline-4-carboxylic acid and 13.5 mg (0.9 mmol) of o-phthal aldehyde acid was suspended in 4 ml of dimethyl formamide and the whole mixture was heated with stirring at 120°C for seven hours in an oil bath. When the reaction was completed, the mixture was allowed to cool to a room temperature and, by collecting the crystals separated out therefrom by filtration, the title compound was obtained as powder. Yield 207 mg (47.7 percent). Melting point 261 to 262°C (decomposition). Elementary analysis calculated as $C_{24}H_{20}FN_3O_5S$: C 59.87, H 4.19, N 8.73; Found: C 60.09, H 4.32, N 8.64.

Infrared absorption spectra (KBr, $cm^{-1}$): 1750, 1690, 1600

Nuclear magnetic resonance δ ($CF_3CO_2D$):

3.50—4.80 (10H, multiplet, $C_2$—H, 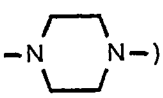),

4.88—5.40 (2H, multiplet, $C_1$—H), 7.00-7.35 (1H, multiplet, $C_9$—H), 7.40—8.60 (4H, multiplet, aromatic ring) 8.15 (1H, doublet, $C_6$—H), 9.70 (1H, wide singlet,

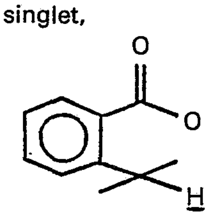

## Example 22

7-Fluoro-8-(4-(2-hydroxy-3-methoxy-1-propyl)-1-piperazinyl)-5-oxo-1,2-dihydro-5H-thiazolo(3,2-a)-quinoline-4-carboxylic acid

A mixture of 500 mg (1.67 mmol) of 8-chloro-7-fluoro-5-oxo-1,2-dihydro-5H-thiazolo(3,2-a)-quinoline-4-carboxylic acid and 1.45 grams (8.3 mmol) of N-(2-hydroxy-3-methoxy-1-propyl)-piperazine was suspended in 20 ml of pyridine and the mixture was heated to reflux for thirty six hours. When the reaction was completed, pyridine was evaporated therefrom under reduced pressure and ethanol was added to the residue. Insoluble matters were filtered, washed with ether, and recrystallized from acetonitrile to give the title compound as flaky crystals. Yield 397.8 mg (54.5 percent). Melting point 224°C. Elementary analysis calculated as $C_{20}H_{24}FN_3O_5S$: C 54.91, H 5.53, N 9.60; Found: C 54.94, H 5.55, N 9.60

Infrared absorption spectra (KBr, $cm^{-1}$): 2830, 1695, 1630, 1590

Nuclear magnetic resonance spectra δ ($CF_3CO_2D$): 3.60 (3H, singlet, N—$CH_3$),

3.20—4.90 (18H, multiplet, $C_2$—H, 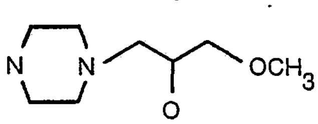,

7,15 (1H, doublet, $C_9$—H), 8,12 (1H, doublet, $C_6$—H).

## Example 23

7-Fluoro-8-(4-(2-hydroxyethyl)-1-piperazinyl)-5-oxo-1,2-dihydro-5H-thiazolo(3,2-a)-quinoline-4-carboxylic acid

A mixture of 500 mg (1.67 mmol) of 8-chloro-7-fluoro-5-oxo-1,2-dihydro-5H-thiazolo(3,2-a)-quinoline-4-carboxylic acid and 1.09 grams (8.34 mmol) of N-(beta-hydroxyethyl)-piperazine was suspended in 20 ml of pyridine and the mixture was heated to reflux for thirty two hours. When the reaction was completed, the mixture was cooled to a room temperature, crystals separated out therefrom were collected by filtration, successively washed with pyridine, ethanol and ether, and recrystallized from dimethyl formamide to give the title compound as pale yellow powders. Yield 426.6 mg (65 percent). Melting point 256 to 256.5°C. Elementary analysis calculated as $C_{18}H_{20}FN_3O_4S$: C 54.95, H 5.12, N 10.68; Found: C 54.52, H 5.14, N 10.73.

Infrared absorption spectra (KBr, $cm^{-1}$): 1695, 1625, 1590

Nuclear magnetic resonance δ ($CF_3CO_2D$):

3.20—4.70 (14H, multiplet, 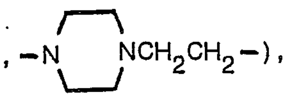),

5.18 (2H, multiplet, $C_2$—H, $C_1$—H), 7.17 (1H, doublet, $C_9$H), 8.12 (1H, doublet, $C_6$—H).

## Example 24

8-Chloro-7-fluoro-5-oxo-1,2-dihydro-5H-thiazolo(3,2-a)-quinoline-4-carboxylic acid ethyl ester

(a) To ten grams (0.029 mol) of diethyl (3-chloro-5-fluorophenyl)-aminomercaptomethylene-malonate were added 20 ml of acetonitrile and 6.0 grams (0.043 mol) of anhydrous potassium carbonate, then 4.5 grams (0.029 mol) of p-methoxybenzyl chloride was dropped thereinto during about ten minutes at room temperature with stirring and, when the dropping was completed, the mixture was stirred at room temperature for three hours. Anhydrous potassium carbonate was filtered out and washed with a small amount of acetonitrile. Washings were combined, acetonitrile was evaporated therefrom under reduced pressure, the residue was dissolved in ethyl acetate, the solution was washed with water, and dried on magnesium sulfate.

Ethyl acetate was evaporated therefrom to give 14.0 grams of yellow oil. The yellow oil was subjected to a column chromatography on silica gel (Wakogel C—300) and eluted with benzene to give diethyl (3-chloro-4-fluorophenyl)amino(4-methoxybenzyl)-thiomethylenemalonate. Pale yellow oil. Yield 10.9 grams (81 percent). Elementary analysis calculated as $C_{22}H_{23}ClFNO_5S$: C 56.47, H 4.95, N 2.99; Found: C 56.71, H 5.06, N 3.00.

Infrared absorption spectra (liquid memebrane, $cm^{-1}$): 1730, 1660, 1610, 1510

Nuclear magnetic resonance δ ($CDCl_3$): 1.27 (6H, triplet, $CO_2CH_2\underline{CH_3}$ × 2), 3.60 (2H, singlet, $SCH_2$—), 3.72 (3H, singlet, $O\underline{CH_3}$), 4.18 (4H, quartet, $O\underline{CH_2}CH_3$ × 2), 6.58—7.45 (7H, multiplet, aromatic ring-H), 10.45 (1H, wide singlet, N$\underline{H}$), vanished when $D_2O$ was added thereto.

(b) To 7.2 grams (0.0154 mol) of diethyl (3-chloro-4-fluorophenyl) amino-(4-methoxybenzyl)-thiomethylenemalonate was added 14.4 grams of biphenyl ether and the mixture was heated with stirring for three minutes at 250°C. After it was cooled to a room temperature, 30 ml of n-hexane was added thereto so that crystals were appeared. The crystals were collected by filtration, washed with n-hexane and recrystallized from isopropyl ether to give ethyl 7-chloro-6-fluoro-2-(4-methoxybenzylthio)-4-oxo-1,4-dihydroquinoline-3-carboxylate. Yield 4.3 grams (66 percent) Melting point 142°C. Elementary analysis calculated as $C_{20}H_{17}ClFNO_4S$: C 56.94, H 4.06, N 3.32; Found: C 56.98, H 4.26, N 3.68

Infrared absorption spectra (KBr, $cm^{-1}$): 1655, 1626, 1592, 1556

Nuclear magnetic resonance spectra δ ($CDCl_3$): 1.45 (3H, triplet, $OCH_2\underline{CH_3}$), 3.74 (3H, singlet, $O\underline{CH_3}$), 4.38 (2H, singlet, $S\underline{CH_2}$), 4.45 (2H, quartet, $O\underline{CH_2}CH_3$), 6.75, 7.32 (4H, doublet, doublet, aromatic ring), 7.75 (1H, doublet, $C_5$—H), 7.78 (1H, singlet, $C_8$—H), 13.17 (1H, wide singlet, N$\underline{H}$), disappeared by addition of $D_2O$.

(c) To 1.7 ml (0.01896 mol) of trifluoromethane sulfonic acid were added 2.06 ml (0.0189 mol) of anisole and 8.8 ml (0.11378 mol) of trifluoroacetic acid and the mixture was cooled at 0°C in an ice-sodium chloride bath. With stirring, 2.0 grams (0.0047 mol) of ethyl 7-chloro-6-fluoro-2-(4-methoxybenzylthio)-4-oxo-dihydroquinoline-3-carboxylate was added thereto and the mixture was stirred for about twenty minutes. Then trifluoroacetic acid and anisole were evaporated therefrom under reduced pressure and 15 grams of ice water was added to the residue. Yellow crystals appearing immediately. The crystals were collected by filtration, washed with water, dried, and recrystallized from dimethyl formamide and ether to give ethyl 7-chloro-6-fluoro-2-mercapto-4-oxo-1,4-dihydroquinoline-3-carboxylate. Yellow crystals. Yield 1.4 grams (98 percent). Melting point 221 to 223°C (decomposition). Elementary analysis calculated at $C_{12}H_{19}ClFNO_3S$: C 47.—7, H 3.01, N 4.64; Found: C 47.81, H 3.25, N 4.81

Infrared absorption spectra (KBr, $cm^{-1}$): 3100, 2700—1940, 1643, 1615, 1590

Nuclear magnetic resonance spectra δ (DMF-$d_6$): 1.28 (3H, triplet, $OCH_2\underline{CH_3}$), 4.25 (2H, quartet, $O\underline{CH_2}CH_3$), 7.66 (1H, doublet, $C_8$—H), 7.89 (1H, doublet, $C_5$—H), 13.15 (1H, wide singlet) disappeared by addition of $D_2O$.

(d) Three grams (0.01 mol) of ethyl 7-chloro-6-fluoro-2-mercapto-4-oxo-1,4-dihydroquinoline-3-carboxylate was dissolved in 10 ml of dimethyl formamide, then 4.1 grams (0.03 mol) of anhydrous potassium carbonate was added thereto and, with stirring at a room temperature, 1.9 grams (0.01 mol) of ethylene dibromide was dropped thereinto. The mixture was stirred at a room temperature overnight. The content was concentrated under reduced pressure and water was added to the residue. Crystals separated out therefrom were collected by filtration immediately, washed with water, dried and the crystals were recrystallized from ethanol to give the title compound. Colorless crystals. Yield 3.5 grams (94 percent). All of melting point, elementary analysis data, infrared absorption spectra, and nuclear magnetic resonance spectra coincided with those in example 15.

Example 25

Ethyl 6-chloro-7-fluoro-5-oxo-1,2-dihydro-5H-thiazolo(3,2-a)-quinoline-4-carboxylate and ethyl 8-chloro-7-fluoro-5-oxo-1,2-dihydro-5H-thiazolo(3,2-a)-quinoline-4-carboxylate

(a) Two hundred and thirty one mg (0.53 mmol) of diethyl (2-acetoxyethyl)thio-(3-chloro-4-fluorophenyl)-aminomethylenemalonate was dissolved in 0.5 ml of diphenyl ether and the mixture was heated with stirring at 250°C for five minutes. After cooled, insoluble matters were collected by filtration, washed with ether and air dried to give 82 mg of pale yellow powders. The powders were subjected to separation and purification by a silica gel column chromatography to give 12 mg (6.6 percent) of ethyl 6-chloro-7-fluoro-5-oxo-1,2-dihydro-5H-thiazolo(3,2-a)-quinoline-4-carboxylate and 48 mg (26.2 percent) of ethyl 8-chloro-7-fluoro-5-oxo-1,2-dihydroxy-5H-thiazolo(3,2-a)-quinoline-4-carboxylate.

(b) Diethyl (3-chloro-4-fluorophenyl)amino-(2-trimethylsilyloxyethyl)-thiomethylenemalonate (173 mg, 0.37 mmol) was dissolved in 0.4 ml of diphenyl ether and the solution was heated with stirring at 250°C for five minutes. After cooled, insoluble matters were collected by filtration, washed with ether and air dried to give 93 mg of pale yellow powders. The powders were subjected separation and purification by a silica gel column chromatography to give 9 mg (7.0 percent) of ethyl 6-chloro-7-fluoro-5-oxo-1,2-dihydro-5H-thiazolo(3,2-a)-quinoline-4-carboxylate and 76 mg (59.3 percent) of ethyl 8-chloro-7-fluoro-5-oxo-1,2-dihydro-5H-thiazolo(3,2-a)-quinoline-4-carboxylate.

(c) Two hundred and thirty eight mg (0.5 mmol) of diethyl (3-chloro-4-fluorophenyl)-amino-(2-(2-tetrahydropyranyloxy)ethyl)-thiomethylene malonate was dissolved in 0.6 ml of diphenyl ether and the solution was heated with stirring at 250°C for five minutes. After cooled, the insoluble matters appeared were collected by filtration, washed with ether, and air dried to give 65 mg of yellow powder. The powder was subjected to separation and purification by a silica gel column chromatography to give 7 mg (4.0 percent) of ethyl 6-chloro-7-fluoro-5-oxo-1,2-dihydro-5H-thiazolo(3,2-a)-quinoline-4-carboxylate and 43 mg (25.0 percent) of ethyl 8-chloro-7-fluoro-5-oxo-1,2-dihydro-5H-thiazolo-(3,2-a) quinoline-4-carboxylate.

Physical constants of the above compounds were identical with those in compounds of Example 15.

Another aspect of the present invention relates to novel substituted quinolinecarboxylic acid derivatives having antibacterial activity. More particularly, it relates to compounds represented by the following general formula (A) or pharmaceutically acceptable salts thereof:

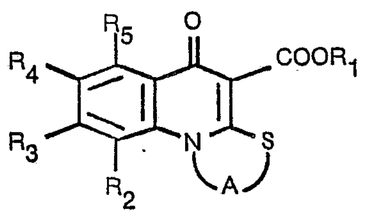

(A)

in which:

A is saturated or unsaturated hydrocarbon chain with 1 to 5 carbon atoms which may optionally be substituted with ($C_1$—$C_2$)-alkyl, hydroxyl, phenyl, ethoxy, phenoxy, phenylthio, halogen, halo-substituted methyl, methyl substituted with dimethylamino or with methylpiperazino, ethoxycarbonyl-substituted ($C_1$—$C_2$)-alkyl, carboxysubstituted ($C_1$—$C_2$)-alkyl, methoxy-substituted methyl, tetrahydropyranyloxymethyl, hydroxy-substituted methyl, ($C_1$—$C_2$)-acyloxy-substituted methyl, phenylamino, carboxy, nitro, cyano, carbonyl, thiocarbonyl or imino group, wherein A may form a ring,

$R_1$ is hydrogen, lithium, sodium, potassium, calcium, methyl, ethyl, pivaloyloxymethyl or phthalidyl,

$R_2$, $R_3$, $R_4$ and $R_5$ are same or different and they are hydrogen, fluorine, chlorine, bromine, hydroxy, ethoxy or

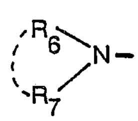

wherein

$R_3$and $R_4$ may form a ring and wherein

$R_6$ and $R_7$ are same or different and they form five to seven-membered heterocyclic ring together with adjacent nitrogen atoms, wherein said heterocyclic ring may contain other hetero atom(s), may have substituent(s) or may form a salt.

Examples of pharmaceutically acceptable salts of the above compound (A) are, for example, metal salts such as lithium salt, sodium salt, potassium salt, calcium salt and the like; salts with organic bases such as salt with ethanolamine, salt with diethanolamine and the like; salts with inorganic acids such as hydrochloride, sulfate, phophate and the like; and salts with organic acids such as acetate, methanesulfonate, succinate, lactate and the like.

In recent days, nalidixic acid, piromidic acid and pipemidic acid have been widely used as synthetic antibacterial agents for the therapy of diseases infected by Gram negative bacteria. However such agents are not satisfactory for treatment of diseases infected by Pseudomonas aeruginosa or by Gram positive bacteria which are intractable diseases and have been increasing in recent days. In order to straighten out this problem, various kinds of compounds have been synthesized and their antibacterial activities have been tested against various bacteria. Examples of improved types of the known synthetic antibacterial agents are 6-halogeno-1-substituted-7-(4-substituted piperazino)-4-oxo-1,4-dihydroquinoline-3-carboxylic acid derivatives (cf. Japanese laid open applications Sho 53-65887, 53-141286, 54-66686 and 55-47658) and 6-fluoro-1,8-naphthylidine derivatives (cf. Japanese laid open application Sho-55-83785) and they are now in a stage of development. However, among many synthetic antibacterial agents which are known or in a developing stage, there is no substituted quinolinecarboxylic acid having a substituent at 2-position.

Journal of Medicinal Chemistry (volume 20, page 791, 1977), the same journal (volume 21, page 485, 1978) and Journal of Heterocyclic Chemistry (volume 17 page 1972, 1980) disclosed the compounds of the above type having methyl or hydroxyl group at 2-position or the compounds in which a nitrogen atom at 1-position is bonded with the 2-position forming a ring. However, all of them do not exhibit marked antibacterial activity.

In order to find compounds having antibacterial activity out of the said 2-substituted compounds, the present inventors synthesized the compounds (A) which have not been known in past literatures yet and which are composed of entirely new skeleton. As a result of the tests, it has now been found that such new compounds exhibit far more marked antibacterial activity not only than the known synthetic antibacterial drugs but also than synthetic antibacterials now in a stage of development.

Antibacterial activity of the compounds (A) is that they are effective not only for Pseudomonas aeruginosa but also for both Gram positive and negative bacteria in such small doses that have not been known in literatures. The said compounds (A) can be safely administered to human being by oral route or as injections.

Examples of manufacturing methods of the present invention compounds are given in a scheme for the purpose of explanation. First, ethyl 4-oxo-1,4-dihydroquinoline-3-carboxylate derivatives (G) which are very important intermediates in the present invention are, for instance, synthesized by the following reaction steps.

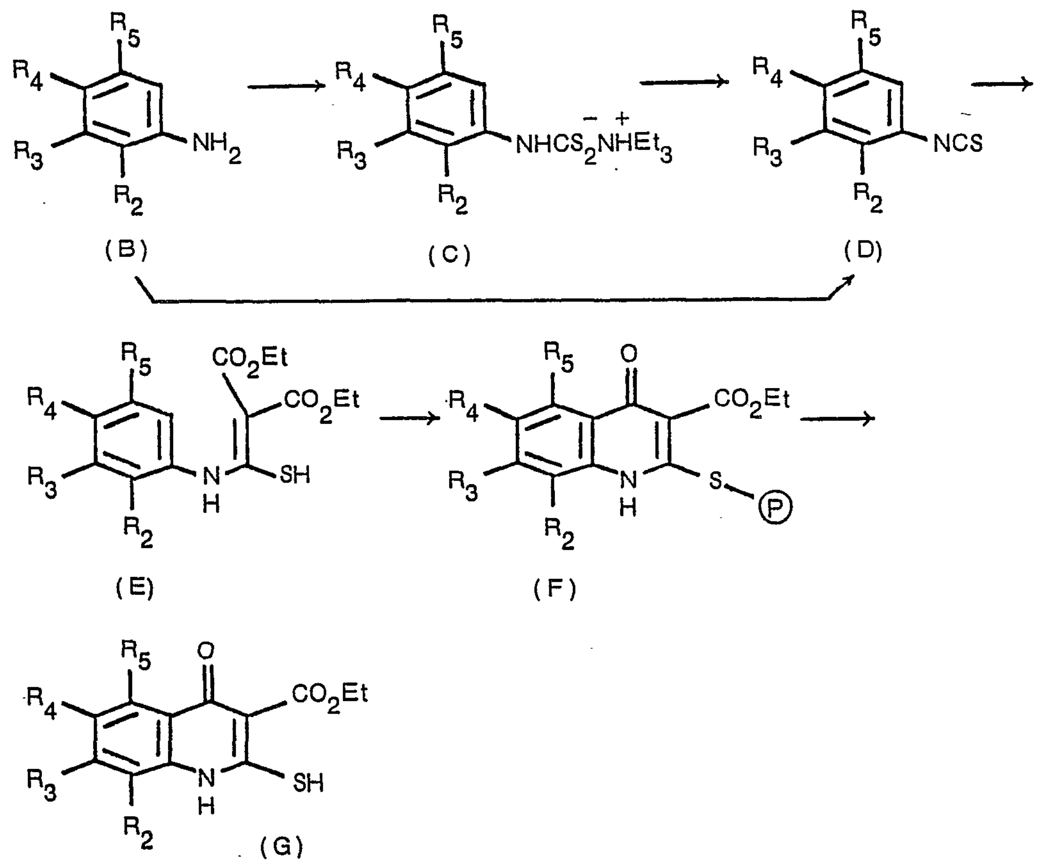

(B) (C) (D)

(E) (F)

(G)

In the above formulas, $R_2$, $R_3$, $R_4$ and $R_5$ are the same as already defined and Ⓟ represents a protective group.

Thus, substituted aniline (B) in a suitable solvent such as benzene or without solvent is made to react with carbon disulfide with cooling in the presence of a large excess of triethylamine or in the presence of amine compounds other than triethylamine and alkali metals to give substituted phenyldithiocarbamic acid salt (C). This is then made to react with ethyl chlorocarbonate in a solvent such as chloroform or methylene chloride in the presence of triethylamine or is made to react with copper sulfate, lead nitrate, iron sulfate, zinc sulfate or the like to give substituted phenyl isothiocyanate (D). The (D) can also be manufactured directly from (B) by a known method disclosed in Organic Syntheses, Collective Volume I, page 447.

Reaction of the resulting (D) with sodium salt of diethyl malonate to afford diethyl substituted pnenylaminomerceptomethylenemalonate (E). This is protected with protective groups generally used for protecting thiols (cf. The Chemistry of the Thio Group, Part Two, edited by Soul Patai, published by John Wiley and Sons, page 669, 1974) or is protected by substituted alkyls by the known method per se and the resulting protected compound is heated in a high boiling solvent such as dichlorobenzene, tetraline, diphenyl ether, diethylglycol dimethyl ether, and the like so that ring closure reaction takes place to give the compound (F).

As to protective groups for sulfur generally used, it is useful to use the following as Ⓟ. They are substituted benzyl, alkoxymethyl, 2,4-dinitrophenyl, disulfide as a dimer of (E), alkylthiomethyl, substituted carbamoyl, diphenylmethyl, triphenylmethyl, picolyl, acetamidomethyl, beta,beta,beta-trifluoro-alpha-acylaminoethyl, beta,beta,diethoxycarbonylethyl, acetyl, benzoyl, benzyloxycarbonyl, tetrahydropyranyl, benzylthiomethyl, phenylthiomethyl, isobutyloxymethyl, and the like. Taking substituted benzyl as an example, the reaction will be illustrated in some detail.

Thus, p-methoxybenzyl chloride is made to react with (E) in the presence of an alkali such as sodium carbonate or potassium carbonate in a solvent such as acetonitrile, dimethyl formamide, tetrahydrofuran and the like and the resulting product is heated in a high boiling solvent such as diphenyl ether so that a ring closure takes places to give the desired (F) quantitatively.

When the resulting (F) is treated so as to remove protective groups, then (G) which is very important intermediate compound is obtained in high yields. For example, when p-methoxybenzyl derivative of (F) is used, it is treated with methanesulfonic acid, trifluoromethane sulfonic acid, trifluoroacetic acid, or a mixture thereof and anisole with cooling whereupon the protective group can be removed in high yields. The resulting (G) is, for example, made to react with dihalide represented by a formula (H) (in which Y is halogen and A is the same as already defined) in dimethyl formamide solvent in the presence of potassium carbonate to give a group of compounds (H) wich possess sulfur atoms and nitrogen atom of 2-mercapto-quinolone skeleton in the same ring. Or, the reaction of (G) with halogeno-substituted aldehyde, halogeno-substituted ketone, halogeno-substituted acetal, halogeno-substituted ketal, halogeno-substituted cyclic ketone, etc. represented by the general formulas (P) and (O) gives a group of compounds (I) and (L) which possess hydroxyl or alkoxy group as a substituent in the said ring.

Treatment of those (I) and (L) with mineral acids such as sulfuric acid, hydrochloric acid, nitric acid, and the like to give a group of compounds (K) and (M) which possess unsaturated bond in the said ring. In the formulas, $R_2$, $R_3$, $R_4$, $R_5$ and A are the same as already defined. $R_8$ is hydrogen, lower alkyl, halogeno-substituted alkyl, substituted phenyl, substituted benzyl, substituted alkoxy, substituted phenoxy, substituted alkylthio, substituted phenylthio, or other thio group having heterocyclic ring such as tetrazol or thiadiazol. $R_9$, $R_{10}$, $R_{11}$, $R_{12}$ and $R_{13}$ are hydrogen, lower alkyl or substituted phenyl. Z is oxygen or alkoxy. Y is halogen. n and o are intergers of 0, 1 or 2. When n is zero, there is a five-membered ring and forms a thiazoloquinoline ring and, therefore, one of $R_{10}$ and $R_{11}$ will not be present. When Z is oxygen, the Z in (I) will become ZH by incorporating hydrogen atom. With reference to the position of a double bond in (M), it will be that, when $R_{12}$ is H, it is in "endo" and $R_{12}$ will not be present and, when $R_{12}$ is other than H, it is in "exo", and $R_{12}$ will be present.

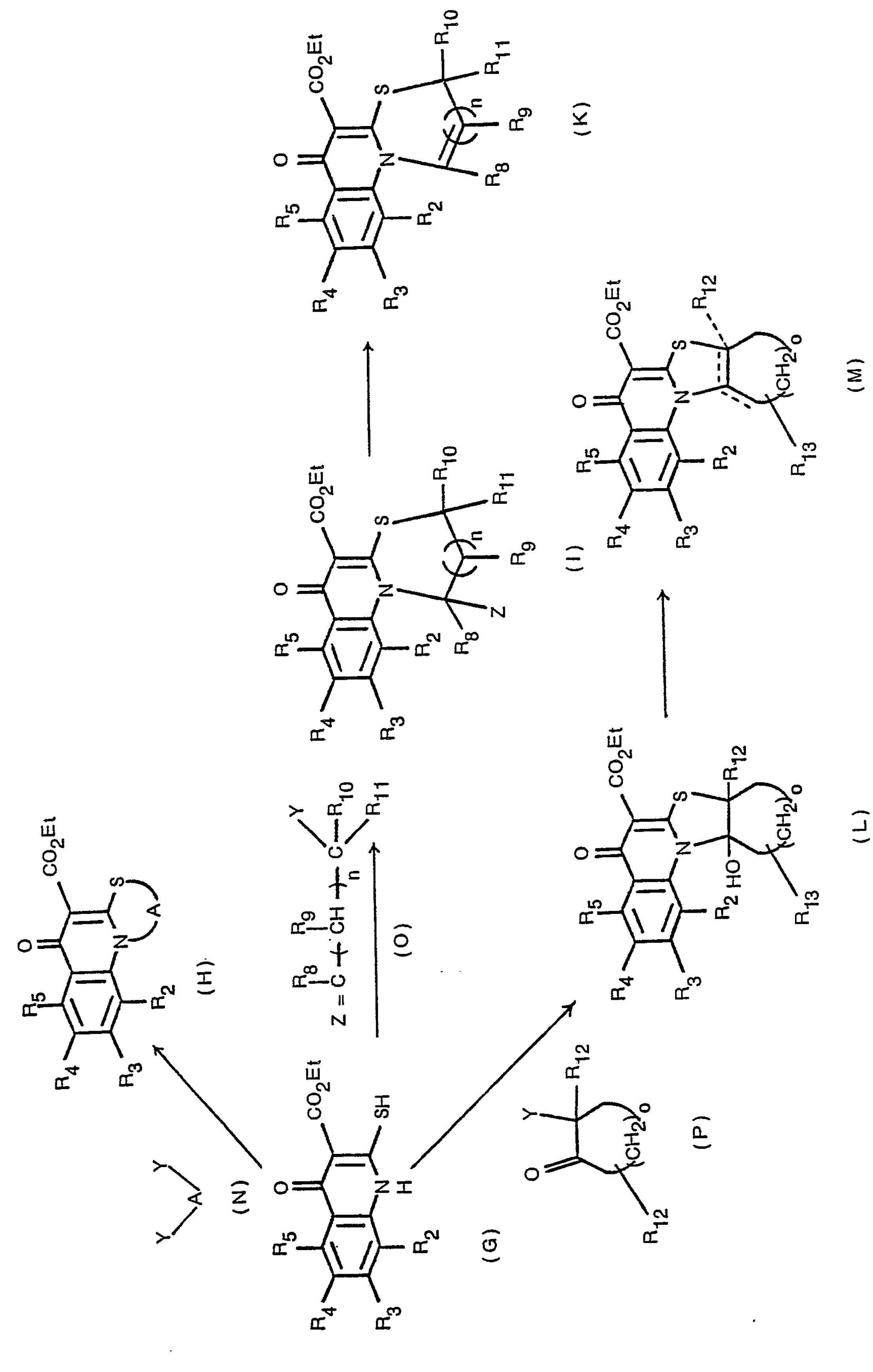

(G) (H) (N) (O) (I) (K) (P) (L) (M)

The resulting (H), (I), (K), (L), and (M) are hydrolyzed with sodium hydroxide or potassium hydroxide in an alcohol or hydrolyzed with concentrated sulfuric acid or concentrated hydrochloric acid to give desired carboxylic acids in high yields.

Those carboxylic acids derived from general formulas (H) to (M) are, in case $R_3$ is halogen, made to react with secondary amines of general formula (Q),

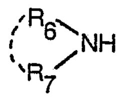

(Q) to give a group of compounds represented by general formulas (R) to (V). In the above formula, $R_6$ and $R_7$ are same or different and they are lower alkyl or a heterocyclic ring of five to seven members together with adjacent nitrogen atom. The said heterocyclic ring may contain other heteroatoms such as nitrogen, oxygen, sulfur, etc. as its constituents, may possess substituent(s) or may form a salt.

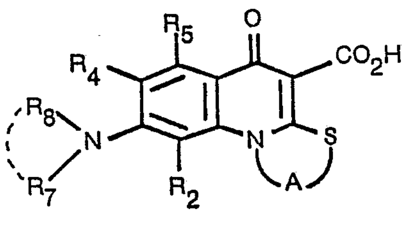

(R)

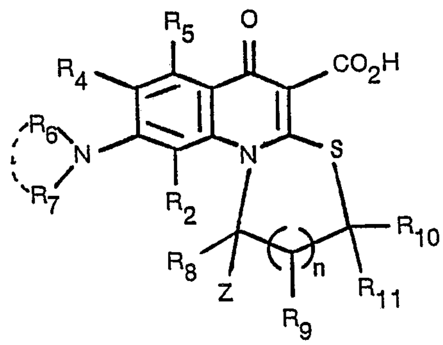

(S)

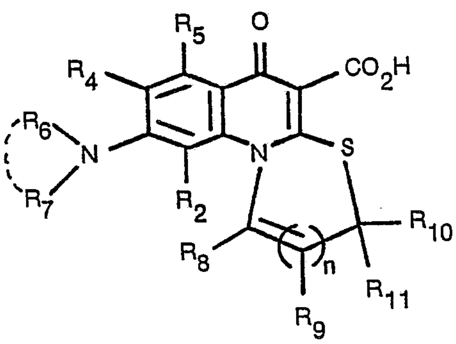

(T)

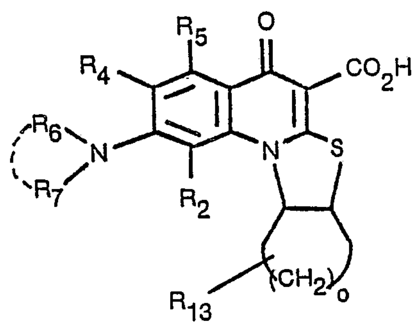

(U)

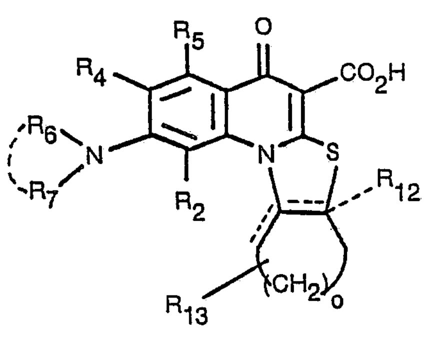

(V)

The carboxylic acids derived from (I) to (M) can be condensed with secondary amines represented by the general formula (Q) also when $R_5$ is halogen. Further, when $R_4$ is fluorine and $R_3$ is chlorine, the carboxylic acids are made to react with a compound represented by a general formula (Q) under controlled reaction conditions to give a group of compounds in which

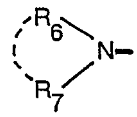 group

is introduced in $R_4$.

When the resulting compound represented by a general formula (W)

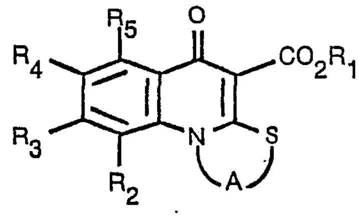

(W)

is oxidized by the known method per se to give the corresponding sulfoxide and sulfone. Also, it can be changed to the corresponding sulfylimine, sulfoxyimine, etc. by the known method per se. Anyway, the compound represented by a general formula (I) as well as salts thereof can be manufactured in high yields in low cost.

An alternative way is that (H) and (Y) are synthesized starting from the compound (E) by the following reaction steps. In the formulas, meanings of A, $R_2$, $R_3$, $R_4$ and $R_5$ are the same as already defined.

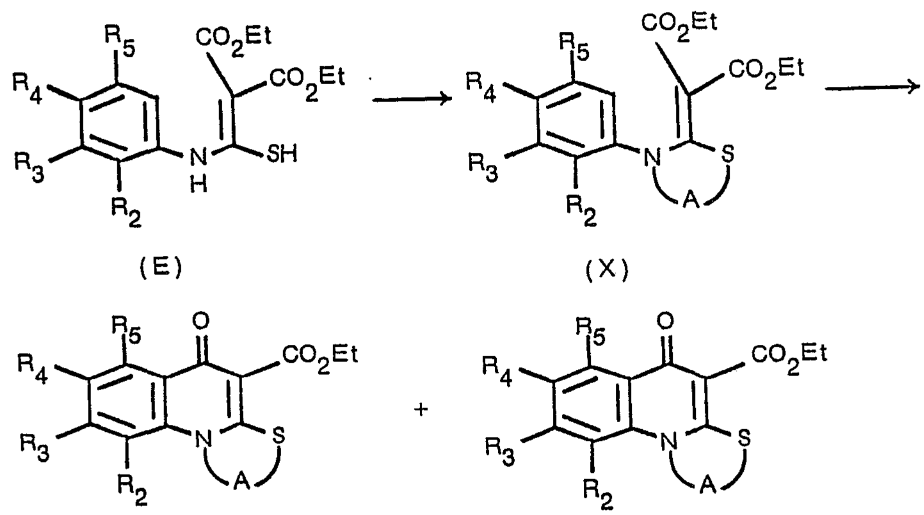

(E) (X)

(H) + (Y)

Thus, (E) is made to react, instead of with a protective group of sulfur, with dihalogeno compound represented by a general formula (N) in dimethyl formamide solvent in the presence of potassium carbonate or is made to react with halogeno-substituted aldehyde, halogeno-substituted ketone, halogeno-substituted acetal, halogeno-substituted ketal, or halogeno-substituted cyclic ketone represented by a general formula (O) and (P) followed by a treatment with sulfuric acid or with hydrochloric acid to afford (X). Cyclization of the resulting (X) can be done by the known various cyclization reactions. Examples of them are cyclization by heating, cyclization by the use of acidic substances such as phosphorus oxychloride, phosphorus pentachloride, phosphorus trichloride, thionyl chloride, concentrated sulfuric acid, polyphosphoric acid, polyphophoric acid esters, and the like, etc. Cyclization by the use of acidic substances may, for example, be done by the use of equimolar to a large excess of acidic substances to the compound (X), preferably 10 to 20 molar substances being used, by heating generally at 100 to 150°C for 0.5 to 2 hours. In this case, when $R_2$ is hydrogen, (Y) besides (H) will be formed as cyclization products. Separation of (H) and (Y) from their mixture can be done by the conventional method such as, for example, recrystallization method or column chromatography.

When the present invention compound (A) in which A is ethylene is aimed, the following routes are alsó applicable.

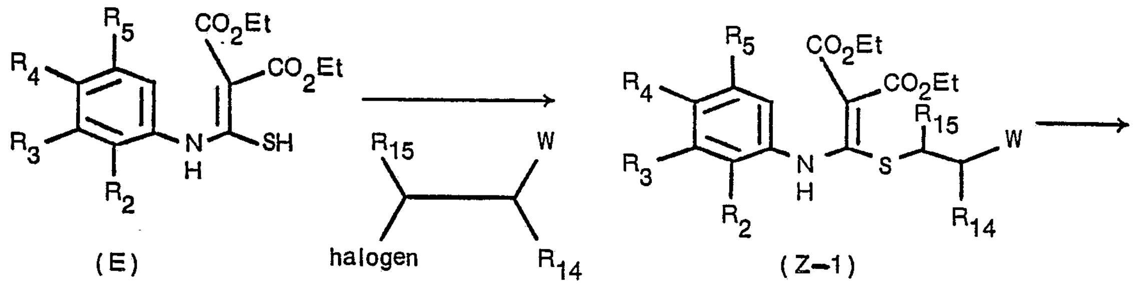

(E) (Z) (Z—1)

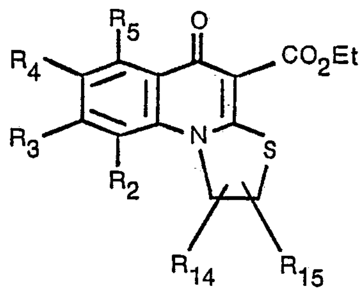

(Z—2)

In the above formulas, meanings of $R_2$, $R_3$, $R_4$ and $R_5$ are the same as already defined. $R_{14}$ and $R_{15}$ are hydrogen or lower alkyl. W is hydroxyl, alkoxy, acetoxy, trimethylsilyloxy, alkylthio or substituted amino.

Thus, a compound represented by a general formula (E) is made to react with substituted ethylene halide represented by a general formula (Z) to afford a compound represented by a formula (Z—1). By heating the resulting compound in high boiling solvent such as dichlorobenzene, tetraline, diphenyl ether, diethyleneglycol dimethyl ether, and the like, a compound represented by a general formula (Z—2) can be obtained in high yields.

Another convenient method in which no protective group for sulfur is used is as follows. Thus, the compound (E) is made to react with a compound of a formula (O) (halogeno-substituted aldehyde, halogeno-substituted ketone, halogeno-substituted acetal, halogeno-substituted ketal, and the like) by the use of potassium carbonate as acid-removing agent in a solvent such as dimethyl formamide or acetonitrile to give a compound of a formula (Z—3). By heating the (Z—3) is a high boiling solvent such as dichlorobenzene, tetraline, diphenyl ether, diethylene glycol dimethyl ether, and the like, a compound of a formula (Z—4) can be obtained in high yields.

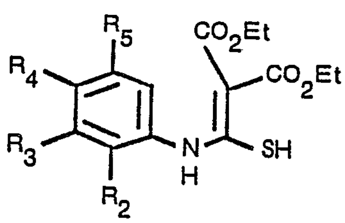

(E)

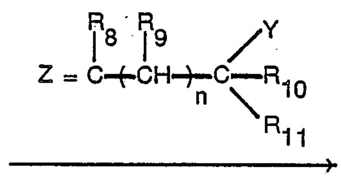

(O)

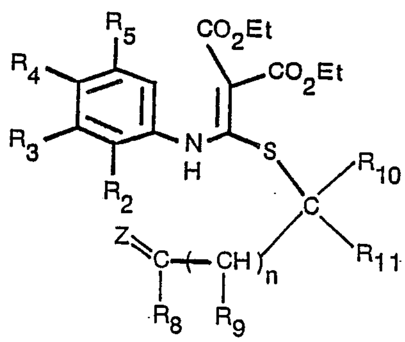

(Z—3)

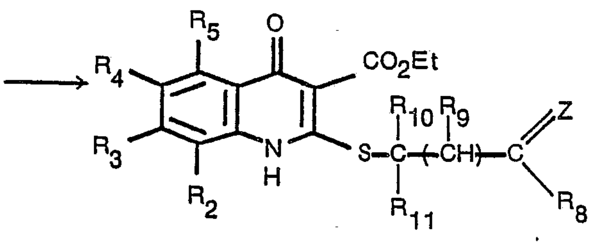

(Z—4)

When the resulting (Z—4) is treated with acidic substances such as concentrated hydrochloric acid or concentrated sulfuric acid, the afore mentioned (I) and (K) can be manufactured in high yields in low cost.

In case the compound of a formula (O) is dichloroacetone, the resulting (Z—5) is made to react with various kinds of nucleophilic reagents (Nu) such as various amines, mercaptane, alkoxy, and the like to afford (Z—6), then it is hydrolyzed, and the resulting corresponding carboxylic acid is made to react with (Q) to give the desired (Z—7) in high yields.

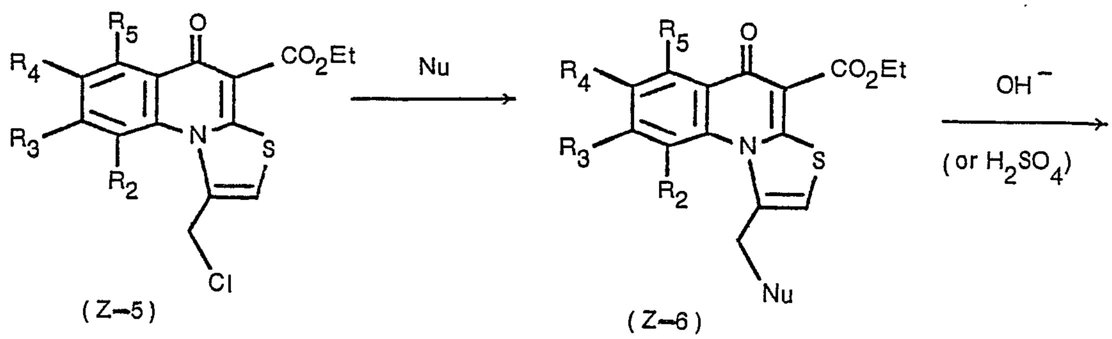


(Z—5) (Z—6)

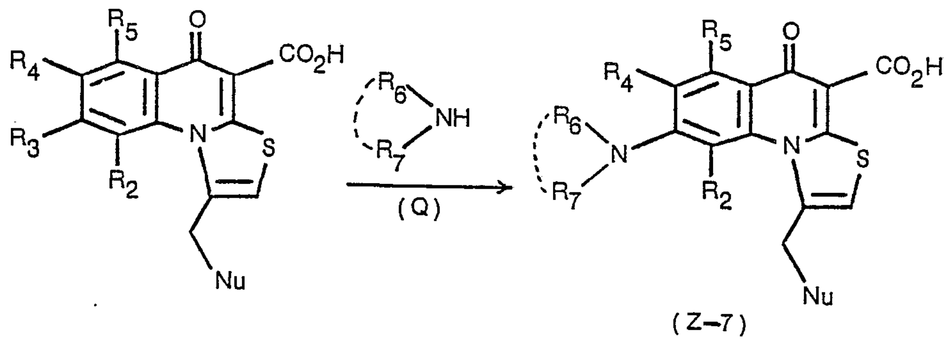


(Z—7)

Examples of the present invention compounds manufactured by the above methods are as follows.

7-Fluoro-5-oxo-1-phenoxy-8-(1-piperazinyl)-1,2-dihydro-5H-thiazolo(3,2-a)quinoline-4-carboxylic acid

7-Fluoro-1-methylthio-5-oxo-8-(1-piperazinyl)-1,2-dihydro-5H-thiazolo(3,2-a)quinoline-4-carboxylic acid

7-Fluoro-5-oxo-1-phenylthio-8-(1-piperazinyl)-1,2-dihydro-5H-thiazolo(3,2-a)quinoline-4-carboxylic acid

7-Fluoro-1-methylthiomethyl-5-oxo-8-(1-piperazinyl)-1,2-dihydro-5H-thiazolo(3,2-a)quinoline-4-carboxylic acid

7-Fluoro-5-oxo-8-(1-piperazinyl)-1,2-dihydro-5H-thiazolo(3,2-a)quinoline-2,4-carboxylic acid

7-Fluoro-1-hydroxy-5-oxo-8-(1-piperazinyl)-5H-thiazolo(3,2-a)quinoline-4-carboxylic acid

7-Fluoro-1-methoxy-5-oxo-8-(1-piperazinyl)-5H-thiazo-o(3,2-a)quinoline-4-carboxylic acid

7-Fluoro-1-methylthio-5-oxo-8-(1-piperazinyl)-5H-thiazolo(3,2-a)quinoline-4-carboxylic acid

1-Chloro-7-fluoro-5-oxo-8-(1-piperazinyl)-5H-thiazolo(3,2-a)quinoline-4-carboxylic acid

7-Fluoro-1-methylthiomethyl-5-oxo-8-(1-piperazinyl)-5H-thiazolo(3,2-a)quinoline-4-carboxylic acid

1-Dimethylamino-7-fluoro-5-oxo-8-(1-piperazinyl)-5H-thiazolo(3,2-a)quinoline-4-carboxylic acid

7-Fluoro-5-oxo-8-(1-piperazinyl)-5H-thiazolo(3,2-a)quinoline-1,4-dicarboxylic acid

7-Fluoro-1-nitro-5-oxo-8-(1-piperazinyl)-5H-thiazolo(3,2-a)quinoline-4-carboxylic acid

1-Cyano-7-fluoro-5-oxo-8-(1-piperazinyl)-5H-thiazolo(3,2-a)quinoline-4-carboxylic acid

7-Fluoro-2-hydroxy-5-oxo-8-(1-piperazinyl)-5H-thiazolo(3,2-a)quinoline-4-carboxylic acid

7-Fluoro-2-methoxy-5-oxo-8-(1-piperazinyl)-5H-thiazolo(3,2-a)quinoline-4-carboxylic acid

7-Fluoro-5-oxo-8-(1-piperazinyl)-5H-thiazolo(3,2-a)quinoline-2,4-carboxylic acid

Biological properties of the present invention compounds are as follows.

(1) Antibacterial spectra of the present invention compounds.

Antibacterial activity of the compounds according to Examples 39, 38, 47b, 46 and 40 has been carried out in accordance with the method of Japan Chemotherapeutic Society (cf. Nippon Kagaku Ryoho Gakkai Shi, volume 29, number 1, pages 76 to 79, 1981) and the result is given in Table 1.

As apparent from the table, the present invention compounds exhibit strong activity against both Gram positive and Gram negative bacteria including Pseudomonas aeruginosa and showed stronger antibacterial activity than 1-ethyl-6-fluoro-7-(1-piperazinyl)-4-oxo-1,4-dihydroquinoline-3-carboxylic acid which was used as a comparison.

TABLE 1

| Names of Microorganisms Tested | Gram | Names of Compounds Tested (indicated by example numbers) Minimum Growth Inhibiting Concn (μg/ml) | | | | | |
|---|---|---|---|---|---|---|---|
| | | 39 | 38 | 47b | 46 | 40 | Comparison Compd |
| Staphylococcus aureus 209–PJC | + | 0.1 | 0.025 | 0.013 | 0.025 | 0.025 | 0.2 |
| Staphylococcus aureus Smith | + | 0.05 | 0.05 | 0.053 | 0.013 | 0.013 | 0.39 |
| Micrococcus luteus ATCC 9341 | + | 0.78 | 0.78 | 0.78 | 0.39 | 1.56 | 12.5 |
| Streptococcus pyogenes S–23 * | + | 0.2 | 0.1 | 0.2 | 0.1 | 0.1 | 0.78 |
| Streptococcus pneumoniae Type I * | + | 0.2 | 0.1 | 0.39 | 0.2 | 0.39 | 1.56 |
| Bacillus subtilis ATCC 6633 * | + | 0.0063 | 0.0063 | 0.0008 | 0.0063 | 0.0063 | 0.2 |
| Listeria monocytogenes RIMD 1205020 | + | 1.56 | 0.2 | 0.2 | 0.2 | 0.39 | 1.56 |
| Escherichia coli NIHJ JC–2 | – | 0.1 | 0.05 | 0.025 | 0.025 | 0.2 | 0.05 |
| Escherichia coli KC–14 | – | 0.013 | 0.0063 | 0.013 | 0.0063 | 0.05 | 0.05 |
| Klebsiella pneumoniae K–1966 | – | 0.1 | 0.025 | 0.05 | 0.013 | 0.05 | 0.05 |
| Serracia marcescens IFO 3736 | – | 0.39 | 0.2 | 0.39 | 0.2 | 0.39 | 0.2 |
| Proteus mirabilis 181 | – | 0.013 | 0.0063 | 0.2 | 0.05 | 0.2 | 0.1 |
| Proteus vulgaris DX–19 | – | 0.0063 | 0.013 | 0.1 | 0.0016 | 0.05 | 0.05 |
| Pseudomonas aeruginosa No. 12 | – | 0.39 | 0.2 | 0.78 | 0.39 | 0.78 | 0.39 |
| Pseudomonas aeruginosa E–2 | – | 0.39 | 0.2 | 0.39 | 0.39 | 1.56 | 0.78 |
| Pseudomonas sepacia ATCC 25416 | – | 0.78 | 0.78 | 0.2 | 0.2 | 1.56 | 1.56 |
| Pseudomonas maltophilia ATCC 13637 | – | 0.0025 | 0.5 | 0.025 | 0.013 | 0.0063 | 0.2 |
| Alcaligenes faecalis ATCC 8750 | – | 0.78 | 0.78 | 0.78 | 0.78 | 0.78 | 3.13 |
| Acinetobacter calcoaceticus 54 | – | 0.39 | 0.78 | 0.2 | 0.2 | 0.39 | 3.13 |

*: 5% Horse blood added agar medium for sensitivity measurement being used.

(2) Therapeutic effect of the present invention compounds against general infection of mice.

ddY—Strain mice (male; body weights 20 ± 1 grams) of SPF were used for the experiments of therapy of infected diseases. Bacteria to be infected were all supended in 4% mucin solution and were infected by administering into abdominal cavity. Therapeutic effect was measured by the following way. Thus, the present invention compound was given for one time per os two hours after the infection and, from the survival rate after one week, 50% effective doses ($ED_{50}$) were calculated by Behrens-Kärber method. The test result of the present invention compounds against general infection diseases caused by several bacteria are given in Table 2. It has thereby been found that the present invention compounds showed stronger infection-preventive effect as compared with 1-ethyl-6-fluoro-7-(1-piperazinyl)-4-oxo-1,4-dihydroquinoline-3-carboxylic acid which was used as a comparison compound. Also it has been found that the present invention compounds exhibit marked therapeutic effect to infectious diseases by Gram positive bacteria.

TABLE 2

| Names of Bacteria to be Infected | Amount of Bacteria Infected (per mouse) | Names of Compounds Tested (indicated by example numbers) $ED_{50}$ in mg/kg 39 | 47b | 40 | Comparison Compd. |
|---|---|---|---|---|---|
| Pseudomonas aeruginosa E–2 | $2 \times 10^5$ | 16.0 | 13.1 | 10.7 | 260 |
| Escherichia coli KC–14 | $5 \times 10^4$ | 1.5 | 1.1 | 2.0 | 3.3 |
| Klebsiella pneumoniae K–1966 | $3 \times 10^3$ | —* | 2.7 | 3.3 | >13.0 |
| Serracia marcescens T–55 | $5 \times 10^6$ | — | 5.4 | — | 6.0 |
| Proteus mirabilis 181 | $2 \times 10^7$ | — | 13.0 | — | 29.0 |
| Acimetobacter calcoaceticus | $1 \times 10^7$ | — | 17.5 | >35.0 | >35.0 |
| Staphylococcus aureus Smith | $33 \times 10^6$ | — | 2.4 | — | >25.0 |

*: Not tested yet

(3) Effect of the present invention compounds against ascending urinary tract infection of mice.

ICR Female mice (body weights 20 ± 1 grams) of SPE were used in the experiments. The mice have not been given any water for seventeen hours, then compulsorily urinated, anesthesized by the administration of 60 mg/kg of pentabarbital into abdominal cavity, then $1.5 \times 10^5$ of Pseudomonas aeruginosa E-2 strain were impregnated into bladder via urinary tract, and the external urinary meatus was stopped for four hours by a pin clip whereupon the mice were infected at the ascending urinary tract.

Therapeutic effect was observed as follows. Thus, the present invention compounds were given orally once 4 hours after infection, then twice a day, and the administration was continued for three days. On the fourth day, kidney was isolated, homogenized in a physiological saline solution, and the numbers of bacteria was calculated on suitable medium such as, for example, Drigalski medium. The case where the number per one gram of kidney is less than $10^4$ is judged as "effective". The result is given in Table 3.

The present invention compound exhibited marked therapeutic effect in lower doses as compared with 1-ethyl-6-fluoro-7-(1-piperazinyl)-4-oxo-1,4-dihydroquinoline-3-carboxylic acid which was used as a comparison compound. The present invention compound used here is the compound of Example 47b.

TABLE 3

| Doses Administered (mg/kg) | (Effective Numbers/Tested Numbers) Compd. of Ex. 47b | Comparison Compd.* |
|---|---|---|
| 100 | 7/7 | 6/7 |
| 50 | 7/7 | 4/7 |
| 25 | 6/7 | 1/7 |
| 12.5 | 4/7 | 0/7 |
| 6.25 | 4/7 | 0/7 |
| 3.13 | 1/7 | 0/7 |
| 50% Effective Doses: | 8 mg/kg | 58 mg/kg |

*: 1-Ethyl-6-fluoro-7-(1-piperazinyl)-4-oxo-1,4-dihydroquinoline-3-carboxylic acid

(4) Blood concentrations of the present invention compounds (the compound of Example 47b).

In the experiment of measuring blood concentrations, ddY strain mice (male; body weights 30 ± 2 grams) were used. Sixty mg/kg of the present invention compounds were given per os. The blood concentration was calculated from calibration curves obtained by the disc method using Escherichia coli NIHJ strain as tested bacteria and diluting the present invention compounds in mice serum. In Table 4 were given the concentrations in blood serum on the elapse of time of the present invention compound (the compound of Example 47b). It will be apparent from the table that the present invention compound administered orally gave high and continued blood concentrations promptly.

TABLE 4

| Time Elapsed (hours) | Concentrations in Blood Serum (µg/ml) Average ± Standard Error Compd. of Ex. 47b | Comparison Compd.* |
|---|---|---|
| 0.25 | 3.4 ± 0.82 | 0.64 ± 0.09 |
| 0.5 | 5.4 ± 0.32 | 0.90 ± 0.29 |
| 1 | 4.2 ± 0.70 | 0.58 ± 0.10 |
| 2 | 3.3 ± 0.37 | 0.33 ± 0.15 |
| 4 | 2.5 ± 0.23 | 0.09 ± 0.01 |

*: 1-Ethyl-6-fluoro-7-(1-piperazinyl)-4-oxo-1,4-dihydroquinoline-3-carboxylic acid

(5) Excretion into urine and recovery of the present invention compound (the compound of Example 47b).

In the measurement of excretion into urine, ddY strain mice (male; body weights 30 ± 2 grams) were used. Sixty mg/kg of the present invention compound (the compound of Example 47b) was administered per os and the mice were fed in metabolic cages and urine was taken out from the mice.

It has been thereby found that the present invention compound (the compound of Example 47b) was metabolized to another compound (the compound of Example 46) and was excreted into urine almost completely. Therefore, in the quantitative determination, the calibration curve of the present invention compound (the compound of Example 46) calculated by the disc method using Escherichia coli NIHJ strain and using the compound dissolved in phosphoric acid buffer (pH 8.0; 1/15 M) is used. In Table 5 were given excretion into urine on the elapse of time, total recovery amount and recovery rate of the present invention compound (the compound of Example 47b). It is apparent that the present invention compound is excreted into urine promptly and in high concentration which is continued and exhibits high recovery rate.

TABLE 5

| | Amount excreted into Urine (μg) Average ± Standard Error | |
|---|---|---|
| | Compd. of Ex. 47b | Comparison Compd.* |
| 0—3 hrs | 268.1 ± 84.4 | 60.25 ± 17.9 |
| 3—6 | 108.3 ± 33.1 | 29.25 ± 9.0 |
| 6—24 | 437.5 ± 21.8 | 19.78 ± 6.6 |
| Total Recovery | 813.9 μg | 109.3 μg |
| Recovery Rate | 40.7% | 5.46% |

*: 1-Ethyl-6-fluoro-7-(1-piperazinyl)-4-oxo-1,4-dihydroquinoline-3-carboxylic acid

(6) Acute toxicity of the present invention compounds by oral administration.

Ten ddY strain male mice (body weights: 20 ± 1 grams) were used in a group and the acute toxicity tests of the present invention compounds by oral administration were carried out. The result is given in Table 6, There is no example in which mice were died during the tests by administration of 2000 mg/kg.

TABLE 6

| Compounds Tested | 50% Lethal Doses ($LD_{50}$) in mg/kg |
|---|---|
| Compd. of Ex. 39 | more than 2000 |
| Compd. of Ex. 38 | more than 2000 |
| Compd. of Ex. 47b | more than 2000 |
| Compd. of Ex. 46 | more than 2000 |
| Compd. of Ex. 40 | more than 2000 |

The present invention compounds and salts thereof are generally obtained in crystalline powder or in powder. Since they are with little toxicity and exhibit marked hydrophilic and olephilic properties, the present invention compounds can be used as antibacterial agents in safe by parentheral or by oral route in various dosage forms such as, for example, injection, tablet, capsule, liquid, suppository, ointment, and the like.

In preparing injections and dropping injections, the present invention compounds are, for example, dissolved in physiological saline solution or in aqueous solution of glucose or other additive(s) by the known method per se.

Tablets, capsules and the like may also be prepared by the known methods. For example, the present invention compounds are mixed with suitable solid carriers (such as, for example, diluents, bulking agents, etc.) or are absorbed therein and, if necessary, other suitable additives such as, for example, emulsifiers, dispersing agents, suspending agents, spreaders, penetrating agents, wetting agents, mucilages, stabilizers, and the like may be added thereto making, for example, into solutions, granules, emulsions, suspensions, ointments, powders, sprays, pastes, cataplasms, and the like.

Such pharmaceutical preparations are used by suitable administration routes depending upon the purpose of the administration. For example, in the case of injection solutions, they are given by parentheral route such as intravenous, hypodermic, direct application to the infected part, and the like. In the case of tablets, capsules, and the like, they are given by oral route.

Dosages to human being are, in general, about 0.1 to 150 mg/kg per day and, more preferably, about 5 to 20 mg/kg per day and may be suitably selected according to the degree of the disease, administration route, etc. Administration may be, in general, once to four times per day and may be consecutively.

Although the amount of the antibacterial agent is not to be limited, it is general that about 0.01 to 70 percent by weight of the present invention compound is contained in the preparation and, more preferably, the concentration is about 0.1 to 5 percent by weight. Administration of antifungal agents is done for once to several times per day by such means as painting, spraying, and the like.

As hereinafter, examples concerning the manufacture of the present invention compounds are given.

Example 26

Ethyl 8-chloro-7-fluoro-1-(2-tetrahydropyranyloxymethyl)-5-oxo-1,2-dihydro-5H-thiazolo(3,2-a)quinoline-4-carboxylate

A mixture of 6.02 grams (20 mmol) of ethyl 7-chloro-6-fluoro-4-hydroxy-2-mercaptoquinoline-3-carboxylate and 6.64 grams (22 mol) of 1,2-dibromo-3-(2-tetrahydropyranyloxy)-propane in 50 ml of dimethylformamide was heated at 80°C for three hours with stirring in the presence of 6.08 grams (44 mmol) of potassium carbonate (anhydrous). Dimethyl formamide was evaporated therefrom in vacuo and water was added to the residue followed by extracting with chloroform. The chloroform solution was washed with water, dried, concentrated, and purified by silica gel chromatography to give 6.53 grams of ethyl 8-chloro-7-fluoro-1-(2-tetrahydropyranyloxymethyl)-5-oxo-1,2-dihydro-4H-thiazolo(3,2-a)-quinoline-4-carboxylate. The yield was 73.9 percent. Melting point was 183 to 186°C. Elementary analysis calculated as $C_{20}H_{21}ClFNO_5S$: C 54.36, H 4.79, N 3.17; Found: C 54.18, H 4.83, N 3.34.

Infrared absorption spectra (KBr, $cm^{-1}$): 1700, 1600, 1335, 1125.

Nuclear magnetic resonance spectra δ ($CDCl_3$): 1.2—1.8 (9H, multiplet, —$OCH_2CH_3$, $CCH_2CH_2CH_2C$), 3.2—4.0 (6H, multiplet, $SCH_2$—, $OCH_2$ × 2), 4.1—4.7 (3H, multiplet, $OCH_2CH_3$,

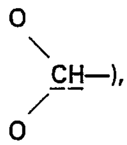

5.1—5.6 (1H, multiplet,

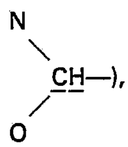

7.55 (1H, doublet, $C_9$—H), 7.98 (1H, doublet, $C_6$—H).

Example 27

8-Chloro-7-fluoro-1-hydroxymethyl-5-oxo-1,2-dihydro-5H-thiazolo(3,2-a)quinoline-4-carboxylic acid

a) In 10 ml of ethanol was dissolved 2.20 grams (4.98 mmol) of ethyl 8-chloro-7-fluoro-1-(2-tetrahydropyranyloxymethyl)-5-oxo-1,2-dihydro-5H-thiazolo(3,2-a)quinoline-4-carboxylate, then 252 mg (6.3 mmol) of sodium hydroxide and 20 ml of water were added thereto, and the mixture was heated to reflux for one hour and a half. This was then allowed to cool to room temperature, neutralized with acetic acid, crystals separated out therefrom were collected by filtration, and dried to give 2.00 grams of 8-chloro-7-fluoro-1-(tetrahydropyranyloxymethyl)-5-oxo-1,2-dihydro-5H-thiazolo(3,2-a)quinoline-4-carboxylic acid. Yield was 97.1 percent. Melting point was 241 to 246°C. Elementary analysis calculated as $C_{18}H_{17}ClFNO_5S$: C 52.24, H 4.14, N 3.38; Found: C 53.02, H 4.49, N 3.14.

Infrared absorption spectra (KBr, $cm^{-1}$): 1700, 1595, 1125.

Nuclear magnetic resonance spectra δ ($CDCl_3$): 1.2—1.8 (6H, multiplet, C—$CH_2CH_2CH_2$—C), 3.2—4.1 (6H, multiplet, S—$CH_2$, O—$CH_2$ × 2), 4.35—4.6 (1H, multiplet,

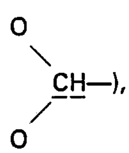

5.2—5.7 (1H, multiplet,

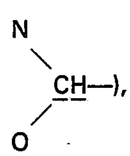

7.6—8.1 (2H, multiplet, $C_6$—H, $C_9$—H), 15.05 (1H, singlet, COOH).

b) Ten ml of trifluoroacetic acid was cooled with ice, stirred then 1.47 grams (3.55 mmol) of 8-chloro-7-fluoro-1-(2-tetrahydropyranyloxymethyl)-5-oxo-1,2-dihydro-5H-thiazolo(3,2-a)quinoline-4-carboxylic acid was gradually added thereto, and stirred with stirring and ice cooling for thirty minutes. Then trifluoroacetic acid was removed therefrom under reduced pressure on ice water bath, 30 ml of water was added thereto, and crystals separated therefrom were collected by filtration and dried to give 1.16 grams of 8-chloro-7-fluoro-1-hydroxymethyl-5-oxo-1,2-dihydro-5H-thiazolo(3,2-a)quinoline-4-carboxylic acid. The yield was 99.1 percent and melting point was 314 to 316°C (with decomposition). Elementary analysis calculated as $C_{13}H_9ClFCO_4S$: C 47.35, H 2.75, N 4.25; Found: C 47.58, H 2.83, N 4.31.

Infrared absorption spectra (KBr, $cm^{-1}$): 3600 to 3200, 1700, 1595.

Nuclear magnetic resonance spectra δ ($CF_2CO_2D$): 3.1—3.7 (4H multiplet, S—$\underline{CH}_2$—, O—$\underline{CH}_2$—), 5.8—6.3 (1H, multiplet,

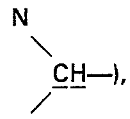

7.8—8.4 (2H, multiplet, $C_6$—H, $C_9$—H).

Example 28

7-Fluoro-1-hydroxymethyl-8-(4-methyl-1-piperazinyl)-5-oxo-1,2-dihydro-5H-thiazolo(3,2-a)quinoline-4-carboxylic acid

A mixture of 0.98 gram (2.97 mmol) 8-chloro-7-fluoro-1-hydroxymethyl-5-oxo-1,2-dihydro-5H-thiazolo(3,2-a)quinoline-4-carboxylic acid and 1.50 grams (14.97 mmol) of N-methylpiperazine was heated to reflux in 20 ml of pyridine for eighteen hours. Pyridine was removed therefrom under reduced pressure, then 5 ml of dimethyl formamide was added thereto, the mixture was heated so that the content was dissolved, then allowed to stand at room temperature, crystals separated therefrom were collected by filtration and were recrystallized from a mixture of trifluoroacetic acid and water to give 264 ml of the title compound. The yield was 22.6 percent and melting point was 264 to 266°C (with decomposition). Elementary analysis calculated as $C_{18}H_{20}FN_3O_4S$: C 54.95, H 5.12, N 10.68; Found: C 55.02, H 5.31, N 10.84.

Infrared absorption spectra (KBr, $cm^{-1}$): 3310, 1700, 1630, 805.

Nuclear magnetic resonance spectra δ ($CF_3CO_2D$): 3.15 (3H, singlet, N—$\underline{CH}_3$),

3.3—4.5 (12H, multiplet, 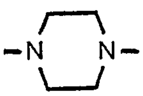, S—$\underline{CH}_2$, O$\underline{CH}_2$),

5.9—6.3 (1H, multiplet,

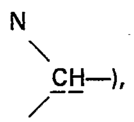

7.42 (1H, doublet, $C_9$—H), 7.12 (1H, doublet, $C_6$—H).

Example 29

Ethyl 8-chloro-1-(N,N-dimethylaminomethyl)-7-fluoro-5-oxo-1,2-dihydro-5H-thiazolo(3,2-a)quinoline-4-carboxylate

A mixture of 6.02 grams (20 mmol) of ethyl 7-chloro-6-fluoro-4-hydroxy-2-mercaptoquinoline-3-carboxylate and 7.20 gram (29.4 mmol) of 1,2-dibromo-3-dimethylaminopropane was stirred in 100 ml of dimethyl formamide for fifteen hours in the presence of 9.30 grams (67.3 mmol) of potassium carbonate. Dimethyl formamide was then evaporated therefrom under reduced pressure, water was added to the residue, and extracted with chloroform. The resulting chloroform solution was washed with water, dried, concentrated, and ether was added thereto. Insoluble crystals obtained thereby were collected by filtration to give 6.84 grams of ethyl 8-chloro-1-dimethylamino-methyl-7-fluoro-5-oxo-1,2-dihydro-5H-thiazolo(3,2-a)quinoline-4-carboxylate. The yield was 89.1 percent and the melting point was 165 to 168°C. Elementary analysis calculated as $C_{17}H_{18}ClFN_2O_3S$: C 53.05, H 4.71, N 7.28; Found: C 53.31, H 4.50, N 7.62.

Infrared absorption spectra (KBr, $cm^{-1}$): 1705, 1595, 805.

Nuclear magnetic resonance spectra δ ($CDCl_3$): 1.41 (3H, triplet, $OCH_2\underline{CH}_3$), 2.36 (6H, singlet,

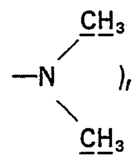 ),

3.45—3.65 (2H, multiplet, S$\underline{CH_2}$), 4.35 (2H, quartet, O$\underline{CH_2}CH_3$), 4.9—5.3 (1H, multiplet,

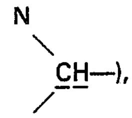 ),

7.29 (1H, doublet, $C_9$—H), 7.95 (1H, doublet, $C_6$—H).

Example 30

8-Chloro-1-(N,N-dimethylaminomethyl)-7-fluoro-5-oxo-1,2-dihydro-5H-thiazolo(3,2-a)-quinoline-4-carboxylic acid.

Ethyl 8-chloro-1-dimethylaminomethyl-7-fluoro-5-oxo-1,2-dihydro-5H-thiazolo(3,2-a)-quinoline-4-carboxylate (3.35 grams, 8.7 mmol) was dissolved in 40 ml of ethanol, 965 mg (24.1 mmol) of sodium hydroxide and 6 ml of water were added thereto, and the mixture was heated to reflux for forty minutes. Ethanol was removed therefrom under reduced pressure, the residue was neutralized with acetic acid, crystals separated therefrom were collected by filtration, dried, and recrystallized from dimethyl formamide to give 1.81 grams of the title product. The yield was 58.3 percent and the melting point was 214 to 216°C. Elementary analysis calculated as $C_{15}H_{14}ClFN_2O_3S$: C 50.49, H 3.95, N 7.85; Found: C 50.63, H 3.78, N 7.61.

Infrared absorption spectra (KBr, $cm^{-1}$): 1705, 1959, 1045, 810.

Nuclear magnetic resonance spectra δ ($CF_3CO_2D$): 3.29 (3H, singlet, =N—$\underline{CH_3}$), 3.40 (3H, singlet, =N—$\underline{CH_3}$), 3.3—4.6 (4H, multiplet, S—$\underline{CH_2}$, N—$\underline{CH_2}$), 6.1—6.7 (1H, multiplet,

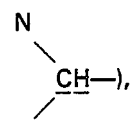 ),

8.07 (1H, doublet, $C_9$—H), 8.26 (1H, doublet, $C_6$—H).

Example 31

1-(N,N-Dimethylaminoethyl)-7-fluoro-8-(4-methyl-1-piperazinyl)-5-oxo-1,2-dihydro-5H-thiazolo(3,2-a)-quinoline-4-carboxylic acid

A mixture of 1.0 gram (2.8 mmol) of 8-chloro-1-(N,N-dimethylaminomethyl)-7-fluoro-5-oxo-1,2-dihydro-5H-thiazolo(3,2-a)-quinoline-4-carboxylic acid and 3.20 grams (31.9 mmol) of N-methylpiperazine was heated to reflux in 15 ml of pyridine for fifteen hours. Pyridine was removed therefrom under reduced pressure, then 5 ml of dimethyl formamide was added thereto, the content was dissolved by heating, allowed to cool to room temperature, and crystals separated therefrom were collected by filtration and recrystallized from dimethyl formamide to give 384 mg of the title compound in 32.6 percent yield. Melting point was 221 to 223°C. Elementary analysis calculated as $C_{20}H_{25}FN_4O_3S$: C 57.13, H 5.99, N 13.32; Found: C 57.02, H 6.13, N 13.63.

Infrared absorption spectra (KBr, $cm^{-1}$): 1700, 1625, 1580, 805.

Nuclear magnetic resonance spectra δ ($CF_3CO_2D$): 3.18 (3H, singlet, =N—$\underline{CH_3}$), 3.25 (3H, singlet,

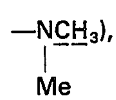 ),

3.36 (3H, singlet,

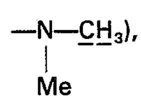 ),

3.2—4.5 (12H, multiplet, $S\underline{CH_2}$, 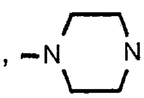, $N\underline{CH_2}$—),

6.3—6.7 (1H, multiplet,

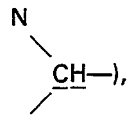),

7.20 (1H, doublet, $C_9$—H), 8.13 (1H, doublet, $C_6$—H).

Example 32

Ethyl 8-chloro-7-fluoro-5-oxo-1-phenyl-1,2-dihydro-5H-thiazolo(3,2-a)-quinoline-4-carboxylate

In dimethyl formamide was dissolved 2.0 grams (6.6 mmol) of ethyl 7-chloro-6-fluoro-4-hydroxy-2-mercaptoquinoline-3-carboxylate and the mixture was heated at 40°C for four hours with stirring after addition of 1.8 grams (13.2 mmol) of potassium carbonate and 2.1 geams (8.0 mmol) of (1,2-dibromoethyl) benzene. The content was concentrated under reduced pressure, water was added to the residue, extracted with chloroform, the chloroform extract was washed with water, dried, concentrated, and the resulting crystals were recrystallized from a mixture of chloroform and ether to give the title compound. The yield was 2.17 grams (81.3 percent) and melting point was 172°C. Elementary analysis calculated as $C_{20}H_{15}ClFNO_3S$: C 59.48, H 3.84, N 3.47; Found: C 59.54, H 3.53, N 3.27.

Infrared absorption spectra (KBr, $cm^{-1}$): 1715, 1675, 1636, 1608, 1480, 1180.

Nuclear magnetic resonance spectra δ ($CF_3CO_2D$): 1.38 (3H, triplet, —$OCH_2\underline{CH_3}$), 4.30 (2H, quartet, —$O\underline{CH_2}CH_3$), 4.40—5.30 (3H, multiplet, $C_1$—H, $C_2$—H), 7.20 (1H, doublet, $C_9$—H), 7.32 (5H, singlet, phenyl group-H), 7.90 (1H, doublet, $C_6$—H).

Example 33

8-Chloro-7-fluoro-5-oxo-1-phenyl-1,2-dihydro-5H-thiazolo(3,2-a)-quinoline-4-carboxylic acid

8-Chloro-7-fluoro-5-oxo-1-phenyl-1,2-dihydro-5H-thiazolo(3,2-a)-quinoline-4-carboxylic acid ethyl ester (2.17 grams, 5.4 mmol) was suspended in a mixture of 0.43 gram (10.7 mmol) of sodium hydroxide, 30 ml of ethanol and 60 ml of water and the whole was heated to reflux for twelve hours. After cooled, the mixture was acidified with acetic acid, crystals separated out therefrom were collected by filtration, washed with water, dried with air, and recrystallized from Methyl Cellosolve to give the title compound in 1.18 grams yield (58.1 percent). Melting point was 237 to 40°C (with decomposition). Elementary analysis calculated as $C_{18}H_{11}ClFNO_3S$: C 57.53, H 2.95, N 3.72; Found: C 57.72, H 2.64, N 3.56.

Infrared absorption spectra (KBr, $cm^{-1}$): 1700, 1592, 1470.

Nuclear magnetic resonance spectra δ ($CF_3CO_2D$): 5.00—5.70 (3H, multiplet, $C_1$—H, $C_2$—H), 7.37(5H, singlet, phenyl group-H), 7.95 (1H, doublet, $C_9$—H), 8.20 (1H, doublet, $C_6$—H).

Example 34

8-Chloro-1-ethoxy-7-fluoro-5-oxo-1,2-dihydro-5H-thiazolo(3,2-a)-quinoline-4-carboxylic acid

Ethyl 7-chloro-6-fluoro-4-hydroxy-2-mercaptoquinoline-3-carboxylate (4.0 grams, 13.3 mmol) was dissolved in 30 ml of dimethyl formamide, the mixture was stirred for a while after addition of 3.66 grams (27 mmol) of potassium carbonate), and heated at 80°C with stirring for eighteen hours with 3.94 grams (20 mmol) of 2-bromo-1,1-diethoxyethane. When the reaction was completed, the solvent was removed therefrom under reduced pressure, water was added to the residue, and crystals separated therefrom were extracted with ethyl acetate. The extract was washed with water, dried with magnesium sulfate, and the solvent was removed therefrom under reduced pressure to give 3.2 grams (57.5 percent) of ethyl 7-chloro-2-(2,2-diethoxyethylthio)-6-fluoro-4-hydroxyquinoline-3-carboxylate.

Ethyl 7-chloro-2-(2,2-diethoxyethylthio)-6-fluoro-4-hydroxyquinoline-3-carboxylate (3.2 grams, 7.6 mmol) was added to 15 ml of concentrated hydrochloric acid and the mixture was stirred with heating at 90°C. When the ester was completely dissolved therein, ice water was poured thereinto, and crystals separated out therefrom were collected by filtration whereupon 2.68 grams (94.3 percent) of ethyl 8-chloro-1-ethoxy-7-fluoro-5-oxo-1,2-dihydro-5H-thiazolo(3,2-a)-quinoline-4-carboxylate was obtained.

Ethyl 8-chloro-1-ethoxy-7-fluoro-5-oxo-1,2-dihydro-5H-thiazolo(3,2-a)-quinoline-4-carboxylate (2.68 grams, 7.2 mmol) was dissolved in 80 ml of hot ethanol and 30 ml of 2N sodium hydroxide solution was added thereto. The mixture was heated to reflux for ten minutes, then cooled to a room temperature, and insoluble matters were filtered off. The mother liquor was adjusted to pH 7.0 to 6.5 and separated crystals therefrom were collected by filtration. The crystals were then washed with water, ethanol and ether, air-dried, and the resulting crude crystals were recrystallized from dimethyl formamide to give 1.56 grams (62.4 percent) of the title compound. Melting point was 308.5°C (with decomposition). Elementary analysis calculated as $V_{14}H_{11}ClFNO_4S$: C 48.92, H 3.23, N 4.05; Found: C 48.89, H 3.20, N 4.00.

Infrared absorption spectra (KBr, $cm^{-1}$): 1695, 1590, 1470, 810.

Nuclear magnetic resonance spectra δ ($CF_3CO_2D$): 1.31 (3H, multiplet, $—OCH_2CH_3$), 3.50—4.30 (4H, multiplet, $—O—CH_2CH_3$, $C_2$—H), 6.98 (1H, double doublet, $C_1$—H), 8.00—8.30 (2H, multiplet, phenyl group-H).

Example 35

1-Ethoxy-7-fluoro-8-(4-methyl-1-piperazinyl)-5-oxo-1,2-dihydro-5H-thiazolo(3,2-a)-quinoline-4-carboxylic acid

8-Chloro-1-ethoxy-7-fluoro-5-oxo-1,2-dihydro-5H-thiazolo(3,2-a)quinoline-4-carboxylic acid (800 mg, 2.32 mmol) was suspended in 20 ml of pyridine and the mixture was heated to reflux after addition of N-methylpiperazine. After thirteen hours, 4 ml more of N-methylpiperazine was added thereto, and the mixture was heated to reflux for four hours more. When the reaction was completed, pyridine was removed therefrom under reduced pressure, water was added to the residue, and crystalline substance was collected by filtration. This was recrystallized from acetonitrile to give 340 mg of the title product. Yield was 36 percent. Melting point was 266.5°C (with decomposition). Elementary analysis calculated as $C_{19}H_{22}FN_3O_4S$: C 56.00, H 5.44, N 10.31; Found: C 56.13, H 5.58, N 10.40.

Infrared absorption spectra (KBr, $cm^{-1}$): 1700, 1630, 1490.

Nuclear magnetic resonance spectra δ ($CF_3CO_2D$): 1.29 (3H, triplet, $—OCH_2CH_3$), 3.11 (3H, singlet, $NCH_3$),

3.35—4.50 (12H, multiplet, 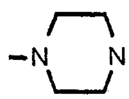, $—OCH_2CH_3$, $C_2$—H),

6.90—7.25 (1H, multiplet, $C_1$—H) 7.38 (1H, wide doublet, $C_9$—H), 8.14 (1H, doublet, $C_6$—H).

Example 36

Ethyl 8-chloro-7-fluoro-5-oxo-5H-thiazolo(3,2-a)quinoline-4-carboxylate

Ethyl 7-chloro-6-fluoro-4-hydroxy-2-mercaptoquinoline-carboxylate (3.0 grams, 10 mmol) was dissolved in 20 ml of dimethyl formamide and the solution was heated to reflux at 80°C for twelve hours after addition of 2.7 grams of potassium carbonate and 2.9 grams (15 mmol) of bromoacetaldehyde diethyl acetal. The content was concentrated under reduced pressure, water was added to the residue, then extracted with ethyl acetate, and the extract was washed with water and concentrated to give 4.3 grams of crystalline residue. To this was added 22.0 grams of concentrated sulfuric acid, the mixture was heated with stirring at 90°C for twenty minutes, poured over into ice water, crystals separated out therefrom were collected by filtration, washed with water, air dried and recrystallized from dimethyl formamide to give 2.6 grams (yield 80.0 percent) of the title compound. Melting point was 257 to 8°C (with decomposition). Elementary analysis calculated as $C_{14}H_9ClFNO_3S$: C 51.46, H 2.78, N 4.29; Found: C 51.55, H 2.57, N 3.95.

Infrared absorption spectra (KBr, $cm^{-1}$): 1600, 1610, 1478, 1245, 1205.

Nuclear magnetic resonance spectra δ ($CF_3CO_2D$): 1.66 (3H, triplet, $—CH_2CH_3$), 4.82 (2H, quartet, $—CH_2CH_3$), 8.04 (1H, doublet, $C_2$—H), 8.38 (1H, doublet, $C_6$—H), 8.62 (1H, doublet, $C_9$—H), 8.92 (1H, doublet, $C_1$—H).

Example 37

8-Chloro-7-fluoro-5-oxo-5H-thiazolo(3,2-a)-quinoline-4-carboxylic acid

Ethyl 8-chloro-7-fluoro-5-oxo-5H-thiazolo(3,2-a)-quinoline-4-carboxylate (1.94 gram, 6 mmol) was suspended in a mixture of 6 ml of ethanol, 80 ml of water and 0.5 gram (12 mmol) of sodium hydroxide and the whole was heated to reflux for two hours. After cooled, the mixture was acidified with acetic acid, crystals separated therefrom were collected by filtration, washed with water, air dried, and recrystallized from dimethyl formamide to give the title compound. The yield was 1.3 grams (72.6 percent). Melting point was 310 to 15°C (with decomposition). Elementary analysis calculated as $C_{12}H_5ClFNO_3S$: C 48.42, H 1.69, N 4.71; Found: C 48.44, H 1.48, N 4.40.

Infrared absorption spectra (KBr, $cm^{-1}$): 3125, 1690, 1595, 1490.

Nuclear magnetic resonance spectra δ ($CF_3CO_2D$): 8.05 (1H, doublet, $C_2$—H), 8.37 (1H, doublet, $C_8$—H), 8.63 (1H, doublet, $C_9$—H), 8.92 (1H, doublet, $C_1$—H).

Example 38

7-Fluoro-5-oxo-8-(1-piperazinyl)-5H-thiazolo(3,2-a)-quinoline-4-carboxylic acid

A mixture of 10.0 grams (0.034 mol of 8-chloro-7-fluoro-5-oxo-5H-thiazolo(3,2-a)-quinoline-4-carboxylic acid, 29.0 grams (0.34 mol) of anhydrous piperazine and 250 ml of pyridine was heated to reflux for fifty five hours. After cooled, the crystals separated out therefrom were collected by filtration, washed with pyridine and then with ether, the resulting crude crystals were suspended in 100 ml of water, then they were made dissolved therein by acidifying with acetic acid, treated with activated charcoal, adjusted to pH 7 with 2N sodium hydroxide solution, crystals separated out were collected by filtration, washed with water, dried, and recrystallized from dimethyl foramide to give the title compound. The yield was 4.78 grams (40.5

percent). Colorless crystals melting at 283°C (with decomposition). Elementary analysis calculated as $C_{16}H_{14}FN_3O_3S$: C 55.32, H 4.06, N 12.10; Found: 55.52, H 4.14, N 12.37.

Infrared absorption analysis (KBr, $cm^{-1}$): 1670, 1630, 1600, 1490, 1380, 1260.

Nuclear magnetic resonance spectra δ ($CF_3CO_2D$):

3.50—4.30 (8H, multiplet, 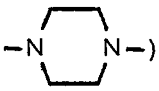),

7.85 (1H, doublet, $C_9$—H), 7.96 (1H, doublet, $C_2$—H), 8.25 (1H, doublet, $C_6$—H), 8.96 (1H, doublet, $C_1$—H).

Example 39

7-Fluoro-8-(4-methyl-1-piperazinyl)-5-oxo-5H-thiazolo(3,2-a)-quinoline-4-carboxylic acid

A mixture of 700 mg (2.35 mmol) of 8-chloro-7-fluoro-5-oxo-5H-thiazolo(3,2-a)-quinoline-4-carboxylic acid, 2.4 grams (23.5 mmol) of N-methylpiperazine and 30 ml of pyridine was heated to reflux for twelve hours on an oil bath. The content was concentrated under reduced pressure, water was added to the residue, insoluble matters therein were collected by filtration, washed with water, air dried and recrystallized from dimethyl formamide twice to give the title compound. The yield was 360 mg (42.4 percent). Melting point was 305 to 8°C (with decomposition). Elementary analysis calculated as $C_{17}H_{16}FN_3O_3S$: C 56.50, H 4.46, N 11.63; Found: C 56.28, H 4.45, N 11.68.

Infrared absorption spectra (KBr, $cm^{-1}$): 1679, 1627, 1488, 1263.

Nuclear magnetic resonance spectra δ ($CF_3CO_2D$): 3.18 (3H, singlet, $=N\underline{CH_3}$),

3.40—4.60 (8H, multiplet, 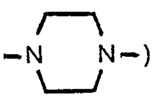),

7.80 (1H, doublet, $C_9$—H), 7.96 (1H, doublet, $C_2$—H), 8.24 (1H, doublet, $C_6$—H), 8.91 (1H, doublet, $C_1$—H).

Example 40

8-(4-Allyl-1-piperazinyl)-7-fluoro-5-oxo-5H-thiazolo(3,2-a)-quinoline-4-carboxylic acid

7-Fluoro-5-oxo-8-(1-piperazinyl)-5H-thiazolo-(3,2-a)-quinoline-4-carboxylic acid (400 mg, 1.2 mol) was dissolved in 5 ml of dimethyl formamide and heated at 70°C for four hours after addition of 190 mg (1.4 mmol) of carbonic acid and 170 mg (1.4 mmol) of allyl bromide. The content was concentrated under reduced pressure and purified by column chromatography (using silica gel and chloroform-methanol (10:1)). Colorless crystals. The yield was 220 mg (68.8 percent). Melting point was 298°C (with decomposition). Elementary analysis calculated as $C_{19}H_{18}FN_3O_3S$: C 58.23, H 4.76, N 10.72; Found: C 58—20— H 4.53, N 10.55.

Infrared absorption spectra (KBr, $cm^{-1}$): 1685, 1627, 1490, 1262, 1135, 1010.

Nuclear magnetic resonance spectra δ ($CF_3CO_2D$):

3.00—4.60 (10H, multiplet, 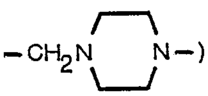),

5.6—6.1 (3H, multiplet, —CH=$CH_2$), 7.85 (1H, doublet, $C_9$—H), 7.98 (1H, doublet, $C_2$—H), 8.30 (1H, doublet, $C_6$—H), 8.97 (1H, doublet, $C_1$—H).

Example 41

8-(4-Ethyl-1-piperazinyl)-7-fluoro-5-oxo-5H-thiazolo(3,2-a)-quinoline-4-carboxylic acid

7-Fluoro-5-oxo-8-(1-piperazinyl)-5H-thiazolo(3,2-a)-quinoline-4-carboxylic acid (300 mg, 0.86 mmol) was dissolved in 5 ml of dimethyl formamide and heated with stirring at 70°C for twelve hours after addition of 142 mg (1.03 mmol) of potassium carbonate and 161 mg (1.03 mmol) of ethyl iodide. The content was concentrated under reduced pressure, water was added to the residue, crystals separated out therefrom were collected by filtration, washed with water, air dried, and recrystallized from dimethyl formamide to give the title compound. Colorless prisms. The yield was 130 mg (40.2 percent) and melting point was 307 to 10°C (with decomposition). Elementary analysis calculated as $C_{18}H_{18}FN_3O_3S$: C 57.59, H 4.83, N 11.19; Found: C 57.59, H 4.83, N 10.92.

Infrared absorption spectra (KBr,$cm^{-1}$): 1680, 1630, 1490, 1262, 1030.

Nuclear magnetic resonance spectra δ ($CF_3CO_2D$): 1.55 (3H, triplet, —$CH_2\underline{CH_3}$),

3.10—4.60 (10H, multiplet, 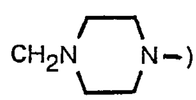),

7.82 (1H, doublet, $C_9$—H), 8.29 (1H, doublet, $C_2$—H), 8.12 (1H, doublet, $C_6$—H), 8.92 (1H, doublet, $C_1$—H).

## Example 42

7-Fluoro-8-{4-(2-hydroxyethyl)-1-piperazinyl}-5-oxo-5H-thiazolo(3,2-a)-quinoline-4-carboxylic acid hydrobromide

7-Fluoro-5-oxo-8-(1-piperazinyl)-5H-thiazolo(3,2-a)-quinoline-4-carboxylic acid (350 mg) was suspended in 50 ml of dimethyl formamide, 150 mg of triethyl amine was added thereto, the mixture was stirred for thirty minutes at room temperature, and heated with stirring at twenty two hours at 80°C with 175 mg of ethylene bromohydrin. During the above reaction, each 120 mg of ethylene bromohydrin was added twice to the reaction solution. Then the reaction mixture was concentrated to about 20 ml under reduced pressure, crystals separated out therefrom were collected by filtration, washed with ethanol and dried to give 250 mg of the title compound as colorless powder. Melting point was 298 to 300°C (with decomposition). Elementary analysis calculated as $C_{18}H_{18}FN_3O_4S.HBr$: C 45.77, H 4.05, N 8.90; Found: C 46.11, H. 4.14, N 8.99.

Infrared absorption spectra (KBr,cm$^{-1}$): 3400, 2700, 2600, 2470, 1690, 1632, 1495, 1273, 1035, 1000, 940, 805, 795.

Nuclear magnetic resonance spectra δ ($CF_3CO_2D$):

3.20–4.65 (12H, multiplet, 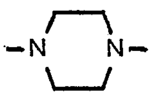 and methylene proton of hydroxyethyl group).

7.90 (1H, doublet, $C_9$—H), 7.97 (1H, doublet, $C_2$—H), 8.28 (1H, doublet, $C_6$—H), 9.00 (1H, doublet, $C_1$—H).

## Example 43

Ethyl 8-chloro-7-fluoro-1-methyl-5-oxo-5H-thiazolo(3,2-a)-quinoline-4-carboxylate

A mixture of 6.02 grams (20 mmol) of ethyl 7-chloro-6-fluoro-4-hydroxy-2-mercaptoquinoline-3-carboxylate and 2.04 grams (22.0 mmol) of 1-chloro-2-oxopropane in 300 ml of ethanol was heated to reflux for twenty four hours. This was concentrated to 20 ml under reduced pressure, allowed to stand at room temperature, crystals* separated out therefrom were collected by filtration, and washed with n-hexane to give 5.74 grams of crystalline substance. This was stirred for one hour at room temperature with 15 ml of concentrated sulfuric acid. The reaction solution was poured over into ice flakes, crystals separated out were collected by filtration, washed with water, dried and recrystallized from dimethyl formamide to give 4.86 grams (yield 71.7 percent) of ethyl 8-chloro-7-fluoro-1-methyl-5-oxo-5H-thiazolo(3,2-a)-quinoline-4-carboxylate. (*The crystals were a mixture of ring closed compound and ring unclosed one and the latter was not isolated at this time). Melting point was 291 to 293°C (with decomposition). Elementary analysis calculated as $C_{15}H_{11}ClFNO_3S$: C 53.03, H 3.26, N 4.12; Found: C 52.47, H 3.47, N 4.57.

Infrared absorption spectra (KBr, cm$^{-1}$): 1695, 1670, 1605, 1310, 800.

Nuclear magnetic resonance spectra δ ($CF_3CO_2D$): 1.66 (3H, triplet, $OCH_2\underline{CH_3}$), 3.23 (3H, singlet, $C_1$—$\underline{CH_3}$), 4.80 (2H, quartet, $O\underline{CH_2}CH_3$), 7.66 (1H, singlet, $C_2$—H), 8.39 (1H, doublet, $C_6$—H), 8.93 (1H, doublet, $C_9$—H).

## Example 44

8-Chloro-7-fluoro-1-methyl-5-oxo-5H-thiazolo(3,2-a)-quinoline-4-carboxylic acid

Ethyl 8-chloro-7-fluoro-1-methyl-5-oxo-5H-thiazolo(3,2-a)-quinoline-4-carboxylate (10.0 grams, 29.4 mmol) was suspended in 40 ml of ethanol, then 3.88 grams of potassium hydroxide and 380 ml of water were added thereto, and the mixture was heated to reflux for six hours with stirring. Then it was concentrated to 300 ml under reduced pressure, neutralized with acetic acid, vigorously stirred overnight, crystals separated out therefrom were collected by filtration, washed with water, dried and recrystallized from dimethyl formamide to give 8.08 grams of the title compound. The yield was 88.1 percent. Melting point was 317 to 319°C (with decomposition). Elementary analysis calculated as $C_{13}H_7ClFNO_3S$: C 50.09, H 2.26, N 4.49; Found: C 50.32, H 2.37, N 4.37.

Infrared absorption spectra (KBr,cm$^{-1}$): 1695, 1590, 1050, 805, 800.

Nuclear magnetic resonance spectra δ ($CF_3CO_2D$): 3.26 (3H, singlet, $C_1$—$\underline{CH_3}$), 7.72 (1H, singlet, $C_2$—H), 8.46 (1H, doublet, $C_6$—H), 8.98 (1H, doublet, $C_9$—H).

## Example 45

7,8-Difluoro-1-methyl-5-oxo-5H-thiazolo(3,2-a)-quinoline-4-carboxylic acid

Ethyl 7,6-difluoro-4-hydroxy-2-mercaptoquinoline-3-carboxylate (2.85 grams, 0.01 mol) and 1.02 grams (0.011 mol) of chloroacetone were dissolved in 30 ml of ethanol and the mixture was heated to reflux for sixteen hours. After cooled, the crystals separated out therefrom were collected by filtration and washed with ether to give 2.50 grams of crude crystals. The crystals were added to 15 grams of concentrated sulfuric acid with ice cooling, the mixture was stirred for one hour, and stirred for another thirty minutes at room temperature. The reaction solution was poured over into ice water, crystals separated out were collected by filtration, washed with water, then with ether, and dried with air to give 2.11 grams of 7,8-difluoro-1-methyl-5-oxo-5H-thiazolo(3,2-a)-quinoline-4-carboxylic acid. This was

recrystallized from a mixture of dimethyl formamide and ethanol to give the product which melted at 270 to 80°C (with decomposition). Elementary analysis calculated as $C_{13}H_7FNO_3S$: C 52.88, H 2.39, N 4.74; Found: C 53.05, H 2.40, N 4.61.

Infrared absorption spectra (KBr,$cm^{-1}$): 1690, 1610, 1595, 1550.

Nuclear magnetic resonance δ ($CF_3CO_2D$): 3.25 (3H, singlet, $C_1$—H), 7.68 (1H, singlet, $C_2$—H), 8.52 (1H, triplet, $C_9$—H), 8.71 (1H, double doublet, $C_6$—H).

Example 46

7-Fluoro-1-methyl-5-oxo-8-(1-piperazinyl)-5H-thiazolo(3,2-a)-quinoline-4-carboxylic acid

a) 8-Chloro-7-fluoro-1-methyl-5-oxo-5H-thiazolo(3,2-a)-quinoline-4-carboxylic acid (15.0 grams, 4.81 mmol) was heated to reflux in a mixture of 4.14 grams (48.1 mmol) of anhydrous piperazine and 28 ml of pyridine for twenty hours. After the reaction was completed, the mixture was allowed to stand at room temperature, crystals separated out therefrom were collected by filtration, washed with an aqueous acetic acid, then dissolved in aqueous 10 percent solution of sodium hydroxide, adjusted to pH 7.2 with aqueous solution of acetic acid, crystals separated out were collected by filtration, washed with water, dried, and recrystallized from dimethyl formamide to give 220 mg of the title compound. The yield was 12.7 percent and melting point was 284 to 6°C (decomposition). Elementary analysis calculated as $C_{17}H_{16}FN_3O_3.{}^1/_2H_2O$: C 55.13, H 4.63, N 11.34; Found: C 55.63, H 4.61, N 11.34.

Infrared absorption spectra (KBr, $cm^{-1}$): 3200 to 3600, 1700, 1635, 805.

Nuclear magnetic resonance spectra δ ($CF_3CO_2D$): 3.25 (3H, singlet, $C_1$—$CH_3$),

3.2—4.4 (8H, multiplet, 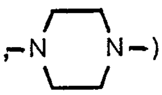),

7.71 (1H, singlet, $C_2$—H), 8.1 to 8.6 (2H, multiplet, $C_6$—H, $C_9$—H).

b) To a mixture of 295 mg (1 mmol) of 7,8-difluoro-1-methyl-5-oxo-5H-thiazolo(3,2-a)-quinoline-4-carboxylic acid and 344 mg (4 mmol) of anhydrous piperazine was added 2 ml of pyridine and the whole was heated with stirring for 2 hours on an oil bath kept at 100°C. After cooled, crystals separated out therefrom were collected by filtration, and washed with ethanol to give 322 mg of crude crystals. Those were recrystallized from a mixture of dimethyl formamide and ethanol to give 264 mg of the title compound. The yield was 73 percent. Melting point was 284 to 6°C (with decomposition). The resulting compound was identified with that obtained in the method a) by infrared absorption spectra and nuclear magnetic resonance spectra.

Example 47

7-Fluoro-1-methyl-8-(4-methyl-1-piperazinyl)-5-oxo-5H-thiazolo(3,2-a)-quinoline-4-carboxylic acid and 8-chloro-1-methyl-7-(4-methyl-1-piperazinyl)-5-oxo-5H-thiazolo(3,2-a)-quinoline-4-carboxylic acid

a) Five grams (16.0 mmol) of 8-chloro-7-fluoro-1-methyl-5-oxo-5H-thiazolo(3,2-a)-quinoline-4-carboxylic acid was heated to reflux for twenty four hours in a mixture of 8.0 grams (80 mmol) of N-methyl piperazine and 150 ml of pyridine and the refluxing was continued for more thirty hours after addition of 8.0 grams (80 mmol) more of N-methylpiperazine. Pyridine was removed therefrom under reduced pressure, ethyl acetate was added thereto, crystalline substance appeared was collected by filtration, and subjected to a silica gel column chromatography. The column was eluted with a mixture (10:1) of chloroform and methanol and from the first eluate was obtained 1.83 grams of title 8-chloro-1-methyl-7-(4-methyl-1-piperazinyl)-5-oxo-5H-thiazolo(3,2-a)-quinoline-4-carboxylic acid. The yield was 26.1 percent. Melting point was 291 to 293°C (with decomposition). Elementary analysis calculated as $C_{18}H_{18}ClN_3O_3S$: C 55.17, H 4.63, N 10.72; Found: C 55.11, H 4.70, N 10.63.

Infrared absorption spectra (KBr, $cm^{-1}$): 1690, 1590, 805.

Nuclear magnetic resonance spectra δ ($CF_3CO_2D$): 3.22 (6H, wide singlet, $C_1$—$CH_3$, =N—$CH_3$),

3.3 to 4.2 (8H, multiplet, 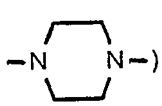),

7.68 1H, singlet, $C_2$—H), 8.29 (1H, singlet, $C_6$—H), 8.96 (1H, singlet, $C_9$—H).

From the succeeding eluate was obtained 2.06 grams (yield 34.2 percent) of 7-fluoro-1-methyl-8-(4-methyl-1-piperazinyl)-5-oxo-5H-thiazolo(3,2-a)-quinoline-4-carboxylic acid. Melting point 288 to 290°C (with decomposition). Elementary analysis calculated as $C_{18}H_{18}FN_3O_3S$: C 57.59, H 4.83, N 11.19; Found: C 57.42, H 4.98, N 11.03.

Infrared absorption spectra (KBr, $cm^{-1}$): 1695, 1630, 800.

Nuclear magnetic resonance spectra δ ($CF_3CO_2D$): 3.22 (3H, singlet, —$NCH_3$), 3.27 (3H, singlet, $C_1$—$CH_3$),

3.3 to 4.4 (8H, multiplet, 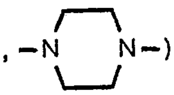),

7.70 (1H, singlet, $C_2$—H), 8.33 (1H, doublet, $C_9$—H), 8.45 (1H, doublet, $C_6$—H).

b) To a mixture of 295 mg (1 mmol) of 7,6-difluoro-1-methyl-5-oxo-5H-thiazolo(3,2-a)-quinoline-4-carboxylic acid and 400 mg (4 mmol) of N-methylpiperazine was added 2 ml of pyridine and the whole mixture was heated with stirring for two hours on an oil bath of 100°C. After cooled, crystals separated out therefrom were collected by filtration and washed with ethanol to give 364 mg of crude crystals. Those were recrystallized from a mixture of dimethylformamide and ethanol to give 284 mg (yield 76 percent) of the title 7-fluoro-1-methyl-8-(4-methyl-1-piperazinyl)-5-oxo-5H-thiazolo(3,2-a)-quinoline-4-carboxylic acid. Melting point was 285 to 7°C (with decomposition). This was identified with the compound obtained in the previous method a) by measurement of infrared absorption spectra and nuclear magnetic resonance spectra.

Example 48

8-(4-Allyl-1-piperazinyl)-7-fluoro-1-methyl-5-oxo-5H-thiazolo(3,2-a)-quinoline-4-carboxylic acid and 7-(4-allyl-1-piperazinyl)-8-chloro-1-methyl-5-oxo-5H-thiazolo(3,2-a)-quinoline-4-carboxylic acid

A mixture of 1.0 gram (3.2 mmol) of 8-chloro-7-fluoro-1-methyl-5-oxo-5H-thiazolo(3,2-a)-quinoline-4-carboxylic acid, 2.0 grams (16 mmol) of N-allyl piperazine, and 50 ml of pyridine was heated to reflux for seventy two hours. The content was concentrated, water was added to the residue, insoluble matter was collected by filtration, washed with water, dried with air, and the resulting crystals obtained by further filtration was purified by a column chromatography using silica gel and a 1:20 mixture of methanol and chloroform. From the firstly eluted fraction was obtained 7-(4-allyl-1-piperazinyl)-8-chloro-1-methyl-5-oxo-5H-thiazolo(3,2-a)-quinoline-4-carboxylic acid. Colorless crystals. The yield was 480 mg (35.8 percent). Melting point was 267 to 268°C (with decomposition). Elementary analysis calculated: C 57.48, H 4.82, N 10.05; Found: 57.02, H 4.68, N 9.79.

Infrared absorption spectra (KBr,$cm^{-1}$): 1690, 1575, 1470, 1010, 810.

Nuclear magnetic resonance spectra δ ($CF_3CO_2D$):

3.00 to 4.30 (13H, multiplet, 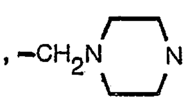, $-CH_3$),

5.6 to 6.1 (3H, multiplet, —CH=$CH_2$), 7.70 (1H, singlet, $C_2$—H), 8.30 (1H, singlet, $C_6$—H), 8.95 (1H, singlet, $C_9$—H).

From the next eluted fraction was obtained 8-(4-allyl-1-piperazinyl)-7-fluoro-1-methyl-5-oxo-5H-thiazolo(3,2-a)-quinoline-4-carboxylic acid. Colorless crystals. The yield was 510 mg (39.8 percent). Melting point was 225 to 6°C (with decomposition). Elementary analysis calculated: C 57.26, H 5.29, N 10.02; Found: C 57.17, H 5.43, N 9.74.

Infrared absorption spectra (KBr, $cm^{-1}$): 1680, 1630, 1490, 1270.

Nuclear magnetic resonance spectra δ ($CF_3CO_2D$): 3.23 (3H, singlet, —$CH_3$),

3.30 to 4.50 (10H, multiplet, 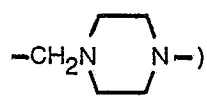),

5.6 to 6.0 (3H, multiplet, —CH=$CH_2$), 7.61 (1H, singlet, $C_2$—H), 8.21 (1H, doublet, $C_9$—H), 8.31 (1H, doublet, $C_6$—H).

Example 49

7-Fluoro-1-methyl-5-oxo-8-4-(thiomorpholino)-5H-thiazolo(3,2-a)-quinoline-4-carboxylic acid

A mixture of 295 mg (1 mmol) of 7,8-difluoro-1-methyl-5-oxo-5H-thiazolo(3,2-a)-quinoline-4-carboxylic acid and 412 mg (4 mmol) of thiomorpholine was added to 2 ml of pyridine and the whole mixture was heated with stirring for five hours on a oil bath kept at 80°C. After cooled, crystals separated out therefrom were collected by filtration, washed with ethanol, and the resulting crude crystals were recrystallized from dimethylformamide to give 272 mg of 7-fluoro-1-methyl-5-oxo-8-(4-thiomorpholino)-5H-thiazolo(3,2-a)-quinoline-4-carboxylic acid. Melting point was 318 to 320°C (with decomposition). Elementary analysis calculated as $C_{17}H_{15}FN_2O_3S$: C 53.95, H 4.00, N 7.40; Found: C 53.63, H 4.04, N 7.19.

Infrared absorption spectra (KBr, $cm^{-1}$): 1695, 1630, 1575, 1490, 1450, 1260, 965, 805, 795.

Nuclear magnetic resonance spectra δ ($CF_3CO_2D$): 3.25 (3H, singlet, $C_1$—$CH_3$), 3.00 to 3.50 (4H, multiplet, $C_6'$—$H_2$ and $C_2'$—$H_2$ of thiomorpholino group), 4.00 to 4.50 (4H, multiplet, $C_5'$—$H_2$ and $C_3'$—$H_2$ of thiomorpholino group), 7.70 (1H, singlet, $C_2$—H), 8.57 (1H, doublet, $C_6$—H), 8.00 (1H, doublet, $C_9$—H).

Example 50

8-Ethoxy-7-fluoro-1-methyl-5-oxo-5H-thiazolo(3,2-a)-quinoline-4-carboxylic acid

7,8-Difluoro-1-methyl-5-oxo-5H-thiazolo(3,2-a)-quinoline-4-carboxylic acid (295 mg, 1 mmol) was suspended in ethanol and sodium ethoxide solution (prepared from 5 ml of anhydrous ethanol and 57 mg of metal sodium) was dropped therein with stirring at room temperature. The mixture was stirred for one hour at room temperature and heated to reflux for 2 hours more. Ethanol was evaporated therefrom, the residue was dissolved in water, acidified with acetic acid, crystals separated therefrom were collected by filtration, and washed with water to give 290 mg of crude crystals. Those were recrystallized from dimethyl formamide to give 230 mg of 8-ethoxy-7-fluoro-1-methyl-5-oxo-5H-thiazolo(3,2-a)-quinoline-4-carboxylic acid. Melting point was 273 to 5°C (with decomposition). Elementary analysis calculated as $C_{15}H_{12}FNO_4S$: C 56.07, H 3.76, N 4.36; Found: C 56.51, H 3.70, N 4.44.

Infrared absorption spectra (KBr,$cm^{-1}$): 3400 to 2400, 1690, 1630, 1590, 1480, 1365, 1275, 1250, 1050, 1030, 935, 810.

Nuclear magnetic resonance spectra δ ($CF_3CO_2D$): 1.68 (3H, triplet, $—OCH_2\underline{CH_3}$), 3.29 (3H, singlet, $C_1—CH_3$), 4.47 (2H, quartet $—OCH_2CH_3$), 7.67 (1H, singlet, $C_2—H$), 8.35 (1H, doublet, $C_9—H$), 8.37 (1H, doublet, $C_6—H$).

Example 51

Ethyl 8-chloro-1-ethyl-7-fluoro-5-oxo-5H-thiazolo(3,2-a)-quinoline-4-carboxylate

a) To 3.0 grams (10 mmol) of ethyl 7-chloro-6-fluoro-4-hydroxy-2-mercaptoquinoline-3-carboxylate were added 1.7 grams (11.5 mmol) of bromoethyl ethyl ketone and 100 ml of ethanol and the mixture was heated to reflux for one hour. Ethanol was removed therefrom under reduced pressure and the residue was recrystallized from ethanol to give 3.2 grams of ethyl 7-chloro-6-fluoro-4-hydroxy-2-(2-oxo-1-butane-thio)-quinoline-3-carboxylate. The yield was 86 percent. Melting point was 239 to 242°C (with decomposition). Elementary analysis calculated as $C_{16}H_{15}ClFNO_4S$: C 51.69, H 4.07, N 3.77; Found: C 51.52, H 4.41, N 3.56.

Infrared absorption spectra (KBr, $cm^{-1}$): 1700, 1595, 1480, 1045.

Nuclear magnetic resonance spectra δ ($CF_3CO_2D$): 1.15 (triplet, $CH_2\underline{CH_3}$), 1.40 (3H, triplet, $CH_2\underline{CH_3}$), 2.20 to 2.80 (2H, multiplet, $—S\underline{CH_2}$), 3.15 to 4.36 (4H, multiplet, $—\underline{CH_2}CH_3 \times 2$), 8.23 (1H, doublet, $C_5—H$), 9.05 (1H, doublet, $C_8—H$).

b) to 1.84 grams (4.95 mmol) of ethyl 7-chloro-6-fluoro-4-hydroxy-2-(2-oxo-1-butylthio)-quinoline-3-carboxylate was added 7.0 grams of sulfuric acid and the mixture was heated with stirring at 70°C for fifteen minutes. After cooled with ice, 20 grams of ice was added thereto, the mixture was stirred for ten minutes, crystals separated out therefrom were collected by filtration, washed with water, dried and recrystallized from ethanol to give 1.5 grams of ethyl 8-chloro-1-ethyl-7-fluoro-5-oxo-5H-thiazolo(3,2-a)-quinoline-4-carboxylate. The yield was 86 percent. Melting point was 260 to 262°C (with decomposition). Elementary analysis calculated as $C_{16}H_{13}ClFNO_3S$: C 54.32, H 3.70, N 3.96; Found: C 54.52, H 3.95, N 3.65.

Infrared absorption spectra (KBr, $cm^{-1}$): 1690, 1585, 1503, 1470.

Nuclear magnetic resonance spectra δ ($CF_3CO_2D$): 1.70 (3H, triplet, $CH_2\underline{CH_3}$), 1.72 (3H, triplet, $—CH_2CH_3$), 3.64 (2H, quartet, $\underline{CH_2}CH_3$), 4.82 (2H, quartet, $—\underline{CH_2}CH_3$), 7.75 (1H, singlet, $C_2—H$), 8.42 (1H, doublet, $C_6—H$), 8.94 (1H, doublet, $C_9—H$).

Example 52

8-Chloro-1-ethyl-7-fluoro-5-oxo-5H-thiazolo(3,2-a)-quinoline-4-carboxylic acid

To 1.5 grams (4.24 mmol) of ethyl 8-chloro-1-ethyl-7-fluoro-5-oxo-5H-thiazolo(3,2-a)-quinoline-4-carboxylate were added 1.2 grams of potassium hydroxide, 100 ml of water and 100 ml of ethanol and the mixture was heated to reflux for one hour. Ethanol was removed therefrom under reduced pressure, acetic acid was added to the residue, crystals separated out therefrom on acidification were collected by filtration, washed with water, dried and recrystallized from dimethyl formamide to give 1.25 grams of 8-chloro-1-ethyl-7-fluoro-5-oxo-thiazolo(3,2-a)-quinoline-4-carboxylic acid. The yield was 91 percent . Melting point was 274 to 5°C (with decomposition). Elementary analysis calculated as $C_{14}H_{19}ClFNO_3S$: C 51.62, H 5.88, N 4.30; Found: C 51.59, H 6.21, N 4.51.

Infrared absorption spectra (KBr,$cm^{-1}$): 2700 to 2000, 1690, 1584, 1470.

Nuclear magnetic resonance spectra δ ($CF_3CO_2D$): 1.71 (3H, triplet, $—CH_2\underline{CH_3}$), 3.62 (2H, quartet, $—\underline{CH_2}CH_3$), 7.76 (1H, singlet, $C_2—H$), 8.43 (1H, doublet, $C_6—H$), 8.94 (1H, doublet, $C_9—H$).

Example 53

1-Ethyl-7-fluoro-5-oxo-8-(1-piperazinyl)-5H-thiazolo(3,2-a)-quinoline-4-carboxylic acid

One gram (3.1 mmol) of 8-chloro-1-ethyl-7-fluoro-5-oxo-5H-thiazolo(3,2-a)-quinoline-4-carboxylic acid was heated to reflux for fifty two hours in a mixture of 2.7 grams (31.0 mmol) of anhydrous piperazine and 50 ml of pyridine. After the reaction was completed, the mixture was allowed to cool to room temperature, crystals separated out therefrom were collected by filtration, washed with ether, purified by silica gel chromatography, and recrystallized from dimethyl formamide to give 140 mg of the title product. The yield was 12 percent. Melting point was 247 to 251°C. Elementary analysis calculated as $C_{18}H_{18}FN_3O_3S$: C 57.59, H 4.83, N 11.19; Found: C 57.42, H 4.99, N 11.08.

Infrared absorption spectra (KBr, $cm^{-1}$): 2700 to 2000, 1680, 1630, 1590i, 1490.

Nuclear magnetic resonance spectra δ ($CF_3CO_2D$): 1.68 (3H, triplet, —$OCH_2CH_3$),

3.00 to 4.47 (11H, triplet, —$O\underline{CH_2}CH_3$, 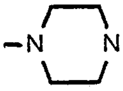),

7.71 (1H, singlet, $C_2$—H), 8.18 (1H, doublet, $C_9$—H), 8.37 (1H, doublet, $C_6$—H).

Example 54

1-Ethyl-7-fluoro-8-(4-methyl-1-piperazinyl)-5-oxo-5H-thiazolo(3,2-a)-quinoline-4-carboxylic acid

To 1.0 gram (3.07 mmol) of 8-chloro-1-ethyl-7-fluoro-5-oxo-5H-thiazolo(3,2-a) quinoline 4-carboxylic acid were added 3.1 grams of N-methyl piperazine and 100 ml of pyridine and the mixture was heated to reflux for forty two hours. Pyridine was evaporated therefrom under reduced pressure, the residue was washed with ether, purified by silica gel chromatography, and recrystallized from dimethyl formamide to give 337 mg of the title compound. The yield of 28 percent. Melting point was 252 to 255°C (with decomposition). Elementary analysis calculated as $C_{19}H_{20}FN_3O_3S$: C 58.60, H 5.18, N 10.79; Found: C 58.43, H 5.20, N 10.52.

Infrared absorption spectra (KBr, $cm^{-1}$): 1710, 1630, 1590, 1530, 1485.

Nuclear magnetic resonance δ ($CF_3CO_2D$): 1.68 (3H, triplet, —$OCH_2\underline{CH_3}$), 3.32 (3H, singlet, $NCH_3$),

3.00 to 4.47 (11H, multiplet, —$O\underline{CH_2}CH_3$, 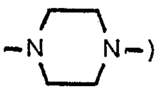),

7.71 (1H, singlet, $C_2$—H), 8.18 (1H, doublet, $C_9$—H), 8.37 (1H, doublet, $C_6$—H).

Example 55

Ethyl-8-chloro-1-ethoxycarbonylmethyl-7-fluoro-5-oxo-5H-thiazolo(3,2-a)-quinoline-4-carboxylate

a) To 3.0 grams (10 mmol) of ethyl 7-chloro-6-fluoro-4-hydroxy-2-mercaptoquinoline-3-carboxylate was added a solution of 0.25 gram (10.87 mmol) of sodium in 100 ml of ethanol. The mixture was stirred for about twenty minutes, then 1.96 grams (11.91 mmol) of ethyl 4-chloroacetoacetate was added thereto, and the mixture was stirred at room temperature for twelve hours. Ethanol was evaporated therefrom under reduced pressure, 100 ml of water was added to the residue, crystals appeared thereby were collected by filtration, and recrystallized from ethanol to give 3,85 grams of ethyl 7-chloro-2-(3-ethoxycarbonyl-2-oxo-propylthio)-6-fluoro-4-hydroxyquinoline-3-carboxylate. The yield was 86 percent. Melting point was 167 to 170°C. Elementary anaylsis calculated as $C_{18}H_{17}ClFNO_6S$; C 50.30, H 3.90, N 3.26; Found: C 50.51, H 4.15, N 3.42.

Infrared absorption spectra (KBr, $cm^{-1}$): 3170, 1735, 1670, 1596, 1465.

Nuclear magnetic resonance spectra δ ($CF_3CO_2D$): 1.15 (3H, —$OCH_2\underline{CH_3}$), 1.39 (3H, triplet, —$OCH_2\underline{CH_3}$), 3.24 (2H, singlet, —CO—$\underline{CH_2}CO_2$—), 4.35 (2H, singlet, —$S\underline{CH_2}CO$), 4.39 (2H, quartet, —$O\underline{CH_2}CH_3$), 4.84 (2H, quartet, —$O\underline{CH_2}$), 8.21 (1H, doublet, $C_5$—H), 9.20 (1H, doublet, $C_8$—H).

b) To 2.0 grams (4.65 mmol) of ethyl 7-chloro-2-(3-ethoxycarbonyl-2-oxopropylthio)-6-fluoro-4-hydroxyquinoline-3-carboxylate was added 10 grams of sulfuric acid and the mixture was heated with stirring at 70°C for about twenty minutes. This was cooled to room temperature, then 20 grams of ice was added thereto, crystals separated out therefrom were collected by filtration, washed with water, dried and recrytallized from ethanol to give ethyl 8-chloro-1-(ethoxycarbonylmethyl)-7-fluoro-5-oxo-5H-thiazolo(3,2-a)-quinoline-4-carboxylate. The yield was 1.66 grams (86 percent). Melting point was 214 to 218°C (with decomposition). Elementary analysis calculated as $C_{18}H_{15}ClFNO_5S$: C 52.50, H 3.67, N 3.40; Found: C 52.59, H 3.91, N 3.62.

Infrared absorption spectra (KBr, $cm^{-1}$): 1730, 1661, 1630, 1608, 1460.

Nuclear magnetic resonance spectra δ ($CF_3CO_2D$): 1.45 (3H, triplet, —$OCH_2\underline{CH_3}$), 1.74 (3H, triplet, —$OCH_2\underline{CH_3}$), 4.42 (2H, quartet, —$O\underline{CH_2}CH_3$), 8.00 (1H, singlet, $C_2$—H), 8.56 (1H, doublet, $C_9$—H), 8.57 (1H, doublet, $C_6$—H).

Example 56

1-Carboxymethyl-8-chloro-7-fluoro-5-oxo-5H-thiazolo(3,2-a)-quinoline-4-carboxylic acid

To 1.5 grams (3.64 mmol) of ethyl 8-chloro-1-(ethoxycarbonylmethyl)-7-fluoro-5-oxo-5H-thiazolo(3,2-a) quinoline 4-carboxylate were added 1.0 grams (18.21 mmol) of potassium hydroxide, 10 ml of water and 100 ml of ethanol and the mixture was heated to reflux for four hours. Ethanol was evaporated therefrom under reduced presdsure, 20 ml of water was added to the residue so that the residue was dissolved therein, then acidified with acetic acid with stirring and ice cooling, crystals separated out therefrom were collected by filtration, washed with water, dried and recrystallized from dimethyl formamide to give 1.21 grams of 8-chloro-1-(carboxymethyl)-7-fluoro-5-oxo-5H-thiazolo(3,2-a)-quinoline-4-carboxylic acid. The yield was 94 percent. Melting point was 236 to 238°C (with decomposition). Elementary analysis calculated as $C_{14}H_{17}ClFNO_5S$: C 47.27, H 4.82, N 3.94; Found: C 47.41, H 4.91, N 4.01.

Infrared absorption spectra (KBr, $cm^{-1}$): 1715, 1685, 1615, 1590, 1480.

Nuclear magnetic resonance spectra δ ($CF_3CO_2D$): 4,85 (2H, singlet, —$CH_2CO_2H$), 8.05 (1H, singlet, $C_2$—H), 8.36 (1H, singlet, $C_9$—H), 8.68 (1H, singlet, $C_6$—H).

Examples 57

1-Carboxymethyl-7-fluoro-8-(4-methyl-1-piperazinyl)-5-oxo-5H-thiazolo(3,2-a)-quinoline-4-carboxylic acid

To 1.0 gram (2.81 mmol) of 8-chloro-1-(carboxymethyl)-7-fluoro-5-oxo-5H-thiazolo(3,2-a)-quinoline-4-carboxylic acid were added 2.8 grams (28.10 mmol) of N-methyl piperazine and 40 ml of pyridine and the mixture was heated to reflux for forty eight hours. Pyridine was evaporated therefrom under reduced pressure, ether was added to the residue, the mixture was washed, then washed with small amount of ethanol, dried, purified by silica gel column chromatography, and recrystallized from dimethyl formamide to give 420 mg of the title compound. The yield was 36 percent. Melting point was 286 to 288°C (with decomposition). Elementary analysis calculated as $C_{19}H_{18}FN_3O_5S$: C 54.41, H 4.33, N 10.02; Found: C 54.72, H 4.18, N 10.16.

Infrared absorption spectra (KBr, $cm^{-1}$): 2700 to 2000, 1690, 1630, 1490, 1450.

Nuclear magnetic resonance spectra δ ($CF_3CO_2D$): 3.25 (3H, singlet, $NCH_3$), 3.30 (2H, singlet, $CH_2CO_2H$),

3.45 to 4.40 (8H, multiplet, 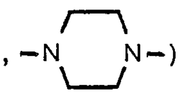),

7.65 (1H, singlet, $C_2$—H), 8.25 )1H, doublet, $C_9$—H), 8.36 (1H, doublet, $C_6$—H).

Example 58

Ethyl 8-chloro-1-(2-ethoxycarbonylethyl)-7-fluoro-5-oxo-5H-thiazolo(3,2-a)-quinoline-4-carboxylate

a) A solution prepared from 0.38 gram (16.6 mmol) of sodium and 200 ml of ethanol was added to five grams (16.6 mmol) of ethyl 7-chloro-6-fluoro-4-hydroxy-2-mercaptoquinoline 3-carboxylate and the mixture was stirred at room temperature for about twenty minutes. The mixture was stirred for further four hours at room temperature after addition of 3.3 grams (18.2 mmol) of ethyl 8-chloro-gamma-oxo-valerate. Ethanol was evaporated therefrom under reduced pressure, water was added to the residue, crystals were collected therefrom by filtration, washed with water, dried and recrystallized from ethanol to give 6.6 grams of ethyl 7-chloro-2-(4-ethoxycarbonyl-2-oxo-1-butylthio)-6-fluoro-4-hydroxyquinoline-3-carboxylate. The yield was 90 percent. Melting point was 144.4°C. Elementary analysis calculated as $C_{19}H_{19}ClFNO_6S$: C 51.41, H 4.32, N 3.16; Found: C 51.62, H 4.39, N 3.29.

Infrared absorption spectra (KBr, $cm^{-1}$): 3240, 1742, 1676, 1591, 1570, 1485.

Nuclear magnetic resonance spectra δ ($CF_3CO_2D$): 1.45 (3H, triplet, —$OCH_2CH_3$), 1.64 (3H, triplet, —$OCH_2CH_3$), 2.50 to 3.36 (4H, wide singlet, —$CH_2CH_2$), 3.95 (2H, singlet, —$SCH_2CO$—), 4.37 (2H, quartet, —$OCH_2CH_3$), 4.75 (2H, quartet, —$OCH_2CH_3$), 8.22 (1H, doublet, $C_8$—H), 9.16 (1H, doublet, $C_5$—H).

b) To 6.0 grams (13.52 mmol) of ethyl 7-chloro-2-(4-ethoxycarbonyl-2-oxo-1-butylthio)-6-fluoro-4-hydroxyquinoline-3-carboxylate was added 30 grams of sulfuric acid. The mixture was stirred at room temperature for about thirty minutes and then heated at 70°C for about twenty minutes with stirring. After cooled, 100 grams of ice was added thereto crystals separated out therefrom were collected by filtration, washed with water, dried and recrystallized from ethanol to give 5.30 grams of ethyl 8-chloro-1-(2-ethoxycarbonylethyl)-7-fluoro-5-oxo-5H-thiazolo(3,2-a)-quinoline-4-carboxylate. The yield was 92 percent. Melting point was 200.0°C. Elementary analysis calculated as $C_{19}H_{17}ClFNO_5S$: C 53.59, H 4.02, N 3.29: Found: C 53.51, H 4.40, N 3.49.

Infrared absorption spectra (KBr, $cm^{-1}$): 1730, 1660, 1635, 1612, 1460.

Nuclear magnetic resonance spectra δ ($CF_3CO_2D$): 1.42 (3H, triplet, —$OCH_2CH_3$), 1.70 (3H, triplet, —$OCH_2CH_3$), 3.21 (2H, triplet, —$CH_2CH_2$), 4.04 (3H, triplet, —$CH_2CH_2$), 4.39 (2H, quartet, —$OCH_2CH_3$), 4.84 (2H, quartet, —$OCH_2CH_3$), 7.82 (1H, singlet, $C_2$—H), 8.48 (1H, doublet, $C_6$—H) 8.86 (1H, doublet, $C_9$—H).

Example 59

1-(2-Carboxyethyl)-8-chloro-7-fluoro-5-oxo-5H-thiazolo(3,2-a)-quinoline-4-carboxylic acid

To 4.2 grams (9.86 mmol) of ethyl 8-chloro-1-(2-ethoxycarbonylethyl)-7-fluoro-5-oxo-5H-thiazolo(3,2-a)-quinoline-4-carboxylate were added 3.9 grams (69.02 mmol) of potassium hydroxide, 100 ml of water and 100 ml of ethanol and the mixture was heated to reflux for four hours. Ethanol was evaporated therefrom under reduced pressure and the residue was acidified with acetic acid. Crystals separated out therefrom were collected by filtration, washed with water, dried and recrystallized from dimethyl formamide to give 3.29 grams of 1-(2-carboxyethyl)-8-chloro-7-fluoro-5-oxo-5H-thiazolo(3,2-a)-quinoline-4-carboxylic acid. The yield was 90 percent. Melting point was 298 to 301°C (with decomposition). Elementary analysis calculated as $C_{15}H_{19}ClFNO_5S$: C 48.73, H 5.18, N 3.79; Found: C 48.91, H 5.24, N 3.81.

Infrared absorption spectra (KBr, $cm^{-1}$): 2700 to 2000, 1730, 1680, 1530, 1440.

Nuclear magnetic resonance spectra δ ($CF_3CO_2D$): 3.20 (2H, triplet, —$CH_2CH_2$—), 3.95 (2H, triplet, —$CH_2CH_2$—), 7.84 (1H, singlet, $C_2$—H), 8.50 (1H, doublet, $C_6$—H), 8.86 (1H, doublet, $C_9$—H).

Example 60

1-(2-Carboxyethyl)-7-fluoro-8-(4-methyl-1-piperazinyl)-5-oxo-5H-thiazolo(3,2-a)-quinoline-4-carboxylic acid

To one gram (2.705 mmol) of 1-(2-carboxyethyl)-8-chloro-7-fluoro-5-oxo-5H-thiazolo(3,2-a) quinoline 4-carboxylic acid were added 2.7 grams (27.05 mmol) of N-methyl piperazine and 70 ml of pyridine and the mixture was heated to reflux for sixty eight hours. Pyridine was evaporated therefrom under reduced pressure, ether was added to the residue and washed, purified by silica gel chromatography, and recrystallized from dimethyl formamide to give 175 mg of the title compound. Yield was 15 percent and melting point was 286°C (with decomposition). Elementary analysis calculated as $C_{20}H_{20}FN_3O_5S$: C 55.42, H 4.65, N 9.69; Found: C 55.16, H 4.85, N 9.42.

Infrared absorption spectra (KBr, $cm^{-1}$): 1690, 1630, 1584, 1482, 1456.

Nuclear magnetic resonance spectra δ ($CF_3CO_2D$): 3.20 (3H, singlet, N$\underline{CH_3}$),

3.02 to 4.50 (12H, multiplet, $CH_2CH_2$, 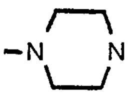 ),

7.84 (1H, singlet, $C_2$—H), 8.50 (1H, doublet, $C_6$—HH), 8.86 (1H, doublet, $C_9$—H).

Example 61

Ethyl 8-chloro-1-chloromethyl-7-fluoro-5-oxo-5H-thiazolo(3,2-a)-quinoline-4-carboxylate

A mixture of 10.0 grams (33 mmol) of ethyl 7-chloro-6-fluoro-4-hydroxy-2-mercaptoquinoline-3-carboxylate and 5.04 grams (40 mmol) of dischloroacetone was heated with stirring for fifteen hours at 100°C in 100 ml of ethanol. After cooled, crystals separated out therefrom were collected by filtration to give 11.40 grams of colorless crystals. These crystals were dissolved in 50 grams of sulfuric acid with cooling, stirred for two hours, poured over ice water, crystals separated out were collected by filtration, washed with water, dried with air and recrystallized from dimethyl formamide to give 9.01 grams of the title compound. The yield was 73.2 percent and melting point was 256°C (with decomposition). Elementary analysis calculated as $C_{15}H_{10}FCl_2NO_3S$: C 48.18, H 2.69, N 3.74; Found: C 48.16, H 2.70, N 3.58.

Infrared absorption spectra (KBr, $cm^{-1}$): 3075, 1657, 1610, 1510, 1460, 1040.

Nuclear magnetic resonance spectra δ ($CF_3CO_2D$): 1.66 (3H, triplet), 4.83 (2H, quartet, —O$\underline{CH_2}$$CH_3$), 5.31 (2H, singlet, —$\underline{CH_2}$Cl), 8.14 (1H, singlet, $C_2$—H), 8.45 (2H, triplet, $C_6$—H), 9.02 (1H, doublet, $C_9$—H).

Example 62

Ethyl 8-chloro-7-fluoro-1-(4-methyl-1-piperazinylmethyl)-5-oxo-5H-thiazolo(3,2-a)-quinoline-4-carboxylate

Two grams (5.3 mmol) of ethyl 8-chloro-1-chloromethyl-7-fluoro-5-oxo-5H-thiazolo(3,2-a) quinoline 4-carboxylate was dissolved in 20 ml of dimethyl formamide, then 0.88 gram (6.4 mmol) of potassium carbonate and 0.64 gram (6.4 mmol) of N-methyl piperazine were added thereto, and the mixture was heated with stirring for one hour at 80°C. The content was concentrated under reduced pressure, water was added to the residue, crystals separated out therefrom were collected by filtration, washed with water, dried with air and recrystallized from dimethyl formamide to give the title compound in 1.72 grams (74.8 percent) yield. Melting point was 265 to 7°C (with decomposition). Elementary analysis calculated as $C_{20}H_{21}FClN_3O_3S$: C 54.85, H 4.83, N 9.60; Found: C 54.65, H 4.88, N 9.39.

Infrared absorption spectra (KBr, $cm^{-1}$): 2800, 1660, 1635, 1602, 1500, 1460.

Nuclear magnetic resonance spectra δ ($CF_3CO_2D$): 1.68 (3H, triplet, —$OCH_2$$\underline{CH_3}$), 3.12 (3H, singlet, =N$\underline{CH_3}$),

2.80 to 4.20 (8H, multiplet, 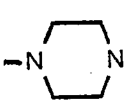 ),

4.65 (2H, singlet, —$\underline{CH_2}$N=), 4.82 (2H, quartet, —O$\underline{CH_2}$$CH_3$), 8.21 (1H, singlet, $C_2$—H), 8.40 (1H, doublet, $C_6$—H), 9.38 (1H, doublet, $C_9$—H).

Example 63

8-Chloro-7-fluoro-1-(4-methyl-1-piperzinyl)methyl-5-oxo-5H-thiazolo(3,2-a)-quinoline-4-carboxylic acid

Ethyl 8-chloro-7-fluoro-1-(4-methyl-1-piperazinyl)methyl-5-oxo-5H-thiazolo(3,2-a)-quinoline-4-carboxylate (0.72 gram, 1.6 mmol) was heated to reflux for one hour in a mixture of 0.18 gram (3.2 mmol) of potassium hydroxide, 1 ml of ethanol and 5 ml of water. The mixture was then acidified with acetic acid, crystals separated out thereby were collected by filtration, and recrystallized from dimethyl formamide to give 0.45 gram (68.2 percent) of the title compound. Melting point was 303°C (with decomposition). Elementary analysis calculated as $C_{18}H_{17}FClN_3O_3S.3H_2O$: C 46.60, H 5.00, N 9.06; Found: C 46.57, H 4.61, N 9.93.

Infrared absorption spectra (KBr, $cm^{-1}$): 1700, 1590, 1515, 1480.

Nuclear magnetic resonance spectra δ ($CF_3CO_2D$): 3.10 (3H, singlet, =N $\underline{CH_3}$),

3.50 to 4.30 (8H, multiplet, 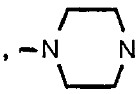),

5.15 (2H, singlet, —$\underline{\underline{CH_2}}$N=), 8.40 (1H, doublet, $C_9$—H), 8.47 (1H, singlet, $C_2$—H), 8.95 (1H, doublet, $C_6$—H).

Example 64

Ethyl 8-chloro-1-(N,N-dimethylaminomethyl)-7-fluoro-5-oxo-5H-thiazolo(3,2-a)-quinolone-4-carboxylate

A mixture of 1.38 grams (3.7 mmol) of ethyl 8-chloro-1-chloromethyl-7-fluoro-5-oxo-5H-thiazolo(3,2-a)-quinoline-4-carboxylate, 710 mg (8.7 mmol) of dimethyl amine hydrochloride, and 1.84 grams (17.4 mmol) of sodium carbonate was suspended in 100 ml of ethanol and the whole was heated to reflux. After twenty hours, the reaction mixture was cooled to room temperature and concentrated under reduced pressure. Water was added to the residue. Insoluble matter obtained thereby was collected by filtration, washed with ethanol and recrystallized from dimethyl formamide to give 1.35 grams (66.8 percent yield) of the title compound. Melting point was 276°C.

Infrared absorption spectra (KBr, $cm^{-1}$): 1655, 1625, 1605, 1500, 1460.

Nuclear magnetic resonance spectra δ ($CF_3CO_2D$): 1.70 (3H, triplet, —$OCH_2\underline{CH_3}$), 3.29 (6H, singlet, —$N(H_3)_2$), 4.88 (2H, quartet, —$O\underline{\underline{CH_2}}CH_3$), 5.52 (2H, singlet, —$CH_2$—N=), 8.30 to 8.70 (3H, multiplet, $C_2$—H, $C_6$—H, $C_9$—H).

Example 65

8-Chloro-1-(N,N-dimethylaminomethyl)-7-fluoro-5-oxo-5H-thiazolo(3,2-a)-quinoline-4-carboxylic acid

Ethyl 8-chloro-1-(N,N-dimethylaminomethyl)-7-fluoro-5-oxo-5H-thiazolo(3,2-a)-quinoline-4-carboxylate (1.35 grams, 3.52 mmol) was suspended in 100 ml of 50% aqueous ethanol and, after addition of 10 ml of 2N-aqueous solution of sodium hydroxide, the mixture was heated to reflux. After one hour, the reaction solution was cooled to room temperature and ethanol was evaporated therefrom under reduced pressure. To the concentrated residual solution was added acetic acid with cooling to adjust to pH 7.0 and the mixture was stirred for a while. Crystals separated therefrom were collected by filtration, washed with water, and dried to give 1.08 grams (yield: 86.4 percent) of the title compound. Melting point was 271.5 to 2°C (with decomposition).

Infrared absorption spectra ($\nu_{max}^{KBr}$ $cm^{-1}$) 1690, 1645, 1610, 1500, 1475.

Nuclear magnetic resonance spectra ($CF_3CO_2D$, δ): 3.30 (6H, singlet, —$N(CH_3)_2$), 5.52 (2H, wide singlet, —$CH_2$N=), 8.25 to 8.70 (3H, multiplet, $C_2$—H, $C_6$—H, $C_9$—H).

Elementary analysis calculated as $C_{15}H_{12}ClFN_2O_3S.1\frac{1}{2}H_2O$): C 47.19, H 3.96, N 7.34; C 47.34, H 3.46, N 7.01.

Example 66

1-Dimethylaminomethyl-7-fluoro-5-oxo-8-(1-piperazinyl)-5H-thiazolo(3,2-a)-quinoline-5-carboxylic acid

A mixture of 900 mg (2.54 mmol) of 8-chloro-1-dimethylaminomethyl-7-fluoro-5-oxo-5H-thiazolo(3,2-a)-quinoline-5-carboxylic acid and 2.2 grams (25.4 mmol) of anhydrous piperazine was suspended in 20 ml of pyridine and heated to reflux. After seventeen hours, the reaction solution was cooled to room temperature, pyridine was evaporated therefrom under reduced pressure, ethanol was added to the resulting residue, the insoluble matter obtained was collected by filtration, washed with ethanol and ether, and dried. The resulting crystals were purified by silica gel column chromatography to give the title compound. Yield was 75 mg (7.3 percent) and melting point was 246°C (with decomposition).

Infrared absorption spectra ($\nu_{max}^{KBr}$ $cm^{-1}$): 1680, 1630, 1495, 800.

Nuclear magnetic resonance spectra ($CF_3CO_2D$) δ: 3.11 (6H, singlet, $NCH_3 \times 2$),

3.40 to 4.40 (8H, multiplet, 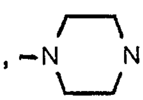),

5.45 (2H, wide singlet, $CH_2$N=), 7.55 (1H, doublet, $C_9$—H), 8.32 (1H, doublet, $C_5$—H), 8.46 (1H, singlet, $C_1$—H).

Elementary analysis calculated as $C_{19}H_{21}FN_4O_3S$: C 56.42, H 5.23, N 13,87; Found: C 56.53, H 5.28, N 13.80.

Example 67

1-(N,N-Dimethylaminomethyl)-7-fluoro-8-(4-methyl-1-piperazinyl)-5-oxo-5H-thiazolo(3,2-a)-quinoline-4-carboxylic acid

A mixture of 920 mg (2.6 mmol) of 8-chloro-1-(N,N-dimethylamino)-7-fluoro-5-oxo-5H-thiazolo(3,2-a)-quinoline-4-carboxylic acid and 2.6 grams (26 mmol) of N-methyl piperazine was added to 20 ml of pyridine and the whole was heated to reflux. After sixty five hours, the reaction solution was cooled to room temperature and pyridine was evaporated therefrom under reduced pressure. To the residue was added ethanol, insoluble matter obtained thereby was collected by filtration, and washed with ether. The resulting crystals were purified by subjecting to silica gel column chromatography to give 250 mg (yield: 23 percent) of the title compound. Melting point was 241 to 2—C (with decomposition).

Infrared absorption spectra (KBr, $cm^{-1}$): 1690, 1630, 1580, 1490.

Nuclear magnetic resonance spectra δ ($CF_3CO_2D$): 3.24 (9H, wide singlet, —$N(CH_3)_2$, =$NCH_3$), 3.00 to 4.50 (8H, multiplet, 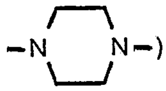), 5.51 (2H, wide singlet, —$CH_2N$=), 8.20 to 8.70 (3H, multiplet, $C_2$—H, $C_6$—H, $C_9$—H).

Elementary analysis calculated as $C_{20}H_{23}FN_4O_3S.\frac{1}{2}H_2O$: C 56.26, H 5.66, N 13.11; Found: C 56.32, H 5.41, N 12.87.

Example 68

Methyl 8-chloro-7-fluoro-1-methoxymethyl-5-oxo-5H-thiazolo(3,2-a)-quinoline-4-carboxylate

Ethyl 8-chloro-1-chloromethyl-7-fluoro-5-oxo-5H-thiazolo(3,2-a) quinoline-4-carboxylate (1.5 grams, 4.0 mmol) was suspended in anhydrous methanol, then sodium methoxide solution prepared from 2.1 grams of metal sodium and 50 ml of anhydrous methanol was added, and the mixture was heated to reflux for twenty minutes. After cooled, water was added to the reaction solution, crystals separated out therefrom were collected by filtration, washed with water, and dried to give 630 mg of the title compound. The yield was 44.4 percent and melting point was 239 to 40°C. Elementary analysis calculated as $C_{15}H_{11}ClFNO_4S$: C 50.64, H 3.12, N 3.94; Found: C 50.51, H 3.08, N 3.80.

Infrared absorption spectra (KBr, $cm^{-1}$): 1665, 1630, 1610, 1470.

Nuclear magnetic resonance spectra δ ($CF_3CO_2D$): 3.79 (3H, singlet, —O$\underline{CH_3}$) 4.32 (3H, singlet, C(:O)O$\underline{CH_3}$), 5.11 (2H, singlet, —$\underline{CH_2}$—), 8.15 (1H, singlet, $C_2$—H), 8.44 (1H, doublet, $C_6$—H), 9.12 (1H, doublet, $C_9$—H).

Example 69

8-Chloro-7-fluoro-1-methoxymethyl-5-oxo-5H-thiazolo(3,2-a)-quinoline-4-carboxylic acid

Methyl 8 - chloro - 7 - fluoro - 1 - methoxymethyl - 5 - oxo - 5H - thiazolo(3,2-a)-quinoline-4-carboxylate (1.8 grams, 3.0 mmol) was suspended in 100 ml of 50% aqueous alcohol, 10 ml of 2N aqueous sodium hydroxide solution was added thereto, and the mixture was heated to reflux for twenty minutes. When the reaction was completed, ethanol was evaporated therefrom under reduced pressure, the residue was adjusted to pH 7.0 with acetic acid, stirred for a while, crystals separated out thereby were collected by filtration, washed with tetrahydrofuran and ether, and dried with air to give the title compound. The yield was 945 mg (92.1 percent) and melting point was 298°C (with decomposition). Elementary analysis calculated as $C_{14}H_9ClFNO_4S$: C 59.20, H 2.65, N 4.10; Found: C 49.11, H 2.60, N 4.00.

Infrared absorption spectra (KBr, $cm^{-1}$): 1690, 1590, 1480.

Nuclear magnetic resonance spectra δ ($CF_3CO_2D$): 3.80 (3H, singlet, O$\underline{CH_3}$), 5.12 (2H, singlet, —$\underline{CH_2}$O—), 8.15 (1H, singlet, $C_2$—H), 8.25 to 8.70 (1H, multiplet, $C_6$—H), 8.75 to 9.30 (1H, multiplet, $C_9$—H).

Example 70

7-Fluoro-1-methoxymethyl-8-(4-methyl-1-piperazinyl)-5-oxo-5H-thiazolo(3,2-a)-quinoline-4-carboxylic acid

A mixture of 878 mg (2.66 mmol) of 8-chloro-7-fluoro-1-methoxymethyl-5-oxo-5H-thiazolo(3,2-a)-quinoline-4-carboxylic acid and 2.7 grams (26.6 mmol) of N-methyl piperazine was suspended in 20 ml of pyridine and the suspension was heated to reflux. After fifty five hours, the reaction solution was evaporated under reduced pressure, ethanol was added to the resulting residue, and insoluble matter obtained thereby was collected by filtration. This was subjected to silica gel column chromatography and recrystallized from aqueous dimethyl formamide to give 220 mg of the title compound. The yield was 20.4 percent and melting point was 243 to 244°C (with decomposition).

Infrared absorption spectra (KBr, $cm^{-1}$): 1690, 1630, 1580, 1490.

Nuclear magnetic resonance spectra δ ($CF_3CO_2D$): 3.21 (3H, singlet, N—$CH_3$), 3.69 (3H, singlet, —$OCH_3$), 3.35 to 4.50 (8H, multiplet, hydrogen atom in piperazine), 5.12 (2H, singlet, —$CH_2OCH_3$), 8.05 (1H, singlet, $C_2$—H), 8.25 (1H, doublet, $C_9$—H), 8.80 (1H, singlet, $C_6$—H).

Elemental analysis calculated as $C_{19}H_{20}FN_3O_4S.H_2O$: C 53.89, H 5.2, N 9.92; Found: C 54.23, H 4.8, N 9.69.

Example 71

8-Chloro-7-fluoro-1-hydroxymethyl-5-oxo-5H-thiazolo(3,2-a)-quinoline-4-carboxylic acid

Three grams (0.8 mmol) of ethyl 8-chloro-1-chloromethyl-7-fluoro-5-oxo-5H-thiazolo(3,2-a) quinoline 4-carboxylate was dissolved in 20 ml of dimethylformamide, 0.72 gram (0.88 mmol) of sodium acetate was added thereto, and the mixture was heated with stirring at 150°C for twelve hours. The content was concentrated under reduced pressure, water was added thereto, and the resulting crystals which were insoluble in water were collected by filtration. The crystals were suspended in a mixture of 1.28 grams of sodium hydroxide, 80 ml of water and 20 ml of ethanol and the suspension was heated with stirring at 140°C for two hours. Then this was acidified with acetic acid, crystals separated out therefrom were collected by filtration, washed with water, and dried with air and recrystallized from dimethyl formamide to

give the title compound. Colorless crystals. The yield was 1.47 grams (56.1 percent). Melting point was 298—9°C (with decomposition). Elementary analysis calculated as $C_{13}H_7ClFNO_4S$: C 47.65, H 2.15,. N 4.27; Found: C 47.83, H 2.09, N 4.00.

Infrared absorption spectra (KBr, $cm^{-1}$): 1665, 1580, 1480.

Nuclear magnetic resonance spectra δ ($CF_3CO_2D$): 5.50 (2H, singlet, —$\underline{CH_2}$OAc), 8.15 (1H, singlet, $C_2$—H), 8.42 (1H, doublet, $C_6$—H), 9.15 (1H, doublet, $C_9$—H).

Example 72

7-Fluoro-1-hydroxymethyl-8-(4-methyl-1-piperazinyl)-5-oxo-5H-thiazolo(3,2-a)-quinoline-4-carboxylic acid

A mixture of 1.49 grams (4.55 mmol) of 8-chloro-7-fluoro-1-hydroxymethyl-5-oxo-5H-thiazolo(3,2-a) quinoline 4-carboxylic acid, 4.6 grams (45.5 mmol) of N-methyl piperazine and 50 ml of pyridine was heated to reflux at 150°C for seventy two hours. After cooled, crystals separated out therefrom were collected by filtration and recrystallized from dimethyl formamide to give the title compound. Colorless crystals. The yield was 700 mg (39.3 percent) and melting point was 302 to 3°C (with decomposition). Elementary analysis calculated as $C_{18}H_{18}FN_3O_4$: C 55.23, H 4.64, N 10.74; Found: C 55.15, H 4.52, N 10.30.

Infrared absorption spectra (KBr, $cm^{-1}$): 1670, 1625, 1580, 1490, 1380, 1275.

Nuclear magnetic resonance spectra δ ($CF_3CO_2D$): 3.15 (3H, singlet, =$NCH_3$),

3.00 to 4.50 (8H, multiplet, 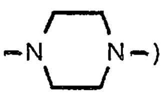),

5.31 (2H, singlet, —$\underline{CH_2}$OH), 7.97 (1H, singlet, $C_2$—H), 8.25 (1H, doublet, $C_6$—H) 8.69 (1H, doublet, $C_9$—H).

Example 73

1-Acetoxymethyl-7-fluoro-8-(4-methyl-1-piperazinyl)-5-oxo-5H-thiazolo(3,2-a)-quinoline-4-carboxylic acid

7-Fluoro-1-hydroxymethyl-8-(4-methyl-1-piperazinyl)-5-oxo-5H-thiazolo(3-2-a)-quinoline-4-carboxylic acid (270) mg, 0.69 mmol) was dissolved in 30 ml of pyridine, then 300 mg (2.9 mmol) of acetic anhydride was added thereto, and the mixture was heated with stirring at 90°C for three hours. Insoluble matter obtained thereby was filtered off, the mother liquor was concentrated, and the crystalline residue was recrystallized from a mixture of chloroform and ether to give the title compound. Colorless crystals. The yield was 220 mg (73.6 percent) and melting point was 237 to 8°C (with decomposition). Elementary analysis calculated as $C_{20}H_{20}FN_3O_5S.H_2O$: C 53.21, H 4.91, N 9.31; Found: C 53.50, H 4.54, N 8,86.

Infrared absorption spectra (KBr, $cm^{-1}$): 1750, 1690, 1630, 1485, 1180, 1270, 1208.

Nuclear magnetic resonance spectra δ ($CF_3CO_2D$): 2.35 (3H, singlet, $COCH_3$), 3.19 (3H, singlet, =$NCH_3$),

3.30 to 4.50 (8H, multiplet, 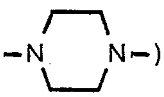),

5.89 (2H, singlet, —$CH_2$OAc), 7.90 to 8.50 (3H, multiplet, $C_2$—H, $C_6$—H, $C_9$—H).

Example 74

8-Chloro-7-fluoro-5-oxo-1-phenyl-5H-thiazolo(3,2-a) quinoline-4-carboxylic acid

Two grams (6.6 mmol) of ethyl 7-chloro-6-fluoro-4-hydroxy-2-mercaptoquinoline-3-carboxylate was suspended in 10 ml of ethanol, then 1.3 grams (6.5 mmol) of phenacyl bromide was added thereto, and the mixture was heated to reflux with vigorous stirring for three hours. After cooled, crystals separated out therefrom were collected by filtration, dried with air, and 2.08 grams of colorless powder was obtained. To this was added 4.5 grams of concentrated sulfuric acid, the mixture was heated at 80 to 90°C for ten minutes cooled, poured over into ice water, crystals separated out were collected by filtration, washed with water, and dried with air. This was suspended in a mixture of 0.29 gram (7.2 mmol) of sodium hydroxide, 60 ml of water and 10 ml of ethanol and the whole mixture was heated to reflux for two hours and a half. Then this was acidified with acetic acid, crystals separated out were collected by filtration, washed with water, dried with air, and recrystallized from dimethyl formamide to give the title compound as colorless crystals in 1.19 grams (48.2 percent yield). Melting point was 330°C (with decomposition). Elementary analysis calculated as $C_{18}H_9ClFNO_3S$: C 57.84, H 2.43, N 3.75; Found C 58.22, H 2.11, N 3.67.

Infrared absorption spectra (KBr, $cm^{-1}$): 3080, 1695, 1585, 1460, 1260, 1045, 800.

Nuclear magnetic resonance spectra δ ($CF_3CO_2D$): 7.40 to 8.00 (7H, multiplet, $C_2$—H, $C_9$—H, phenyl group-H), 8.30 (1H, doublet, $C_6$—H).

Example 75

7-Fluoro-8-(4-methyl-1-piperazinyl)-5-oxo-1-phenyl-5H-thiazolo(3,2-a)-quinoline-4-carboxylic acid and 8-chloro-7-(4-methyl-1-piperazinyl)-5-oxo-1-phenyl-thiazolo(3,2-a)-quinoline-4-carboxylic acid.

A mixture of 970 mg (2.60 mmol) of 8-chloro-7-fluoro-5-oxo-1-phenyl-5H-thiazolo(3,2-a)-quinoline-4-carboxylic acid, 2.6 grams (26 mmol) of N-methyl piperazine and 50 ml of pyridine was heated to reflux for seventy two hours. The content was concentrated under reduced pressure, water was added to the residue,

insoluble matter obtained thereby was collected by filtration, washed with water, dried and recrystallized from dimethyl formamide to give 7-fluoro-8-(4-methyl-1-piperazinyl)-5-oxo-1-phenyl-5H-thiazolo(3,2-a)-quinoline-4-carboxylic acid was colorless crystals in the yield of 494 mg (43.4 percent). Melting point was 300°C (with decomposition). Elementary analysis calculated as $C_{23}H_{20}FN_3O_3S$: C 63.14, H 4.61, N 9.60; Found: C 63.19, H 4.45, N 10.00.

Infrared absorption spectra (KBr, $cm^{-1}$): 1695, 1625, 1480, 1260, 760.

Nuclear magnetic resonance spectra δ ($CF_3CO_2D$): 3.90 (3H, singlet, —$NCH_3$),

3.00 to 4.00 (8H, multiplet, 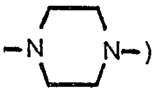),

7.30 (1H, doublet, $C_9$—H), 7.65 (5H, singlet, phenyl group), 7.75 (1H, singlet, $C_2$—HH), 8.25 (1H, doublet, $C_6$—H).

Crystals obtained from the mother liquor were recrystallized from dimethyl formamide to give 8-chloro-7-(4-methyl-1-piperazinyl)-5-oxo-1-phenyl-5H-thiazolo(3,2-a) quinoline 4-carboxylic acid as colorless crystals in the yield of 120 mg (10.2 percent) with a melting point of 207 to 9°C (with decomposition). Elementary analysis calculated as $C_{23}H_{20}ClN_3O_3S$: C 60.86, H 4.44, N 9.26; Found: C 60.83, H 4.49, N 9.35.

Infrared absorption spectra (KBr, $cm^{-1}$): 1690, 1575, 1500, 1480, 1140, 800.

Nuclear magnetic resonance spectra δ ($CF_3CO_2D$): 3.10 (3H, singlet, =$NCH_3$),

3.00 to 4.0 (8H, multiplet, 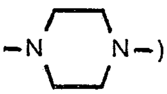),

7.20 to 7.90 (multiplet, phenyl group-H, $C_2$—HH, $C_6$—H), 8.15 (1H, singlet, $C_9$-H).

## Example 76

Ethyl 8-chloro-7-fluoro-1,2-dimethyl-5-oxo-5H-thiazolo(3,2-a)-quinoline-4-carboxylate

(a) To three grams (9.94 mmol) of ethyl 7-chloro-6-fluoro-4-hydroxy-2-mercaptoquinoline-3-carboxylate was added a solution of 0.23 gram of sodium (9,94 mmol) in 100 ml of ethanol and the mixture was stirred at room temperature for eighteen hours after addition of 1.3 grams (12.20 mmol) of 3-chloro-2-butanone. The content was concentrated under reduced pressure, 40 ml of water was added to the residue, crystals obtained thereby were collected by filtration, washed with water and then with ether, dried, and 3.56 grams (96% yield) of ethyl 7-chloro-6-fluoro-4-oxo-2-(2-oxobutyl-3-thio)-1,4-dihydroquinoline-3-carboxylate was obtained. Melting point was 222 to 224°C (with decomposition). Elementary analysis calculated as $C_{17}HH_{16}ClFNO_4S$: C 51.68, H 4.06, N. 3.76; Found: C 51.72, H 4.32, N 3.69.

Infrared absorption spectra (KBr, $cm^{-1}$): 3220, 1680, 1600, 1470.

Nuclear magnetic resonance spectra δ ($CF_3CO_2D$): 1.65 (3H, triplet, —$OCH_2CH_3$), 1.73 (3H, doublet, $SCHCH_3$), 1.92 (3H, singlet, —$COCH_3$), 3.96 to 4.50 (1H, multiplet, =$CHCH_3$), 4.74 (2H, quartet, —$OCH_2CH_3$), 8.24 (1H, doublet, $C_5$—H), 9.30 (1H, doublet, $C_8$—H).

b) To 3.1 grams (8.34 mmol) of ethyl 7-chloro-6-fluoro-4-oxo-2-(2-oxobutyl-3-thio)-1,4-dihydroquinoline-3-carboxylate was added 15 grams of sulfuric acid and the mixture was heated with stirring at 70°C for ten minutes. The content was cooled, added to 20 grams of ice, crystals separated out therefrom were collected by filtration, washed with water and then with ether, and dried to give 2.66 grams (90 percent yield) of the title compound, melting point 223 to 227°C (with composition). Elementary analysis calculated as $C_{16}H_{13}ClFNO_3S$: C 54.31, H 3.70, N 3.95; Found: C 54.62, H 3.81, N 4.07.

Infrared absorption spectra (KBr, $cm^{-1}$): 1660, 1610, 1502, 1452.

Nuclear magnetic resonance spectra δ ($CF_3CO_2D$): 1.74 (3H, triplet, —$OCH_2CH_3$), 2.76 (3H, singlet, $C_2$—$CH_3$), 3.20 (3H, singlet, $C_2$—$CH_3$), 4.85 (2H, quartet, —$OCH_2CH_3$), 8.46 (1H, doublet, $C_6$—H), 9.00 (1H, doublet, $C_9$—H).

## Example 77

8-Chloro-7-fluoro-1,2-dimethyl-5-oxo-5H-thiazolo(3,2-a)-quinoline-4-carboxylic acid

To 2.66 grams (7.52 mmol) of ethyl 8-chloro-7-fluoro-1,2-dimethyl-5-oxo-5H-thiazolo(3,2-a)-quinoline-4-carboxylate were added 40 ml of ethanol and a solution of 1.5 grams (37.60 mmol) of sodium hydoxide in 120 ml of water and the mixture was heated to reflux for five hours on an oil bath. Ethanol was evaporated therefrom under reduced pressure, acetic acid was added to the residue so that it was made acidic, crystals separated out thereby were collected by filtration, washed with water and then with ethanol, dried and the resulting crystals were recrystallized from dimethyl formamide to give 2.3 grams (94 percent yield) of the title compound, melting point 294 to 296°C (with decomposition). Elementary analysis calculated as $C_{14}H_{10}ClFNO_3S$: C 51.62, H 2.78, N 4.29; Found: C 51.84, H 2.69, N 4.51.

Infrared absorption spectra (KBr, $cm^{-1}$): 1690, 1590, 1510, 1470, 1440.

Nuclear magnetic resonance spectra δ ($CF_3CO_2D$): 2.70 (3H, singlet, $C_2$—$CH_3$), 3.14 (3H, singlet, $C_1$—$CHH_3$), 8.46 (1H, doublet, $C_6$—H), 8.95 (1H, doublet, $C_9$—H).

Example 78

7-Fluoro-8-(4-methyl-1-piperazinyl)-1,2-dimethyl-5-oxo-5H-thiazolo(3,2-a)-quinoline-4-carboxylic acid

A mixture of 1.0 gram (3.1 mmol) of 8-chloro-7-fluoro-1,2-dimethyl-5-oxo-5H-thiazolo(3,2-a)-quinoline-4-carboxylic acid, 3.1 grams (31.0 mmol) of N-methyl piperazine and 40 ml of pyridine was heated to reflux on an oil bath for forty eight hours. The content was then concentrated under reduced pressure, water was added to the residue, the insoluble matter thereby obtained were collected by filtration, washed with water and then with ethanol, dried, and the resulting crystals were recrystallized from pyridine for three times to give 0.34 gram (31 percent yield) of the title compound, colorless crystals, melting point 283 to 285°C (with decomposition). Elementary analysis calculated as $C_{19}H_{20}ClFNO_3S$: C 58,59, H 5.17, N 10.78; Found: C 58.71, H 5.18, N 10.51.

Infrared absorption spectra (KBr, $cm^{-1}$): 1690, 1630, 1580, 1485.

Nuclear magnetic resonance spectra δ ($CF_3CO_2D$): 2.67 (3H, singlet, $C_2$—$CH_3$), 3.12 (3H, singlet, $C_1$—$CH_3$),

3.22 (3H, singlet, $\underline{CH_2}$ 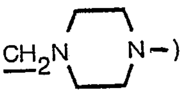), 3.30 to 4.50 (8H, multiplet, 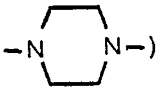),

8.17 (1H, doublet, $C_9$—H), 8.34 (1H, doublet, $C_6$—H).

Example 79

Ethyl 2-chloro-3-fluoro-5-oxo-8,9,10,11-tetrahydro-5H-benzothiazolo(3,2-a)-quinoline-6-carboxylate

a) To three grams (10 mmol) of ethyl 7-chloro-6-fluoro-4-hydroxy-2-mercaptoquinoline 3-carboxylate was added a solution of 0.25 gram of sodium (11 mmol) in 100 ml of ethanol and the mixture was stirred at room temperature for twenty hours after addition of 1.59 grams (12 mmol) of 2-chlorocyclohexanone. Then the mixture was heated to reflux for three hours more. Ethanol was evaporated therefrom under reduced pressure, water was added to the residue and, after being washed with water, it was further washed with ethanol and ether and finally dried to give 3.62 grams (91 percent yield) of ethyl 7-chloro-6-fluoro-4-hydroxy-2-(2-oxocyclohexylthio)-quinoline-3-carboxylate, melting point 252 to 257°C (with decomposition). Elementary analysis calculated as $C_{18}H_{17}ClFNO_4S$: C 54.34, H 4.30, N 3.52; Found: C 54.56, H 4.42, N 3.59.

Infrared absorption spectra (KBr, $cm^{-1}$): 3240, 1682, 1600, 1471.

Nuclear magnetic resonance spectra δ ($CF_3CO_2D$): 1.59 (3H, triplet, —$OCH_2\underline{CH_3}$),

1.10 to 2.80 (8H, multiplet, 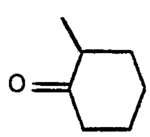), 4,12 to 4.45 (1H, multiplet, 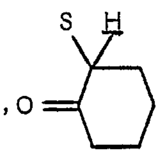),

4.72 (2H, quartet, —$O\underline{CH_2}CH_3$), 8.18 (1H, doublet, $C_5$—H), 9.32 (1H, doublet, $C_8$—H).

b) To 3.56 grams (8.948 mmol) of ethyl 7-chloro-6-fluoro-4-hydroxy-2-(2-oxocyclohexylthio)-quinoline-3-carboxylate was added 17 grams of sulfuric acid and the mixture was heated with stirring at 70 to 80°C for twenty minutes. After cooled, it was added to 150 grams of ice, crystals separated out thereby were collected by filtration, washed with water and then with ethanol and ether, and dried to give 3.26 grams (96 percent yield) of the title product, gray crystals, melting point 294 to 297°C (with decomposition). Elementary analysis calculated as $C_{18}H_{15}ClFNO_3S$: C 56.91, H 3.98, N 3.68; Found: C 57.20, H 4.12, N 3.59.

Infrared absorption spectra (KBr, $cm^{-1}$): 1670, 1630, 1610, 1506.

Nuclear magnetic resonance spectra δ ($CF_3CO_2D$): 1.65 (3H, triplet, —$OCH_2\underline{CH_3}$), 1.90 to 2.56 (4H, multiplet, $C_{9,10}$—$CH_2$), 2.80 to 3.29 (2H, multiplet, $C_8$—$CH_2$), 3.30 to 3,82 (2H, multiplet, $C_{11}$—$CH_2$), 4.82 (2H, quartet, —$O\underline{CH_2}CH_3$), 8.41 (1H, doublet, $C_4$—H), 8.86 (1H, doublet, $C_1$—H).

Example 80

2-Chloro-3-fluoro-5-oxo-8,9,10,11-tetrahydro-5H-benzothiazolo(3,2-a)-quinoline-6-carboxylic acid

To three grams (7.89 mmol) of ethyl 2-chloro-3-fluoro-5-oxo-8,9,10,11-tetrahydro-5H-benzothiazolo(3,2-a)-quinoline-6-carboxylate was added a solution of 1.6 grams (40.10 mmol) of sodium hydroxide in 300 ml of water and the mixture was heated to reflux for twenty hours. After cooled, the solution was acidified with acetic acid, stirred for one hour, crystals separated out thereby were collected by filtration, the crystals were washed with water and then with ethanol, and dried to give 2.65 grams (95 percent yield) of pale brown crystals — the title product. Melting point was 311 to 313°C (with decomposition). Elementary analysis calculated as $C_{16}H_{11}ClFNO_3S$: C 54.62, H 3.15, N 3.98; Found: C 54.75, H 3.42, N 3,89.

Infrared absorption spectra (KBr, $cm^{-1}$): 1695, 1590, 1510, 1455.

Nuclear magnetic resonance spectra δ ($CF_3CO_2D$): 1.81 (4H, multiplet, $C_{9,10}$—$CH_2$—), 2.76 to 3.29 (2H, multiplet, $C_8$—$CH_2$—), 3.30 to 3,82 (2H, multiplet, $C_{11}$—$CH_2$—, 8.42 (1H, doublet, $C_4$—HO, 8,86 (1H, doublet, $C_1$—H).

Example 81

3-Fluoro-2-(4-methyl-1-piperazinyl)-5-oxo-8,9,10,11-tetrahydro-5H-benzothiazolo(3,2-a)-quinoline-6-carboxylic acid

To one gram (2.84 mmol) of 2-chloro-3-fluoro-5-oxo-8,9,10,11-tetrahydro-5H-benzothiazolo(3,2-a) quinoline 6-carboxylic acid were added 2.8 grams (28.4 mmol) of N-methyl piperazine and 50 ml of pyridine and the mixture was heated to reflux on an oil bath for fifty four hours. Pyridine was evaporated therefrom under reduced pressure, the residue was washed with ether and then with water, and dried. The resulting crystals were recrystallized from pyridine twice to give 146 mg (12 percent yield) of the title compound in pale yellow crystals melting at 283 to 285°C (with decomposition). Elementary analysis calculated as $C_{21}H_{22}FN_2O_3S$: C 60.70, H 5.33, N 10.11; Found: C 60.82, H 5.54, N 10.28.

Infrared absorption spectra (KBr, $cm^{-1}$): 1692, 1630, 1580, 1482.

Nuclear magnetic resonance spectra δ ($CF_3CO_2D$): 1.62 to 2.48 (4H, multiplet, $C_{9,10}$—$CH_2$),

2.65 to 4.42 (12H, multiplet, $C_{8,11}$—$CH_2$—, 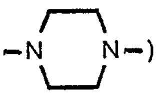),

3.19 (3H, singlet, $CH_3N$), 8.14 (1H, doublet, $C_1$—H), 8.31 (1H, doublet, $C_4$—H).

Example 82

Ethyl 2-chloro-3-fluoro-7a-methyl-5-oxo-7a,8,9,10-tetrahydro-5H-benzothiazolo(3,2-a)-quinoline-6-carboxylate

a) To three grams (9,94 mmol) of ethyl 7-chloro-6-fluoro-4-hydroxy-2-mercaptoquinoline 3-carboxylate was added a solution of 0.23 gram of sodium (9,94 mmol) in 50 ml of ethanol and the mixture was stirred for about ten minutes. Then 1.7 grams of 2-chloro-2-methyl cyclohexanone (11.43 mmol) was added thereto and the mixture was heated to reflux on an oil bath for twenty four hours. Ethanol was evaporated therefrom under reduced pressure, water was added to the residue, crystals separated out thereby were collected by filtration, washed with water, and the crystals obtained upon drying were recrystallized from ethanol to give 1.97 grams (48 percent yield) of ethyl 7-chloro-6-fluoro-4-hydroxy-2-(1-methyl-2-oxocyclohexylthio) quinolinej 3-carboxylate, yellow crystals, melting at 214 to 217°C (with decomposition). Elementary analysis calculated as $C_{19}H_{19}ClFNO_4S$: C 55.40, H 4.64, N 3.40; Found: C 55.52, H 4,81, N 3.46.

Infrared absorption spectra (KBr, $cm^{-1}$): 1730, 1680, 1595, 1470.

Nuclear magnetic resonance spectra δ ($CDCl_3$): 1.41 (4H, triplet, —$OCH_2\underline{CH_3}$), 1.53 (3H, singlet, —$CH_3$),

1.20 to 2.80 (8H, multiplet, 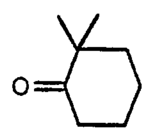),

4.40 (2H, quartet, —$O\underline{CH_2}CH_3$), 7.35 (1H, doublet, $C_5$—H), 8.71 (1H, doublet, $C_8$—H).

b) To 1.14 grams (2.77 mmol) of ethyl 7-chloro-6-fluoro-4-hydroxy-2-(1-methyl-2-oxocyclohexylthio)-quinoline-3-carboxylate was added five grams of sulfuric acid and the mixture was heated at 70°C for five minutes. After cooled, it was added to ten grams of ice, crystals separated out thereby were collected by filtration, washed with water, dried, and the resulting yellow crystals were recrystallized from ethanol to give 0.64 gram (59 percent yield) of the title compound in colorless crystals melting at 196 to 200°C (with decomposition). Elementary analysis calculated as $C_{19}H_{17}ClFNO_3S$: C 57.94, H 4.35, N 3.55; Found: C 58.10, H 4.38, N 3.72.

Infrared absorption spectra (KBr, $cm^{-1}$): 1750, 1645, 1615, 1470.

Nuclear magnetic resonance spectra δ ($CDCl_3$): 1.45 (3H, triplet, —$OCH_2\underline{CH_3}$), 1.64 (3H, singlet, $C_{7a}$—$\underline{CH_3}$),

1.80 to 2.90 (6H, multiplet, 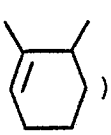),

4.42 (2H, quartet, —$O\underline{CH_2}CH_3$, 6.07 (1H, triplet, $C_{11}$—H), 7.80 (1H, doublet, $C_1$—H), 8.07 (1H, doublet, $C_4$—H).

Example 83

2-Chloro-3-fluoro-7a-methyl-5-oxo-7a,8,9,10-tetrahydro-5H-benzothiazolo(3,2-a)-quinoline-6-carboxylic acid

To 470 mg (1.19 mmol) of ethyl 2-chloro-3-fluoro-7a-methyl-5-oxo-7a,8,9,10-tetrahydro-5H-benzothiazolo(3,2-a) quinoline 6-carboxylate were added 15 ml of ethanol and a solution of 330 mg (5,97 mmol) of potassium hydroxide in 1 ml of water and the mixture was heated to reflux for one hour. Ethanol was evaporated therefrom under reduced pressure, 5 ml of water was added to the residue, the mixture was acidified with acetic acid with cooling, crystals separated out therefrom were collected by filtration, washed

with water, and dried to give 340 mg (90 percent yield) of the title compound in colorless crystals melting at 251 to 254°C (with decomposition). Elementary analysis calculated as $C_{17}H_{13}ClFNO_3S$: C 55.81, H 3.58, N 3.82; Found: C 55,95, H 3.60, N 3,95.

Infrared absorption spectra (KBr, $cm^{-1}$): 1706, 1590, 1490, 1452.

Nuclear magnetic resonance spectra δ ($CF_3CO_2D$): 1.76 (3H, singlet, $C_{7a}$—$CH_3$), 1.90 to 2,80 (tH, multiplet, $C_{8,9,10}$—H), 6.50 to 6.65 (1H. multiplet, $C_{11}$—H), 8.12 (1H, singlet, $C_1$—H), 8.34 (1H, singlet, $C_4$—H).

Example 84

3-Fluoro-7a-methyl-2-(4-methyl-1-piperazinyl)-5-oxo-7a,8,9,10-tetrahydro-5H-benzothiazolo(3,2-a)-quinoline-6-carboxylic acid

To 390 mg (1.07 mmol) of 2-chloro-3-fluoro-7a-methyl-5-oxo-7a,8,9,10-tetrahydro-5H-benzothiazolo(3,2-a)-quinoline-6-carboxylic acid were added 1.10 grams (10.70 mmol) of N-methyl piperazine and 60 ml of pyridine and the mixture was heated to reflux in an oil bath for sixty eight hours. Pyridine was evaporated therefrom under reduced pressure and to the residue was added ether, the resulting crystals were collected by filtration, washed with water and then with ether, dried, and the resulting gray crystals were recrystallized from dimethyl formamide to give 140 mg (30 percent yield) of the title compound melting at 249 to 251°C (with decomposition). Elementary analysis calcuated as $C_{22}H_{24}FN_3O_3S$: C61.52, H 5.63, N 9.78; Found: C 61.64, H 5.84, N 9.62.

Infrared absorption spectra (KBr, $cm^{-1}$): 1715, 1629, 1585, 1490.

Nuclear magnetic resonance spectra δ ($CF_3CO_2D$): 1.82 (3H, singlet, $C_{7a}$—$C\underline{H}_3$), 1.80 to 2.85 (6H, multiplet, $C_{8,9,10}$—H), 3.21 (3H, singlet, $N\underline{CH}_3$),

3.00 to 4.50 (8H, multiplet, 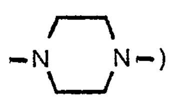),

6.50 to 6.65 (1H, multiplet, $C_{11}$—H), 7.60 (1H, doublet, $C_1$—H), 8.15 (1H, doublet, $C_4$—H).

Example 85

Ethyl 7-chloro-6-fluoro-4-oxo-4H-(1,3)thiazeto(3,2-a)-quinoline-3-carboxylate

A mixture of 2.0 grams (6.63 mmol) of ethyl 7-chloro-6-fluoro-4-hydroxy-2-mercaptoquinoline-3-carboxylate and 1,96 grams (7.3 mmol) of diiodomethane was stirred at room temperature for one hour in 30 ml of dimethyl formamide in the presence of 2.02 grams (14.6 mmol) of potassium carbonate. Dimethyl formamide was evaporated therefrom under reduced pressure, water and ether were added to the residue, crystals separated out therefrom were collected by filtration, washed with water, dried and crystallized from dimethyl formamide to give 910 mg (43.8 percent yield) of ethyl 7-chloro-6-fluoro-4-oxo-4H-(1,3-thiazeto(3,2-a)-quinoline-3-carboxylate melting at 302 to 304°C (with decomposition). Elementary analysis calculated as $C_{13}H_9ClFNO_3S$: C49.77, H 2.89, N 4.46; Found: C 49.61, H 2.92, N 4.63

Infrared absorption spectra (KBr, $cm^{-1}$): 1710, 1595, 1370, 795

Nuclear magnetic resonance spectra δ ($CF_3CO_2D$): 1.47 (3H, triplet, $OCH_2C\underline{H}_3$), 4.53 (2H, quartet, $O\underline{C}H_3$), 6.14 (2H, singlet, —N—$\underline{CH}_2$S—), 7.92 (1H, doublet, $C_5$—H), 8.14 (1H, doublet, $C_8$—H).

Example 86

7-Chloro-6-fluoro-4-oxo-4H-(1,3)thiazeto(3,2-a)-quinoline 3-carboxylic acid

Ethyl 7-chloro-6-fluoro-4-oxo-4H-(1,3)thiazeto(3,2-a)-quinoline-3-carboxylate (365 mg, 1.16 mmol) was dissolved in 3.5 ml of concentrated sulfuric acid and the solution was heated at 80°C for seven hours. This was poured into ice flakes, crystals separated out thereby were collected by filtration, washed with water, dried and recrystallized from dimethyl formamide to give 258 mg of the title compound. The yield was 77.6 percent and melting point was 284 to 287°C (with decomposition). Elementary analysis calculated as $C_{11}H_5ClFNO_3S$: C46.25, H 1.76, N 4.90; Found: C 46.14, H 1.73, N 4.73.

Infrared absorption spectra (KBr, $cm^{-1}$): 1700, 1600, 805.

Nuclear magnetic resonance spectra δ ($CF_3CO_2D$): 6.15 (2H, singlet, —$N\underline{CH}_2$S—), 7.85 (1H, doublet, $C_5$—H), 8.24 (1H, doublet, $C_8$—H).

Example 87

Ethyl 1-methyl-7,8-methylenedioxy-5-oxo-1,2-dihydro-5H-thiazolo(3,2-a)-quinoline-4-carboxylate

A mixture of one gram of ethyl 4-hydroxy-2-mercapto-6,7-methylenedioxyquinoline 3-carboxylate and 1.38 grams of potassium carbonate was added to 50 ml of dimethyl formamide and, with stirring at room temperature, 1.03 grams of 1,2-dibromopropane was dropped therein. The mixture was then stirred at 60°C for seven hours. When the reaction was completed, the solvent was evaporated therefrom under reduced pressure, crystals thereby separated out were well washed with water and recrystallized from 30 ml of ethanol to give 930 mg of the title compound in colorless crystals melting at 197 to 205°C. Elementary analysis calculated as $C_{16}H_{15}O_5NS$: C 57.65, H 4.54, N 4.21, S 9.62; Found: C 57.85, H 4.69, N 4.31, S 9.82.

Infrared absorption spectra (KBr, $cm^{-1}$): 1710, 1670, 1630.

Nuclear magnetic resonance spectra δ ($CDCl_3$): 1.45 (3H, doublet, $C_1$—$CH_3$), 1.41 (3H, triplet, —$OCH_2$—$\underline{C}H_3$), 2.89 (1H, doublet, $C_2$—H), 3.60 (1H, double doublet, $C_2$—H), 4.36 (2H, quartet, —$O\underline{CH}_2CH_3$),

4.8 to 5.2 (1H, wide singlet, $C_1$—H), 6.0 (2H, singlet, —$OCH_2O$—), 6.62 (1H, singlet, $C_9$—H), 7.65 (1H, singlet, $C_6$—H).

Example 88

1-Methyl-7,8-methylenedioxy-5-oxo-1,2-dihydro-5H-thiazolo(3,2-a)-quinoline-4-carboxylic acid

Ethyl 1-methyl-7,8-methylenedioxy-5-oxo-1,2-dihydro-5H-thiazolo(3,2-a)-quinoline-4-carboxylate (930 mg) was dissolved in a solution of one gram of sodium hydroxide in a mixture of 20 ml of water and 20 ml of ethanol. The solution was stirred at 60°C for thirty minutes. After the reaction was completed, ethanol was evaporated therefrom under reduced pressure and neutralized with acetic acid with ice cooling to give crystals. The crystals separated out therefrom were collected by filtration, well washed with water, and recrystallized from 100 ml of dimethyl formamide to give 610 mg of the title compound melting at higher than 302°C. Elementary analysis calcualted as $C_{14}H_{11}NO_5S$: C 55.08, H 3.63, N 4.59; Found: C 55.19, H 3.57, N 4.48.

Infrared absorption spectra (KBr, $cm^{-1}$): 3450, 1690, 1630.

Nuclear magnetic resonance spectra δ ($CF_3CO_2D$): 1.75 (3H, doublet, —$CH_3$), 3.40 (2H, doublet, $C_2$—H), 4.0 (1H, triplet, $C_1$—H), 6.30 (2H, singlet —$OCH_2O$—), 7.25 (1H, singlet, $C_9$—H), 7.75 —1H, singlet, $C_6$—H).

Example 89

Ethyl 7.8-methylenedioxy-5-oxo-1-tetrahydropyranyloxymethyl-1,2-dihydro-5H-thiazolo(3,2-a)-quinoline-4-carboxylate

A mixture of one gram of ethyl 4-hydroxy-2-mercapto-6,7-methylenedioxyquinoline 3-carboxylate and 1.4 grams of potassium carbonate was added to 50 ml of dimethyl formamide. 1,2-Dibromo-3-tetrahydropyranyloxypropane (1.23 grams) was dropped into the solution with stirring at room temperature. After the dropping was completed, the mixture was heated at 80°C for three hours with stirring, allowed to stand at room temperature overnight, then the solvent was evaporated therefrom under reduced pressure, the residue was extracted with 100 ml of chloroform, and the extract was washed with water for three times. Chloroform was evaporated therefrom under reduced pressure whereupon the residue was recrystallized. The crystals were collected by filtration and recrystallized from fifty ml of ethanol to give one gram of the title compound melting at 152 to 7°C. Elementary analysis calculated as $C_{21}H_{23}NO_7S$: C 58.19, H 5.35, N 3.23; Found: C 58.35, H 5.51, N 3.18.

Infrared absorption spectra (KBr, $cm^{-1}$): 1660, 1610, 1500, 1480, 1200, 1138.

Nuclear magnetic resonance spectra δ ($CDCl_3$): 1.45 (3H, triplet, —$OCH_2\underline{CH_3}$), 1.65 (6H, wide singlet, $C_3'$, $C_4'$, $C_5'$—H of tetrahydropyranyl group), 3.30 to 4.0 (6H, multiplet, $C_6'$—H of tetrahydropyranyl group, $C_1$—$CH_2O$—, $C_2$—H), 4.40 (2H, quartet, —$O\underline{CH_3}$), 4.90 to 5.40 (2H, wide singlet, $C_1$—H, $C_2'$—H of tetrahydropyranyl group), 6.01 (2H, singlet, —$OCH_2O$—), 6.79 (1H, singlet, $C_9$—H), 7.67 (1H, singlet, $C_6$—H).

Example 90

1-Hydroxymethyl-7,8-methylenedioxy-5-oxo-1,2-dihydro-5H-thiazolo(3,2-a)-quinoline-4-carboxylic acid

One gram of ethyl 7,8-methylenedioxy-5-oxo-1-tetrahydropyranyloxymethyl-1,2-dihydro-5H-thiazolo(3,2-a)-quinoline-4-carboxylate was dissolved in a solution of 184 mg of sodium hydroxide in a one-to-one ratio mixture (50 ml) of water and ethanol and the whole mixture was stirred at 80°C for two hours. When the reaction was completed, ethanol was evaporated therefrom under reduced pressure, then neutralized with acetic acid with ice cooling, crystals separated out thereby were collected by filtration, and dried with air. The resulting crystals were dissolved in ten ml of trifluoroacetic acid and the solution was stirred at room temperature for one hour. After the reaction was completed, the reaction solution was poured over 50 ml of ice water, crystals separated out were collected by filtration, well washed with water, and then washed with ethanol. This was finally dried under reduced pressure to give 620 mg of the title compound which melted at higher than 320°C. Elementary analysis calculated as $C_{14}H_{11}NO_6S.H_2O$: C 49.38, H 3.29, N 4.15; Found: C 50.36, H 3.38, N 3.87.

Infrared absorption spectra (KBr, $cm^{-1}$): 3300, 1680, 1630, 1270.

Nuclear magnetic resonance spectra δ ($CF_3CO_2D$): 4.20 (4H, doublet, —$\underline{CH_2}OH$, $C_2$—H), 5.7 to 6.2 (1H, broad, $C_1$—H), 6.30 (2H, singlet, —$OCH_2O$—), 7.41 (1H, singlet $C_9$—H), 7.75 (1H, singlet, $C_6$—H).

Example 91

Ethyl 1-methyl-7,8-methylenedioxy-5-oxo-5H-thiazolo(3,2-a)-quinoline-4-carboxylate

Five hundred milligrams of ethyl 4-hydroxy-2-mercapto-6,7-methylenedioxyquinoline-3-carboxylate was dissolved in thirty milliliters of ethanol, then 180 mg of chloroacetone was added thereto, and the mixture was heated to reflux with stirring for four hours. After the reaction was completed, ethanol was evaporated therefrom under reduced pressure, and crystals separated out therefrom were collected by filtration to give ethyl 2-acetylmethylmercapto-4-hydroxy-6,7-methylenedioxyquinoline 3-carboxylate which was an intermediate compound. The resulting crystals were dissolved in five milliliters of concentrated sulfuric acid, the solution was stirred at room temperature for thirty minutes, then poured over into twenty milliliters of ice water, and crystals separated out therefrom were collected by filtration. The crystals were well washed with water and then washed with ethyl ether. Then the crystals were dried under reduced pressure to give 470 mg of the title compound melting at 308 to 311°C. Elementary analysis

calculated as $C_{16}H_{13}NO_5S$: C 58.00, H 3.95, N 4.23; Found: C 58.21, H 3.90, N 4.11.

Infrared absorption spectra (KBr, $cm^{-1}$): 1645, 1610, 1590, 1310, 1040.

Nuclear magnetic resonance spectra δ ($CF_3CO_2D$): 1.70 (3H, triplet, $—OCH_2\underline{CH_3}$), 3.22 (3H, singlet, $—CH_3$), 4.84 (2H, quartet, $—O\underline{CH_2}CH_3$), 6.33 (2H, singlet, $—OCH_2O—$), 7.60 (1H, singlet, $C_9—H$), 7.97 (1H, singlet, $C_6—H$), 8.25 (1H, singlet, $C_2—H$).

## Example 92

1-Methyl-7,8-methylenedioxy-5-oxo-5H-thiazolo(3,2-a)-quinoline-4-carboxylic acid

Ethyl 1-methyl-7,8-methylenedioxy-5-oxo-5H-thiazolo(3,2-a)quinoline-4-carboxylate (470 mg) was dissolved in a solution of 550 mg of potassium carbonate in a mixture of 20 ml of water and 80 ml of ethanol and the whole mixture was heated to reflux for two hours with stirring. After the reaction was completed, ethanol was evaporated therefrom under reduced pressure, the residue was poured over into twenty milliliters of ice water, and neutralized with acetic acid. Crystals separted out therefrom were collected by filtration, washed with water, and then with ethanol and ether. Then it was recrystallized from fifty milliliters of dimethyl formamide to give 360 mg of the title compound melting at 303 to 305°C. Elementary analysis calculated as $C_{14}H_9NO_5S$: C 55.44, H 2.99, N 4.62; Found: C 55.42, H 2.66, N 4.51.

Infrared absorption spectra (KBr, $cm^{-1}$): 3400 to 3500, 1680, 1500, 1260, 1040.

Nuclear magnetic resonance spectra δ ($CF_3CO_2D$): 3.23 (3H, singlet, $—CH_3$), 6.35 (2H, singlet, $—OCH_2O—$), 7.60 (1H, singlet, $C_9—H$), 7.96 (1H, singlet, $C_6—H$), 8.23 (1H, singlet, $C_2—H$).

## Example 93

Ethyl 1-chloromethylene-7,8-methylenedioxy-5-oxo-5H-thiazolo(3,2-a)-quinoline-4-carboxylate

One gram of ethyl 4-hydroxy-2-mercapto-6,7-methylenedioxyquinoline 3-carboxylate was dissolved in sodium ethoxide prepared from 86 mg of sodium and 50 ml of ethanol and the mixture was stirred for thirty minutes at room temperature. Then a mixture of 431 mg of dichloroacetone and 30 ml of ethanol was dropped therein and the whole mixture was stirred at room temperature for four hours. After the reaction was completed, crystals separated out therefrom were collected by filtration to give 1.1 grams of ethyl 2-(1-chloroacetylmethylmercapto)-4-hydroxy-6,7-methylenedioxyquinoline-3-carboxylate melting at 184°C. The resulting crystals were dissolved in ten milliliters of concentrated sulfuric acid with ice cooling and the mixture was stirred for one hour. After the reaction was completed, the reaction solution was poured over into fifty milliliters of ice water, the mixture was neutralized with an aqueous solution (2N) of sodium hydroxide, and crystals separated out therefrom were collected by filtration. The crystals were well washed with water and recrystallized from one hundred milliliters of dimethyl formamide to give 600 mg of the title compound which melted at higher than 320°C. Elementary analysis calculated as $C_{16}H_{12}NO_5SCl$: C 52.54, H 3.31, N 3.83; Found: C 52.81, H 3.43, N 3.99.

Infrared absorption spectra (KBr, $cm^{-1}$): 1650, 1630, 1600, 1580, 1470, 1050.

Nuclear magnetic resonance spectra δ ($CF_3CO_2D$): 1.70 (3H, triplet, $—OCH_2\underline{CH_3}$), 4.85 (2H, quartet, $—O\underline{CH_2}CH_3$), 5.3 (2H, singlet, $—\underline{CH_2}Cl$), 6.35 (2H, singlet, $—OCH_2O—$), 7.98 (1H, singlet, $C_9—H$), 8.02 (1H, singlet, $C_6—H$), 8.35 (1H, singlet, $C_2—H$).

## Example 94

1-Methoxymethyl-7,8-methylenedioxy-5-oxo-5H-thiazolo(3,2-a)-quinoline-4-carboxylic acid

Metal sodium (78 mg) was dissolved in thirty milliliters of methanol, 600 mg of ethyl 1-chloromethyl-7,8-methylenedioxy-5-oxo-5H-thiazolo(3,2-a)-quinoline 4-carboxylate was added thereto at a time with stirring at room temperature and the whole mixture was heated to reflux with stirring for three hours. After the reaction was completed, methanol was evaporated therefrom under reduced pressure, crystals separated out thereby were collected by filtration, and washed well with water. The resulting crystals were dissolved in ethyl acetate, purified by passing through a column (150 g of silica gel), and ethyl acetate in the resulting solution was evaporated therefrom to give 300 mg of methyl 1-methoxymethyl-7,8-methylenedioxy-5-oxo-5H-thiazolo(3,2-a) quinoline 4-carboxylate.

The resulting crystals were dissolved in a solution of one hundred milligrams of sodium hydroxide in a mixture of 25 ml of water and 25 ml of ethanol and the mixture was heated to reflux for three hours with stirring. After the reaction was completed, it was cooled with ice, neutralized with acetic acid, crystals separated out therefrom were collected by filtration, and well washed with water. The resulting crystals were dried by heating under reduced pressure to give 250 mg of the title compound melting at 265°C.

Infrared absorption spectra (KBr, $cm^{-1}$): 3.79 (3H, singlet, $—OCH_3$), 5.18 (2H, singlet, $—\underline{CH_2}OCH_3$), 6.35 (2H, singlet, $—OCH_2O—$), 7.96 (1H, singlet, $C_9—H$), 8.06 (1H, singlet, $C_6—H$), 8.32 (1H, singlet, $C_2—H$).

## Example 95

Ethyl 6,7-methylenedioxy-4-oxo-4H-(1,3)thiazeto(3,2-a)-quinoline-3-carboxylate

A mixture of 500 mg of ethyl 4-hydroxy-2-mercapto-6,7-methylenedioxyquinoline-3-carboxylate and 510 mg of potassium carbonate was added to 300 ml of dimethyl formamide and the whole mixture was stirred at room temperature. Then 660 mg of diiodomethane was dropped thereinto and the mixture was stirred at room temperature for two hours. After the reaction was completed, dimethyl formamide was evaporated therefrom under reduced pressure and the residue was poured over into 50 ml of ice water.

Crystals separated out thereby were collected by filtration and washed with water and with ethanol and finally dried with air to give 450 mg of the title compound. Elementary analysis calculated as $C_{14}H_{11}NO_5S$: C 55.08, H 3.63, N 4.59; Found: C 55.21, H 3.81, N 4.41.

Infrared absorption spectra (KBr, $cm^{-1}$): 1710, 1625, 1370, 1020.

Nuclear magnetic resonance spectra δ ($CF_3CO_2D$): 1.59 (3H, triplet, $—OCH_2\underline{CH_3}$), 4.68 (2H, quartet, $—O\underline{CH_2}CH_3$). 6.08 (2H, singlet, $C_2$—H), 6.30 (2H, singlet, $—OCO_2O—$), 7.02 (1H, singlet, $C_7$—H), 7.72 (1H, singlet, $C_4$—H).

## Example 96

Ethyl 8,9-methylenedioxy-6-oxo-1,2,3,6-tetrahydro(1,3)thiazino(3,2-a)-quinoline-4-carboxylate

A mixture of 500 mg of ethyl 4-hydroxy-2-mercapto-6,7-methylenedioxyquinoline-3-carboxylate and 510 mg of potassium carbonate was added to 30 ml of dimethyl formamide and the whole mixture was stirred at room temperature. Then 520 mg of 1,3-dibromopropane was dropped thereinto and the mixture was stirred at room temperature for thirty minutes. After the reaction was completed, dimethyl formamide was evaporated therefrom under reduced pressure and the residue was poured over into 20 ml of ice water. Crystals separated out therefrom were collected by filtration, washed with water, methanol and ether, and dried with air to give 330 mg of the title compound. Elementary analysis calculated as $C_{16}H_{15}NO_5S$: C 57.65, H 4.54, N 4.20; Found: C 57.45, H 4.61, N 4.11.

Infrared absorption spectra (KBr, $cm^{-1}$): 1720, 1620, 1580, 1480.

Nuclear magnetic resonance spectra δ ($CF_3CO_2D$): 1.56 (3H, triplet $—OCH_2\underline{CH_3}$), 2.30 to 3.00 (2H, multiplet, $C_2$—H), 3.30 (2H, triplet, $C_3$—H), 4.30 to 5.00 (4H, multiplet, $—O\underline{CH_2}CH_3$ and $C_1$—H), 6.25 (2H, singlet, $—OCH_2O—$), 7.41 (1H, singlet, $C_{10}$—H), 7.73 (1H, singlet, $C_7$—H).

## Example 97

8,9-Methylenedioxy-6-oxo-1,2,3,6-tetrahydro-(1,3)thiazino(3,2-a)-quinoline-4-carboxylic acid

Ethyl 8,9-methylenedioxy-6-oxo-1,2,3,6-tetrahydro-(1,3)thiazino (3,2-a)-quinoline-4-carboxylate (510 mg) was dissolved in a solution of one gram of potassium hydroxide in a mixture of 25 ml of water and 25 ml of ethanol. The whole mixture was heated to reflux for two hours. After the reaction was completed, ethanol was evaporated therefrom under reduced pressure, the residue was poured over into thirty milliliters of ice water, neutralized with acetic acid, crystals separated out thereby were collected by filtration, washed with water, ethanol and ether, and recrystallized from 30 ml of dimethyl formamide to give 310 mg of the title compound which melted at 274 to 284°C. Elementary analysis calculated as $C_{14}H_{11}NO_5S$: C 55.01, H 3.63, N 4.59; Found: C 55.31, H 3.82, N 4.60.

Infrared absorption spectra (KBr, $cm^{-1}$): 3400 to 3500, 1685, 1500, 1260, 1060.

Nuclear magnetic resonance spectra δ ($CF_3CO_2D$): 2.50 to 3.0 (2H, multiplet, $C_2$—H), 3.35 (2H, triplet, J = 6.0 Hz, $C_3$—H), 4.68 (2H, triplet, $C_1$—H), 6.29 (2H, singlet, $—OCO_2O—$), 7.42 (1H, singlet, $C_{10}$—H), 7.76 (1H, singlet t, $C_7$—H).

## Example 98

7-Fluoro-8-(4-formyl-1-piperazinyl-5-oxo-5H-thiazolo(3,2-a)-quinoline-4-carboxylic acid

7-Fluoro-5-oxo-8-(1-piperazinyl)-5H-thiazolo(3,2-a)-quinoline-4-carboxylic acid (350 mg) was dissolved in 15 ml of 98% concentrated formic acid, then 5 ml of acetic anhydride was dropped thereinto gradually with ice cooling, the reaction mixture was then stirred at room temperature for four hours, poured over into ice water, and crystals separated out therefrom were collected by filtration. The crystals were then washed with water and with ethanol and dried to give 310 mg of the title compound as colorless powder which melted at 335 to 336°C with decomposition. Elementary analysis calculated as $C_{17}H_{14}FN_3O_4 \cdot \frac{1}{4} H_2O$: C 53.75, H 3.85, N 11.06; Found: C 53.50, H 3.78, N 10.88.

Infrared absorption spectra (KBr, $cm^{-1}$): 3400, 1668, 1625, 1592, 1492, 1440, 1395, 1260, 1228, 1135, 1005, 802, 793.

Nuclear magnetic resonance spectra δ ($CF_3CO_2D$): 3.5 to 4.2 (8H, multiplet, methylene group in piperazine), 7.73 (1H, doublet, $C_9$—H), 7.94 (1H, doublet, $C_2$—H), 8.24 (1H, doublet, $C_6$—H), 8.35 (1H, singlet, $—\underline{CH}O$), 8.87 (1H, doublet, $C_1$—H).

## Example 99

8-(4-Acetyl-1-piperazinyl)-7-fluoro-5-oxo-5H-thiazolo(3,2-a)-quinoline-4-carboxylic acid

7-Fluoro-5-oxo-8-(1-piperazinyl)-5H-thiozolo(3,2-a)-quinoline-4-carboxylic acid (400 mg) was dissolved in 60 ml of pyridine and the solution was heated to reflux at 70°C for five hours with stirring after addition of 500 mg of acetic anhydride. After cooled, this was poured over into ice water, and crystals separated out therefrom were collected by filtration. The crystals were washed with water and ethanol to give 310 mg of the title compound as colorless powder which melted at 326 to 8°C with decomposition. Elementary analysis calculated as $C_{18}H_{17}FN_3O_4$: C 55.38, H 4.39, N 10.76; Found: C 55.13, H 4.05, N 10.49.

Infrared absorption spectra (KBr, $cm^{-1}$): 3400, 1680, 1655, 1630, 1590, 1247, 1223, 1118, 1041, 1000, 890, 802, 795, 730.

Nuclear magnetic resonance spectra δ ($CF_3CO_2D$): 2/68 (3H, singlet, $=N—CO—\underline{CH_3}$), 3.6 toi 4.4 (8H,

multiplet, methylene group in piperazine), 7.70 (1H, doublet, $C_9$—H), 7.95 (1H, doublet, $C_2$—H), 8.25 (1H, doublet, $C_6$—H), 8.88 (1H, doublet, $C_1$—H).

Example 100

8-{4-(3-carboxypropionyl)-1-piperazinyl}-7-fluoro-5-oxo-5H-thiazolo(3,2-a)-quinoline-4-carboxylic acid

A mixture of 350 mg of 7-fluoro-5-oxo-(1-piperazinyl)-5H-thiazolo(3,2-a)-quinoline-4-carboxylic acid, 150 mg of succinic anhydride, 30 ml of dimethyl formamide, and 5 ml of triethylamine was heated with stirring at 70°C for three hours. After cooled, 300 ml of water was added to the reaction solution, the mixture was weakly acidified with acetic acid, and the resulting flowing gel-like substance was collected by filtration. This was washed with water and dried to give 350 mg of the title compound as pale brown powder which melted at 331 to 334°C with decomposition. Elementary analysis calculated as $C_{20}H_{18}FN_3O_6S.\frac{1}{2}H_2O$: C 53.69, H 4.20, N 9.21; Found: C 52.41, H 4.01, N 9.00.

Infrared absorption spectra (KBr, $cm^{-1}$): 3420, 1690, 1628, 1490, 1392, 1260, 1227, 1140, 1012, 802, 795.

Nuclear magnetic resonance spectra δ ($CF_3CO_2D$): 3.05 (4H, singlet, —CO—$C\underline{H}_2C\underline{H}_2$—$CO_2H$), 3.50 to 4.35 (8H, multiplet, methylene group in piperazine), 7.73 (1H, doublet, $C_9$—H), 7.97 (1H, doublet, $C_2$—H), 8.27 (1H, doublet, $C_6$—H), 8.90 (1H, doublet, $C_1$—H).

Example 101

8-{4-(4-Carboxybutyroyl)-1-piperazinyl}-7-fluoro-5-oxo-5H-thiazolo(3,2-a)-quinoline-4-carboxylic acid

A mixture of 210 mg of 7-fluoro-5-oxo-8-(1-piperazinyl)-5H-thiazolo(3,2-a)-quinoline-4-carboxylic acid, 82 mg of glutaric anhydride, 18 ml of dimethyl formamide and 3 ml of triethylamine was heated with stirring at 60°C for two hours. After cooled, 60 ml of water was added thereto, then made weakly acidic with acetic acid, chloroform was added thereto, the mixture was stirred for ten minutes, and crystals separated out thereby were collected by filtration. The crystals were washed with water, ethanol and ether and dried to give 245 mg of the title compound as colorless powder which melted at 276 to 278°C. Elementary analysis calculated as $C_{21}H_{20}FN_3O_6S.\frac{1}{2}H_2O$: C 53.61, H 4.49, N 8.93; Found: C 53.89, H 4.42, N 8.85.

Infrared absorption spectra (KBr, $cm^{-1}$): 3430, 1700, 1627, 1590, 1480, 1390, 1260, 1220, 1140, 1030, 802, 795.

Nuclear magnetic resonance spectra δ ($CF_3CO_2D$): 1.90 (2H, multiplet, —$COCH_2C\underline{H}_2CH_2CO_2H$), 2.50 to 3.20 (4H, multiplet, —$COC\underline{H}_2CH_2C\underline{H}_2CO_2H$), 3.50 to 4.35 (8H, multiplet, methylene group in piperazine), 7.57 (1H, doublet, $C_9$—H), 8.00 (1H, doublet, $C_2$—H), 8.28 (1H, doublet, $C_6$—HO, 8.92 (1H, doublet, $C_1$—H).

Example 102

7-Fluoro-5-oxo-8-(4-palmitoyl-1-piperazinyl)-5H-thiazolo(3,2-a)-quinoline-4-carboxylic acid

A mixture of 210 mg of 7-fluoro-5-oxo-8-(1-piperazinyl)-5H-thiazolo(3,2-a)-quinoline-4-carboxylic acid, 197 mg of palmitoyl chloride, 36 ml of dimethyl formamide and 6 ml of triethylamine was heated with stirring at 70°C for three hours and then the solvent was evaporated therefrom under reduced pressure. Water was added to the residue and the mixture was extracted with chloroform. The chloroform extract was washed with water, dried with magnesium sulfate, and chloroform was evaporated therefrom to give dark yellow sirupy oily residue. This was subjected to separation and purification with silica gel chromatography to give 100 mg of the title compound as colorless waxy substance. Elementary analysis calculated as $C_{32}H_{44}FN_3O_4$: C 65.61, H 7.57, N 7.17; Found: C 65.44, H 7.82, N 6.80.

Infrared absorption spectra (KBr, $cm^{-1}$): 1690, 1650, 1625, 1490, 1260, 1218, 1138, 1025, 897, 802, 792.

Nuclear magnetic resonance spectra δ ($CDCl_3$): 0.86 (3H, singlet, $C\underline{H}_3$—$(CH_2)_{14}CO$—), 1.24 (26H, singlet, $CH_2(C\underline{H}_2)_{13}CH_2CO$—), 2.35 (2H, triplet, $C_{14}H_{29}$—$C\underline{H}_2CO$—), 3.20 to 3.55 (4H, broad singlet, protons at two- and six-positions of piperazine), 3.55 to 4.00 (4H, broad singlet, protons at 3- and 5-positions of piperazine), 7.13 (1H, doublet, $C_9$—H), 7.27 (1H, doublet, $C_2$—H), 7.78 (1H, doublet, $C_6$—H), 8.14 (1H, doublet, $C_1$—H).

Example 103

8-{4-(2-carboxybenzyl)-1-piperazinyl}-7-fluoro-5-5H-thiazolo(3,2-a)-quinoline-4-carboxylic acid

A mixture of 210 mg of 7-fluoro-5-oxo-8-(1-piperazinyl)-5H-thiazolo(3,2-a) quinoline 4-carboxylic acid, 108 mg of phthalic anhydride, 18 ml of dimethyl formamide, and 3 ml of triethylamine was stirred with heating at 60°C for two hours. After cooled, water was added thereto and the mixture was weakly acidified with acetic acid. The resulting floating substances were collected by filtration, washed with water and dried to give 200 mg of the title compound as pale yellow powder which melted at 277 to 278°C. Elementary analysis calculated as $C_{24}H_{22}FN_3O_6S.2H_2O$: C 54.24, H 4.17, N 7.91; Found: C 53.95, H 3.74, N 7.78.

Infrared absorption spectra (KBr, $cm^{-1}$): 3480, 1700, 1628, 1490, 1445, 1400, 1263, 1428, 1142, 1009, 800.

Nuclear magnetic resonance spectra δ ($CF_3CO_2D$): 3.55 to 4.20 (8H, multiplet, methylene group in piperazine), 7.87 (1H, doublet, $C_9$—H), 8.02 (5H, multiplet, proton in benzene ring and $C_2$—H), 8.30 (1H, doublet, $C_6$—H), 8.97 (1H, doublet, $C_1$—H).

**Claims**

1. Quinolinecarboxylic acid derivatives represented by the following general formula (Z) or pharmaceutically acceptable acid addition salts thereof

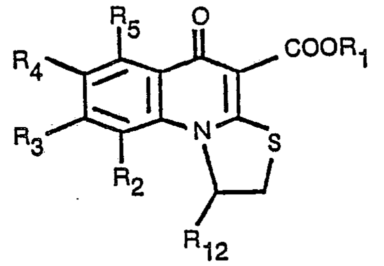

(Z)

in which:

$R_1$ is hydrogen, alkali metal, alkali earth metal, $(C_1$—$C_2)$-alkyl or pivalyloxymethyl

$R_2$ is hydrogen or halogen

$R_3$ is hydrogen, halogen or piperazinyl with or without substituents as $(C_1$—$C_2)$-alkyl, oxymethoxypropyl or oxyethyl, or phthalidyl

$R_4$ is hydrogen or halogen

$R_3$ and $R_4$ may form a ring with carbon chain containing oxygen

$R_5$ is hydrogen, halogen or piperazinyl

$R_{12}$ is $(C_1$—$C_2)$-alkyl.

2. Compounds of claim 1 in which $R_3$ and $R_4$ are methylenedioxy forming a ring.

3. Compounds represented by the following general formula (A) or pharmaceutically acceptable salts thereof:

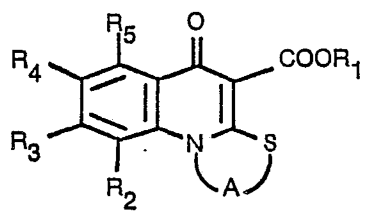

(A)

in which:

A is saturated or unsaturated hydrocarbon chain with 1 to 5 carbon atoms which may optionally be substituted with $(C_1$—$C_2)$-alkyl, hydroxyl, phenyl, ethoxy, phenoxy, phenylthio, halogen, halo-substituted methyl, methyl substituted with dimethylamino or with methylpiperazino, ethoxycarbonyl-substituted $(C_1$—$C_2)$-alkyl, carboxysubstituted $(C_1$—$C_2)$-alkyl, methoxy-substituted methyl, tetrahydropyranyloxymethyl, hydroxy-substituted methyl, $(C_1$—$C_2)$-acyloxy-substituted methyl, phenylamino, carboxy, nitro, cyano, carbonyl, thiocarbonyl or imino group, wherein A may form a ring,

$R_1$ is hydrogen, lithium, sodium, potassium, calcium, methyl, ethyl, pivaloyloxymethyl or phthalidyl,

$R_2$, $R_3$, $R_4$ and $R_5$ are same or different and they are hydrogen, fluorine, chlorine, bromine, hydroxy, ethoxy or

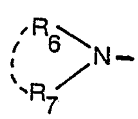

wherein

$R_3$ and $R_4$ may form a ring and wherein

$R_6$ and $R_7$ are same or different and they form five to seven-membered heterocyclic ring together with adjacent nitrogen atoms, wherein said heterocyclic ring may contain other hetero atom(s), may have substituent(s) or may form a salt.

**Patentansprüche**

1. Chinolincarbonsäurederivate der folgenden allgemeinen Formel (Z) oder ihre pharmazeutisch verträglichen Säureadditionssalze

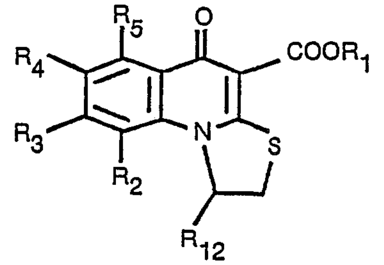

(Z)

wobei:

$R_1$ Wasserstoff, Alkalimetall, Erdalkalimetall, $(C_1—C_2)$-Alkyl oder Pivaloyloxymethyl,

$R_2$ Wasserstoff oder Halogen,

$R_3$ Wasserstoff, Halogen oder durch $(C_1—C_2)$-Alkyl, Oxymethoxypropyl oder -oxyäthyl substituiertes Piperazinyl oder Phthalidyl,

$R_4$ Wasserstoff oder Halogen, wobei $R_3$ und $R_4$ einen Ring mit eine Kohlenstoffkette enthaltendem Sauerstoff bilden können,

$R_5$ Wasserstoff, Halogen oder Piperazinyl und

$R_{12}$ $(C_1—C_2)$-Alkyl ist.

2. Verbindungen nach Anspruch 1, wobei $R_3$ und $R_4$ eine einen Ring bildende Methylendioxygruppe sind.

3. Verbindungen der folgenden allgemeinen Formel (A) oder ihre pharmazeutisch verträglichen Salze:

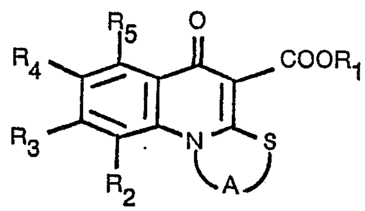

(A)

wobei

A eine gesättigte oder ungesättigte Kohlenwasserstoffkette mit 1 bis 5 Kohlenstoffatom ist, die wahlweise mit $(C_1—C_2)$-Alkyl, Hydroxyl, Phenyl, Äthoxy, Phenoxy, Phenylthio, Halogen, halogensubstituiertem Methyl, mit Dimethylamino oder Methylpiperazino substituiertem Methyl, äthoxycarbonylsubstituiertem $(C_1—C_2)$-Alkyl, carboxysubstituiertem $(C_1—C_2)$-Alkyl, methoxysubstituiertem Methyl, Tetrahydropyranyloxymethyl, hydroxysubstituiertem Methyl, $(C_1—C_2)$-acyloxysubstituiertem Methyl, Phenylamino, Carboxy, Nitro, Cyano, Carbonyl, Thiocarbonyl oder Imino substituiert sein können, wobei A einen Ring bilden kann,

$R_1$ Wasserstoff, Lithium, Natrium, Kalium, Calcium, Methyl, Äthyl, Pivaloyloxymethyl oder Phthalidyl ist,

$R_2$, $R_3$, $R_4$ und $R_5$ gleich oder unterschiedlich und Wasserstoff, Fluor, Chlor, Brom, Hydroxy, Äthoxy oder

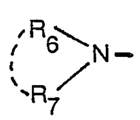

sind, wobei

$R_3$ und $R_4$ einen Ring bilden können und wobei $R_6$ und $R_7$ gleich oder unterschiedlich sind und einen fünf- bis siebengliedrigen heterocyclischen Ring mit benachbarten Stickstoffatomen bilden, wobei dieser heterocyclische Ring ein oder mehrere andere Heteroatome, einen oder mehrere Substituenten enthalten oder ein Salz bilden kann.

**Revendications**

1. Dérivés de l'acide quinoléinecarboxylique caractérisés en ce qu'ils répondent à la formule générale:

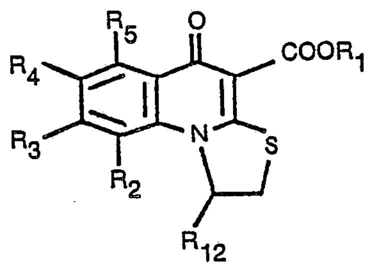

(Z)

dans laquelle

$R_1$ est un hydrogène, un métal alcalin, un mètal alcalinoterreux, un groupe alkyle en $C_1$—$C_2$ ou pivaloyloxyméthyle,

$R_2$ est un hydrogène ou un halogène,

$R_3$ est un hydrogène, unhalogène ou un groupe pipérazinyle avec ou sans substituants, tels qu'alkyle en $C_1$—$C_2$, oxy-méthoxypropyle ou oxyéthyle, ou phtalidyle,

$R_4$ est un hydrogène ou un halogène,

$R_3$ et $R_4$ peuvent former un cycle avec une chaîne carbonée contenant de l'oxygène,

$R_5$ est un hydrogène, un halogène ou un groupe pipérazinyle,

$R_{12}$ est un alkyle ($C_1$—$C_2$); et leurs sels d'addition d'acide pharmaceutiquement acceptables.

2. Composés de la revendication 1 dans laquelle $R_3$ et $R_4$ sont un groupe méthylènedioxy formant un cycle.

3. Composés caractérisés en ce qu'ils répondent à la formule générale (A) suivante et leurs sels pharmaceutiquement acceptables

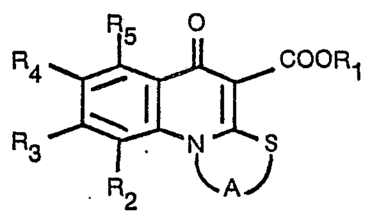

(A)

dans laquelle

A est une chaîne hydrocarbonée saturée ou insaturée ayant de 1 à 5 atomes de carbone qui, le cas échéant, peut être substituée par un groupe alkyle en $C_1$—$C_2$, hydroxyle, phényle, éthoxy, phénoxy, phénylthio, halogène, halogénométhyle, méthyle substitué par un diméthylamino ou un méthylpipérazino, un groupe alkyle en $C_1$—$C_2$ éthoxycarbonyle-substitué, un groupe alkyle en $C_1$—$C_2$ carboxy-substitué, un groupe méthyle méthoxy-substitué, un groupe tétrahydropyrannyloxyméthyle, un groupe méthyle hydroxy-substitué, un groupe méthyle acyloxy ($C_1$—$C_2$)-substitué, phénylamino, carboxy, nitro, cyano, carbonyle, thiocarbonyle ou imino, où A peut former un cycle,

$R_1$ est un atome d'hydrogène, de lithium, de sodium, de potassium, de calcium, un groupe méthyle, éthyle, pivaloyloxyméthyle ou phtalidyle,

$R_2$, $R_3$, $R_4$ et $R_5$, qui peuvent être identiques ou différents, représentent un atome d'hydrogène, de fluor, de chlore, de brome, un groupe hydroxy, un éthoxy ou

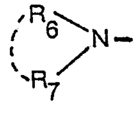

où

$R_3$ et $R_4$ peuvent former un cycle et ou $R_6$ et $R_7$ qui peuvent être identiques ou différents, forment un cycle hétérocyclique de cinq à sept sommets avec les atomes d'azote adjacents, ledit cycle hétérocyclique pouvant contenir un ou plusieurs autres hétéroatomes, pouvant avoir un ou plusieurs substituants ou pouvant former un sel.